# EUROPEAN PATENT SPECIFICATION

(11) **EP 3 615 694 B1**
(45) Date of publication and mention of the grant of the patent: **30.03.2022**
(21) Application number: 18727474.1
(22) Date of filing: 24.04.2018
(51) Int. Cl.: C12Q 1/6883

(54) **IL-8, IL-6, IL-1B AND TET2 AND DNMT3A IN ATHEROSCLEROSIS**
IL-8, IL-6, IL-1B UND TET2 SOWIE DNMT3A BEI ATHEROSKLEROSE
IL-8, IL-6, IL-1B ET TET2 ET DNMT3A DANS L'ATHÉROSCLÉROSE

(30) Priority: 25.04.2017 US 201762489823 P; 03.10.2017 US 201762567735 P
(43) Date of publication of application: 04.03.2020
(73) Proprietor: The Brigham and Women's Hospital, Inc., Boston, MA 02115 (US); The General Hospital Corporation, Boston, MA 02114 (US)
(72) Inventor: SIDDHARTHA, Jaiswal, Cambridge, Massachusetts 02142 (US); KATHIRESAN, Sekar, Cambridge, Massachusetts 02142 (US); EBERT, Benjamin, Cambridge, Massachusetts 02142 (US)
(74) Representative: Høiberg P/S
(86) International application number: PCT/US2018/029098
(87) International publication number: WO 2018/200489

(56) References cited:
- WO-A1-2009/120186
- WO-A1-2010/045346
- WO-A1-2012/163589
- WO-A1-2016/086197
- US-A1- 2004 208 873
- US-A1- 2013 136 726
- US-A1- 2016 377 632
- PAUL M RIDKER ET AL: "Interleukin-1 inhibition and the prevention of recurrent cardiovascular events: Rationale and Design of the Canakinumab Anti-inflammatory Thrombosis Outcomes Study (CANTOS)", AMERICAN HEART JOURNAL, ELSEVIER, AMSTERDAM, NL, vol. 162, no. 4, 20 June 2011 (2011-06-20) , pages 597-605, XP028308077, ISSN: 0002-8703, DOI: 10.1016/J.AHJ.2011.06.012 [retrieved on 2011-06-23]
- CAVUSOGLU ERDAL ET AL: "Elevated baseline plasma IL-8 levels are an independent predictor of long-term all-cause mortality in patients with acute coronary syndrome", ATHEROSCLEROSIS, ELSEVIER, AMSTERDAM, NL, vol. 242, no. 2, 17 August 2015 (2015-08-17), pages 589-594, XP029271880, ISSN: 0021-9150, DOI: 10.1016/J.ATHEROSCLEROSIS.2015.08.022

## Description

### FIELD

IL-8 inhibitor, IL-6 inhibitor, and/or IL-1β inhibitor for use in methods of treating atherosclerosis in subjects having a loss-of-function *TET*2 and/or *DNMT3A* mutation.

### BACKGROUND

Aging is associated with an increased incidence of both cancer and cardiovascular disease, including atherosclerosis and elevated cholesterol. Whole exome sequencing data has been used to identify a common, age-related disorder marked by an expansion of hematopoietic clones carrying recurrent somatic mutations, most commonly loss-of-function alleles in the genes *DNMT3A, TET2,* and *ASXL1* (1-3). These mutations, which are also common in the myelodysplastic syndrome and acute myeloid leukemia, provide a selective advantage to the hematopoietic stem cells in which they occur, and are detectable as clones in peripheral blood samples because the mutated stem cells maintain the ability to differentiate into circulating granulocytes, monocytes, and lymphocytes. Individuals under the age of 40 rarely accumulate these clones, but they become common in aging, with over 10% of those over age 70 harboring such a mutation. Carriers of these mutations have a ~ 10-fold increased risk of developing a hematologic malignancy. WO 2016/086197 A1 relates to methods for identifying and selecting a subject with increased risk of developing a cardiometabolic disease and discloses that mutations in *TET2, DNMT3A, ASXL1* and /or *JAK2* are markers of a cardiometabolic disease such as atherosclerosis.

Ridker et al. (Am Heart J 2011, 162(4):597-605) relates to interleukin-1beta inhibition to prevent cardiovascular disease.

US2004/0208873 relates to a method of treating atherosclerosis with IL-8 inhibition.

Clonal hematopoiesis of indeterminate potential (CHIP), defined by the presence of an expanded somatic blood cell clone in those without other hematologic abnormalities, is common in older individuals and associates with an increased risk of developing hematologic cancer. Some evidence of a connection between somatic *TET*2 and/or *DNMT3A* mutations in blood cells and atherosclerosis has also been demonstrated. However, the nature of this association was unclear and these mutations were not previously known to be associated with increased IL-8, IL-6, IL-1β levels or a need to inhibit IL-8 activity. Thus, a new method of treating and diagnosing atherosclerosis is now warranted, relying on the presence of both at least one *TET*2 and/or *DNMT3A mutation* and elevated IL-8 levels.

### SUMMARY

The invention is defined by the claims. Any subject matter falling outside the scope of the claims is provided for information purposes only.

The present inventors have found that individuals with CHIP are at increased risk for all-cause mortality and, surprisingly, for developing coronary heart disease. While traditional risk factors such as hypercholesterolemia, type 2 diabetes, hypertension, and smoking account for a large proportion of the risk for coronary heart disease, some individuals who develop coronary heart disease lack known risk factors, suggesting that unknown factors may also contribute to atherosclerotic complications.

As described in detail herein, carriers of clonal hematopoiesis of indeterminate potential (CHIP) had a 1.9-fold (95% confidence interval 1.4-2.7) increased risk of coronary heart disease compared to non-carriers in two prospective case-control cohorts. In two case-control cohorts for early-onset myocardial infarction, those with CHIP had a 4.0-fold greater risk (95% confidence interval 2.4-6.7) of having myocardial infarction. Those without clinical coronary heart disease but with clonal hematopoiesis also had increased coronary artery calcification, a marker of atherosclerotic burden and risk. Mutations in *DNMT3A, TET2, ASXL1,* and JAK2 individually associated with coronary heart disease in at least one set of cohorts. Hyperlipidemic mice engrafted with *Tet2-*/*-* or *Tet*2+/*-* bone marrow developed larger atherosclerotic lesions in the aortic root and aorta than mice receiving control marrow. Accordingly, clonal hematopoiesis associates with coronary heart disease in humans and causes accelerated atherosclerosis in a mouse model.

It was found that *TET*2 mutations, as well as those in *DNMT3A, ASXL1,* and JAK2, individually associate with risk of coronary heart disease in at least one set of human cohorts.

The invention relates to an IL-8 inhibitor, IL-6 inhibitor, and/or IL-1β inhibitor for use in a method of treating atherosclerosis in a human subject, the method comprising administering an effective amount of at least one IL-8 inhibitor, IL-6 inhibitor, and/or IL-1β inhibitor, wherein the subject has a loss-of-function *TET*2 and/or *DNMT3A* mutation, thereby treating atherosclerosis.

In some embodiments, the presence of said mutations has been determined by sequencing at least a part of a genome comprising *TET*2 and/or *DNMT3A* of one or more cells in a blood sample of the subject.

In some embodiments, the use in a method of treating atherosclerosis in a human subject comprises administering an effective amount of at least one IL-8 inhibitor, IL-6 inhibitor, and/or IL-1β inhibitor, wherein the subject's plasma IL-8 level is at least 20 ng/mL thereby treating atherosclerosis.

In some embodiments, the subject further has an increased level of plasma IL-8, wherein the level of plasma IL-8 is at least 20 ng/mL.

In some embodiments, the use further comprises administering an effective amount of at least one cholesterol-lowering medication to the subject. In some embodiments, the use further comprises prescribing exercise, cessation of smoking, diet modification, and/or stress reduction to the subject.

In some embodiments, the at least one loss-of-function *TET*2 and/or *DNMT3A* mutation comprises a frameshift mutation, nonsense mutation, missense mutation, or splice-site variant mutation. In some embodiments, the mutation in *TET*2 results in an amino acid change in TET2 chosen from S145N, S282F, A308T, N312S, L346P, P399L, S460F, D666G, S817T, P941S, C1135Y, R1167T, I1175V, S1204C, R1214W, D1242R, D1242V, Y1245S, R1261C, R1261H, R1261L, F1287L, W1291R, K1299E, K1299N, R1302G, E1318G, P1367S, C1396W, L1398R, V1417F, G1869W, L1872P, I1873T, C1875R, H1881Q, H1881R, R1896M, R1896S, S1898F, V1900A, G1913D, A1919V, R1926H, P1941S, P1962L, R1966H, R1974M, and R2000K.

In some embodiments, the mutation in *DNMT3A* results in an amino acid change in DNMT3A chosen from F290I, F290C, V296M, P307S, P307R, R326H, R326L, R326C, R326S, G332R, G332E, V339A, V339M, V339G, L344Q, L344P, R366P, R366H, R366G, A368T, A368V, R379H, R379C, I407T, I407N, I407S, F414L, F414S, F414C, A462V, K468R, C497G, C497Y, Q527H, Q527P, Y533C, S535F, C537G, C537R, G543A, G543S, G543C, L547H, L547P, L547F, M548I, M548K, G550R, W581R, W581G, W581C, R604Q, R604W, R635W, R635Q, S638F, G646V, G646E, L653W, L653F, I655N, V657A, V657M, R659H, Y660C, V665G, V665L, M674V, R676W, R676Q, G685R, G685E, G685A, D686Y, D686G, R688H, G699R, G699S, G699D, P700L, P700S, P700R, P700Q, P700T, P700A, D702N, D702Y, V704M, V704G, I705F, I705T, I705S, I705N, G707D, G707V, C710S, C710Y, S714C, V716D, V716F, V716I, N717S, N717I, P718L, R720H, R720G, K721R, K721T, Y724C, R729Q, R729W, R729G, F731C, F731L, F731Y, F731I, F732del, F732C, F732S, F732L, E733G, E733A, F734L, F734C, Y735C, Y735N, Y735S, R736H, R736C, R736P, L737H, L737V, L737F, L737R, A741V, P742P, P743R, P743L, R749C, R749L, R749H, R749G, F751L, F751C, F752del, F752C, F752L, F752I, F752V, W753G, W753C, W753R, L754P, L754R, L754H, F755S, F755I, F755L, M761I, M761V, G762C, V763I, S770L, S770W, S770P, R771Q, F772I, F772V, L773R, L773V, E774K, E774D, E774G, I780T, D781G, R792H, W795C, W795L, G796D, G796V, N797Y, N797H, N797S, P799S, P799R, P799H, R803S, R803W, P804L, P804S, K826R, S828N, K829R, T835M, N838D, K841Q, Q842E, P849L, D857N, W860R, E863D, F868S, G869S, G869V, M880V, S881R, S881I, R882H, R882P, R882C, R882G, A884P, A884V, Q886R, L889P, L889R, G890D, G890R, G890S, V895M, P896L, V897G, V897D, R899L, R899H, R899C, L901R, L901H, P904L, F909C, P904Q, A910P, C911R, C911Y.

In some aspects, the human subject has at least one somatic blood cell clone with one mutant loss-of-function *TET*2 allele and one wildtype *TET*2 allele. In some embodiments, the human subject has at least one somatic blood cell clone with two mutant loss-of-function *TET*2 alleles. In some aspects, the human subject has at least one somatic blood cell clone with one mutant loss-of-function *DNMT3A* allele and one wildtype *DNMT3A* allele. In some embodiments, the human subject has at least one somatic blood cell clone with two mutant loss-of-function *DNMT3A* alleles. In some embodiments, the human subject has clonal hematopoiesis of indeterminate potential (CHIP).

The human subject may have at least one loss-of-function *TET*2 and/or *DNMT3A* mutation in at least 5%, 10%, 13.5%, 15%, 20%, 25%, 27%, 30% of nucleated peripheral blood cells. The human subject may also have a plasma level of IL-8 that is at least 25 ng/mL, 30 ng/mL, 40 ng/mL, 45 ng/mL, 50 ng/mL, 55 ng/mL, 60 ng/mL, 65 ng/mL, 70 ng/mL, 75 ng/mL, or 80 ng/mL.

In some embodiments, the at least one IL-6 inhibitor and/or IL-1β inhibitor is methotrexate. Optionally, the methotrexate is administered at a dose of from 15 to 20 mg/week.

In some embodiments, the at least one IL-8 inhibitor is an IL-8 depleting drug. In some embodiments, the at least one IL-8 inhibitor is an IL-8 activity reducing drug. In some embodiments, the at least one IL-8 inhibitor comprises an anti-IL-8 antibody or an antigen binding fragment thereof. In some embodiments, the anti-IL-8 antibody or antigen binding fragment thereof comprises HuMaxIL-8, HuMab-10F8, or an antigen binding fragment thereof. In some embodiments, the at least one IL-8 inhibitor is an inhibitor of the IL-8 receptor CXCR2. In some embodiments, the at least one IL-8 inhibitor comprises an anti-CXCR2 antibody or an antigen binding fragment thereof. In some embodiments, the at least one IL-8 inhibitor comprises the CXCR2 inhibitor SB-332235 (GlaxoSmithKline).

In some embodiments, the IL-6 inhibitor is an IL-6 depleting drug. In some embodiments, the IL-6 inhibitor is an IL-6 activity reducing drug. In some embodiments, the IL-6 inhibitor comprises an anti-IL-6 antibody or an antigen binding fragment thereof. In some embodiments, the anti-IL-6 antibody or antigen binding fragment thereof comprises siltuximab, olokizumab, elsilimomab, mAb 1339, BMS-945429, sirukumab, CPSI-2364, ALX-0061, clazakizumab, ARGX-109, MEDI5117, FE301, FM101, or C326. In some embodiments, the at least one IL-6 inhibitor is an inhibitor of the IL-6 receptor IL-6R or an inhibitor of gp130. In some embodiments, the inhibitor of IL-6R comprises tocilizumab or sarilumab. In some embodiments, the IL-6 inhibitor comprises tamibarotene or ATRA.

In some embodiments, the IL-1β inhibitor is an IL-1β depleting drug. In some embodiments, the IL-1β inhibitor is an IL-1β activity reducing drug. In some embodiments, the IL-1β inhibitor comprises an anti- IL-1β antibody or antigen binding fragment thereof. In some embodiments, the anti-IL-1β antibody or antigen binding fragment thereof comprises canakinumab. In some embodiments, the IL-1β inhibitor is an inhibitor of the IL-1β receptor. In some embodiments, the IL-1β inhibitor is an inhibitor of IL-1 receptor. In some embodiments, the inhibitor of the IL-1 receptor is anakinra.

In some embodiments, at least one cholesterol-lowering medication comprises at least one PCSK9 inhibitor, at least one statin, at least one selective cholesterol absorption inhibitor, at least one resin, at least one lipid-lowering therapy, at least one CETP inhibitor, at least one pantothenic acid derivative, at least one microsomal triglyceride transfer protein (MTP) inhibitor, at least one adenosine triphosphate-binding cassette transporter A1 (ABCA1)-promoter, aspirin, estrogen, and/or at least one lipoprotein complex.

In some embodiments, the cholesterol-lowering medication comprises at least one PCSK9 inhibitor. In some embodiments, the PCSK9 inhibitor is chosen from at least one of (i) an anti-PCSK9 antibody or antigen-binding fragment thereof, (ii) an antisense or RNAi therapeutic agent that inhibits the synthesis of PCSK9, (ii) a PCSK9-targeting vaccine. In some embodiments, the anti-PCSK9 antibody or antigen-binding fragment thereof is evolocumab, alirocumab, bococizumab, LGT209, RG7652, or LY3015014. In some embodiments, the RNAi therapeutic agent that inhibits the synthesis of PCSK9 is inclisiran. In some embodiments, the PCSK9-targeting vaccine is AT04A or AT06A. In some embodiments, the PCSK9 inhibitor is a polypeptide that binds PCSK9 (such as adnectin). In some embodiments, the PCSK9 inhibitor is a locked nucleic acid targeting PCSK9 (such as SPC5001). In some embodiments, the PCSK9 inhibitor is an antisense RNA that inhibits the synthesis of PCSK9 is ISIS-405879/BMS-844421.

In some embodiments, the cholesterol-lowering medication comprises at least one statin. In some embodiments, the statin is chosen from at least one of atorvastatin, fluvastatin, lovastatin, pravastatin, rosuvastatin, simvastatin, and pitavastatin. In some embodiments, the statin comprises a combination therapy chosen from (i) lovastatin and niacin, (ii) atorvastatin and amlodipine, and (iii) simvastatin and ezetimibe.

In some embodiments, the cholesterol-lowering medication comprises at least one selective cholesterol absorption inhibitor. In some embodiments, the selective cholesterol absorption inhibitor is ezetimibe.

In some embodiments, the cholesterol-lowering medication comprises at least one resin. In some embodiments, the resin is chosen from cholestyramine, colestipol, and colesevelam.

In some embodiments, the cholesterol-lowering medication comprises at least one lipid-lowering therapy. In some embodiments, the lipid-lowering therapy is chosen from at least one fibrate, niacin, and at least one omega-3 fatty acid. In some embodiments, the lipid-lowering therapy comprises at least one fibrate. In some embodiments, the fibrate is chosen from gemfibrozil, fenofibrate, and clofibrate. In some embodiments, the lipid-lowering therapy comprises at least one omega-3 fatty acid. In some embodiments, the omega-3 fatty acid is chosen from at least one of omega-3 fatty acid ethyl esters and omega-3 polyunsaturated fatty acids. In some embodiments, the omega-3 fatty acid ethyl esters are icosapent ethyl. In some embodiments, the omega-3 polyunsaturated fatty acids are marine-derived omega-3 polyunsaturated fatty acids.

In some embodiments, the cholesterol-lowering medication comprises a CETP inhibitor. In some embodiments, the CETP inhibitor is chosen from at least one of anacetrapib and obicetrapib. In some embodiments, the cholesterol-lowering medication comprises at least one MTP inhibitor. In some embodiments, the MTP inhibitor is chosen from at least one of (i) a small molecule that inhibits function of MTP, (ii) an RNAi therapeutic agent that inhibits the synthesis of MTP, and (iii) an antisense RNA that inhibits synthesis of MTP. In some embodiments, the small molecule that inhibits function of MTP is chosen from at least one of lomitapide, JTT-130, Slx-4090, and dirlotapide.

In some embodiments, the cholesterol-lowering medication comprises adenosine triphosphate-binding cassette transporter A1 (ABCA1)-promoter. In some embodiments, the adenosine triphosphate-binding cassette transporter A1 (ABCA1)-promoting drug is chosen from at least one of (i) an apoA-1 mimetic peptide, (ii) a full-length apoA-1, and (iii) a reconstituted HDL. In some embodiments, the apoA-1 mimetic peptide is FAMP type 5 (FAMP5). In some embodiments, the full-length apoA-1 is ApoA-1-Milano or ETC-216. In some embodiments, the cholesterol-lowering medication comprises estrogen. In some embodiments, the cholesterol-lowering medication comprises at least one lipoprotein complex. In some embodiments, the lipoprotein complex is chosen from at least one of CER-001, CSL-111, CSL-112, and ETC-216. In some embodiments, the lipoprotein complex is chosen from at least one of apolipoprotein or apolipoprotein peptide mimic. In some embodiments, the (i) apolipoprotein is chosen from at least one of ApoA-I, ApoA-II, ApoA-IV, and ApoE and/or (ii) the peptide mimetic is chosen from at least one of ApoA-I, ApoA-II, ApoA-IV, and ApoE peptide mimic.

In some embodiments, the human subject also exhibits one or more risk factors of being a smoker, having level of total cholesterol of at least 200 mg/dL, or having level of low-density lipoprotein (LDL) of at least 130 mg/dL. In some embodiments, the human subject has a total cholesterol of at least 240 mg/dL and/or an LDL of at least 160 mg/dL. In some embodiments, the human subject has elevated hsCRP and optionally an hsCRP level of at least 2 mg/L.

In some embodiments, the use comprises prescribing exercise. In some embodiments, the method comprises prescribing exercise for at least 3, 4, 5, 6, or 7 days a week. In some embodiments, the method comprises prescribing cardiovascular conditioning exercise. In some embodiments, the method comprises prescribing strength training exercise. In some embodiments, the method comprises prescribing cessation of smoking. In some embodiments, the method comprises administering a medication to support smoking cessation. In some embodiments, the medication to support smoking cessation is chosen from at least one of nicotine replacement therapy, antidepressants (such as bupropion, nortriptyline, or an SSRI), varenicline, and clonidine.

In some embodiments, the use comprises diet modification. In some embodiments, the diet modification is chosen from at least one of a reduction in fat consumption, a reduction in cholesterol consumption, a reduction in sugar consumption, an increase in fruit and/or vegetable consumption, an increase in omega fatty acids, and/or reduction of alcohol consumption. In some embodiments, the method comprises stress reduction. In some embodiments, the stress reduction is chosen from at least one of relaxation techniques, mediation, breathing exercises, exercise, and/or anger management. In some embodiments, the method comprises prescribing psychiatric medication. In some embodiments, the method comprises anti-anxiety medication and/or anti-depressant medication. In some embodiments, the anti-anxiety medication and/or anti-depressant medication is chosen from at least one of citalopram, escitalopram, fluoxetine, paroxetine, sertraline, duloxetine, venlafaxine, imipramine, hydroxyzine, propanolol, gabapentin, and pregabalin. In some embodiments, the method comprises prescribing psychological counseling.

In some embodiments, the loss-of function *TET*2 and/or *DNMT3A* mutation is identified by whole exome sequencing (WES). In some embodiments, loss-of-function *TET*2 and/or *DNMT3A* mutation is identified by sequencing DNA.

### BRIEF DESCRIPTION OF THE DRAWINGS

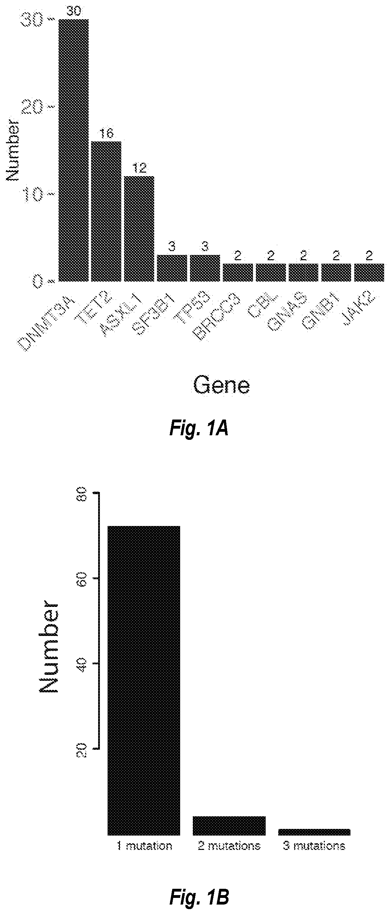

**Figures 1A-1B** show variant characteristics in Bioimage and MDC studies. **A)** Top 10 most frequently mutated genes. Total number of variants per gene is listed. **B)** Number of people with 1, 2, or 3 somatic variants in BioImage and MDC studies.

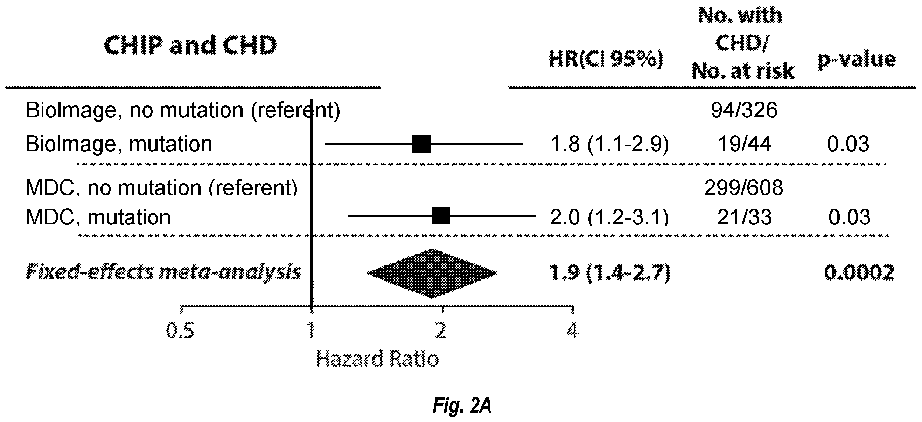

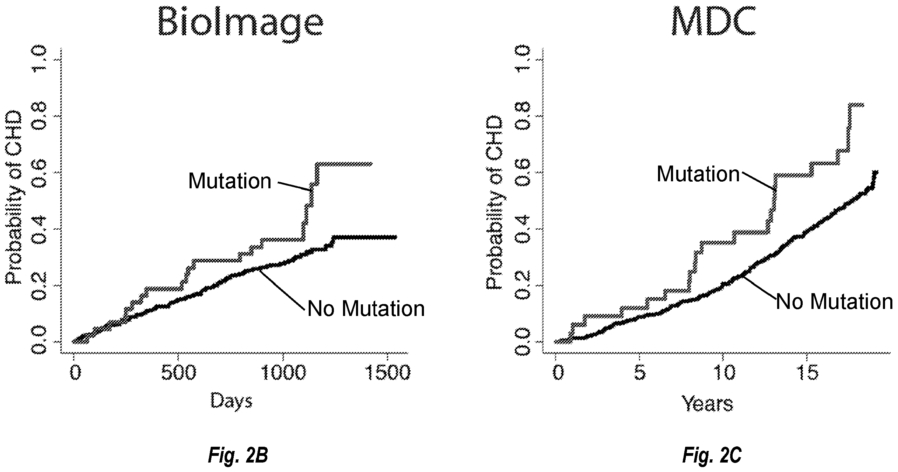

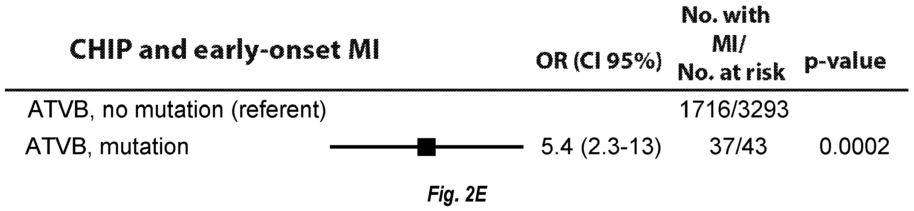

**Figures 2A-2D** show that CHIP associates with coronary heart disease. **A)** Forest plot for association between CHD and CHIP in BioImage and MDC. Hazard ratio for having CHD in those with mutations was obtained by a Cox proportional hazards model adjusted for age, sex, type 2 diabetes, total cholesterol, high density lipoprotein cholesterol, smoking status, and hypertension. **B)** Cumulative incidence plots for CHD in BioImage and MDC. C) Forest plot for association between CHD and CHIP, segregated by variant allele fraction above or below median (13.4%). Hazard ratio for having a mutation obtained as in (A). **D)** Forest plot for association between myocardial infarction and CHIP in ATVB. Odds ratio for MI risk was obtained by a logistic regression model adjusted for age, sex, type 2 diabetes, smoking status. CHIP (clonal hematopoiesis of indeterminate potential), CHD (coronary heart disease), HR (hazard ratio), MDC (Malmo Diet and Cancer Study), VAF (variant allele fraction), MI (myocardial infarction), OR (odds ratio), ATVB (Atherosclerosis, Thrombosis, and Vascular Biology Italian Study Group).

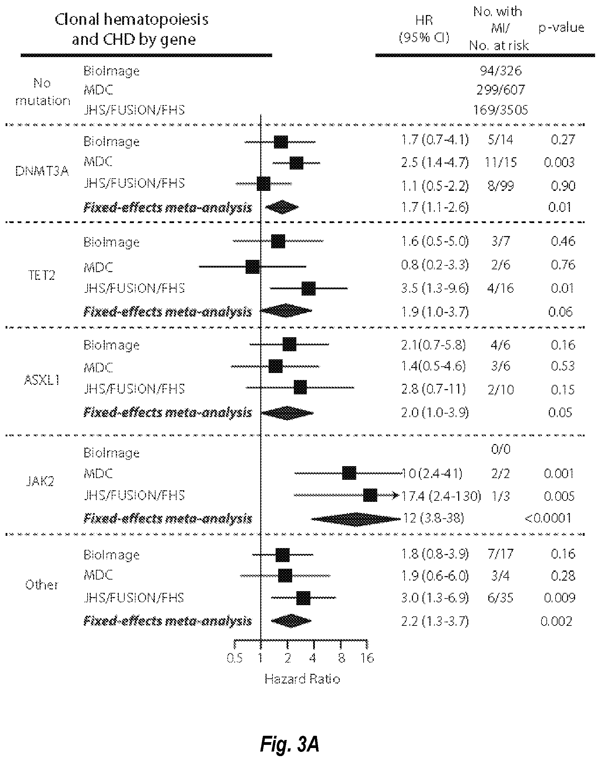

**Figures 3A-3B** show mutations in *DNMT3A, TET2, ASXL1,* and JAK2 associate with coronary heart disease. A) Forest plot for risk of CHD in BioImage and MDC by mutated gene. Hazard ratio for listed mutations was obtained by a fixed-effects meta-analysis of Cox proportional hazards models adjusted for age, sex, type 2 diabetes, total cholesterol, high-density lipoprotein cholesterol, triglycerides, smoking status, and hypertension from BioImage, MDC, and JHS/FUSION/FHS. Forest plot for risk of CHD in FHS, JHS, and FUSION by mutated gene. Hazard ratio for listed mutations was obtained by a Cox proportional hazards model adjusted for age, sex, type 2 diabetes, total cholesterol, high-density lipoprotein cholesterol, smoking status, and hypertension. **B)** Table for risk of early-onset MI in ATVB by mutated gene. Odds ratio for having MI in those with listed mutations was obtained by Fisher's exact test, p-values not adjusted for multiple hypothesis testing.

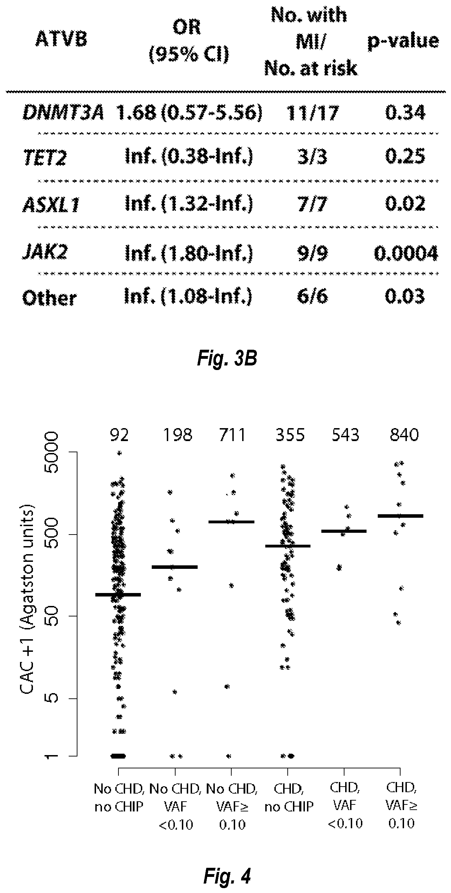

**Figure 4** shows coronary artery calcification by variant allele fraction (VAF) in those with and without incident CHD. Horizontal bar represents the median value for coronary artery calcification score plus 1. The median value for each group is also listed above each plot.

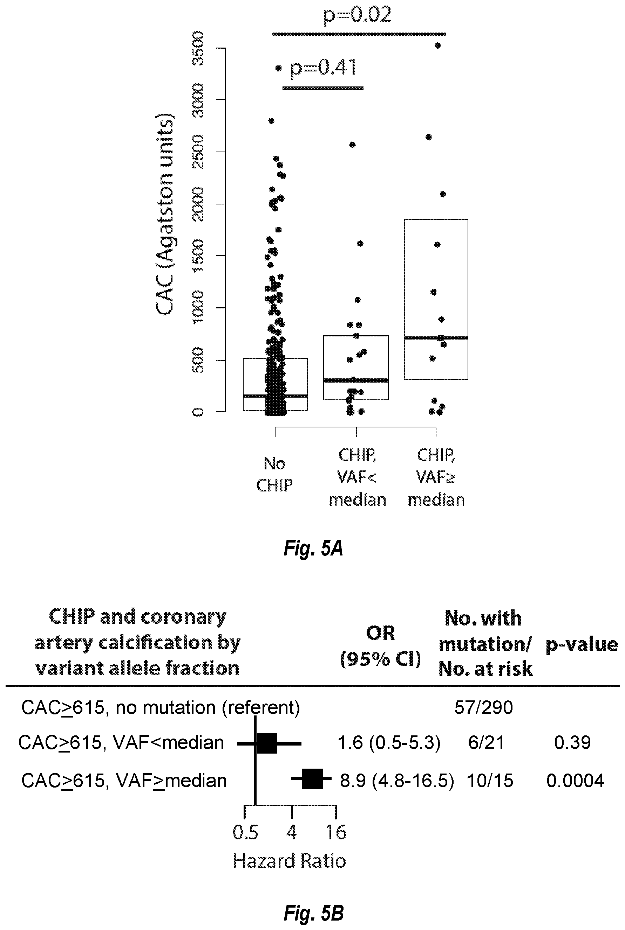

**Figures 5A-5B** show that CHIP is associated with subclinical atherosclerosis in humans. A) Coronary artery calcification scores in those without CHIP, CHIP with variant allele fraction below median (13.5%), or CHIP with variant allele fraction greater than equal to median. Wald p-values obtained by linear regression on CAC values that were natural logarithm transformed after adding 1 and adjusted for age, sex, type 2 diabetes, total cholesterol, high-density lipoprotein cholesterol, smoking status, and hypertension. Box represents 25th to 75th percentile and black line represents the median. **B)** Forest plot for association between coronary artery calcification score ≥615 and mutation status in BioImage. Odds ratio was obtained by a logistic regression model adjusted for age, sex, type 2 diabetes, total cholesterol, high-density lipoprotein cholesterol, smoking status, and hypertension. CAC (coronary artery calcification), VAF (variant allele fraction).

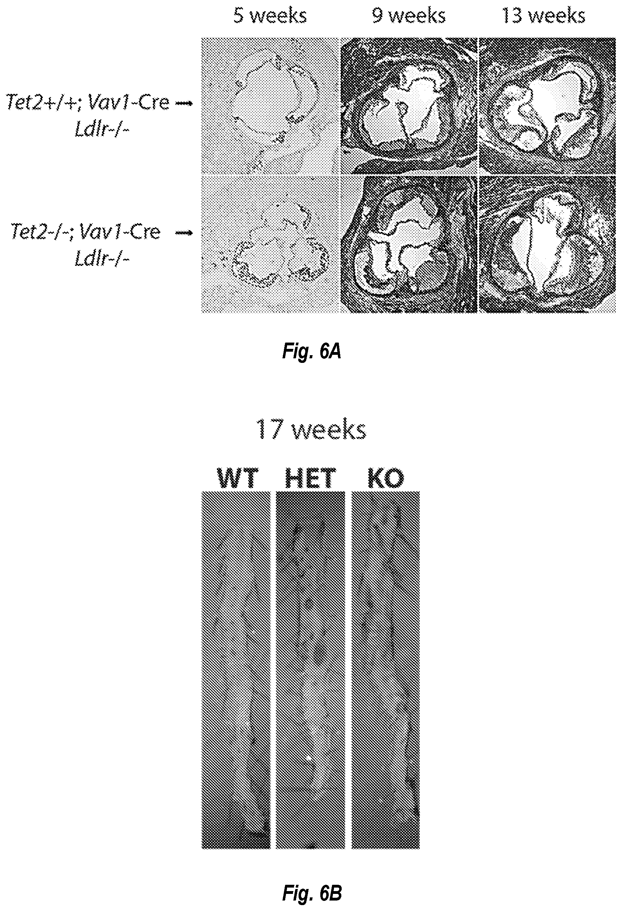

**Figures 6A-6E** show that loss of *Tet2* in hematopoietic cells accelerates atherosclerosis in a mouse model. A) Aortic root sections in female *Ldlr*-/- mice transplanted with either *Tet*2+/+*; Vav1-Cre* or *Tet*2-/-*; Vav1-Cre* marrow followed by 5, 9, or 13 weeks of feeding on high cholesterol diet. Oil red O (left) and Masson's trichrome (center, right) images are shown (40X magnification). Dashed lines indicate lesion area. **B)** Descending aorta lesions stained with oil red O at 17 weeks in female Ldlr-/- mice transplanted with either *Tet*2+/+*; Vav1-Cre* (WT), *Tet*2+/-*; Vav1-Cre* (HET), or *Tet*2-/-*; Vav1-Cre* (KO) marrow. **C)** Quantification of aortic root lesions in female Ldlr-/- mice transplanted with either *Tet*2+/+*; Vav1-Cre* (WT), *Tet*2+/-*; Vav1-Cre* (HET), or *Tet*2-/-*; Vav1-Cre* (KO) marrow at 5, 9, or 13 weeks on diet. P-values obtained by Wilcoxon rank-sum test for 5- and 9-week time points, and by Dunn's Kruskal-Wallis test for multiple comparisons using Benjamini-Hochberg correction for the 13-week time point. **D)** Quantification of descending aorta lesions at 17 weeks in female *Ldlr*-/- mice transplanted with either *Tet*2+/+*; Vav1-Cre* (WT), *Tet*2+/-*; Vav1-Cre* (HET), or *Tet*2-/-*; Vav1-Cre* (KO) marrow. P-values obtained by Dunn's Kruskal-Wallis test for multiple comparisons using Benjamini-Hochberg correction. **E)** Quantification of aortic root lesions in female *Ldlr*-/- mice transplanted with either *Tet*2+/+*; Lyz2-Cre* (WT) or *Tet*2-/-*; Lyz2-Cre* (KO) at 10 weeks on diet. P-value obtained by Wilcoxon rank sum test. For all plots, black horizontal line represents the median value.

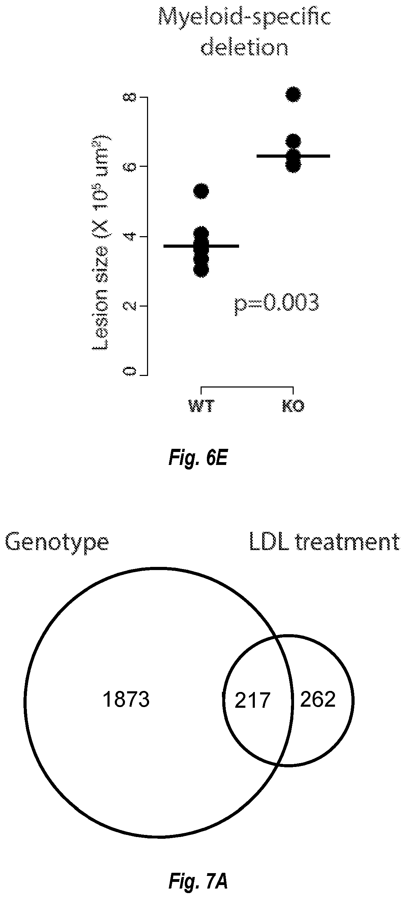

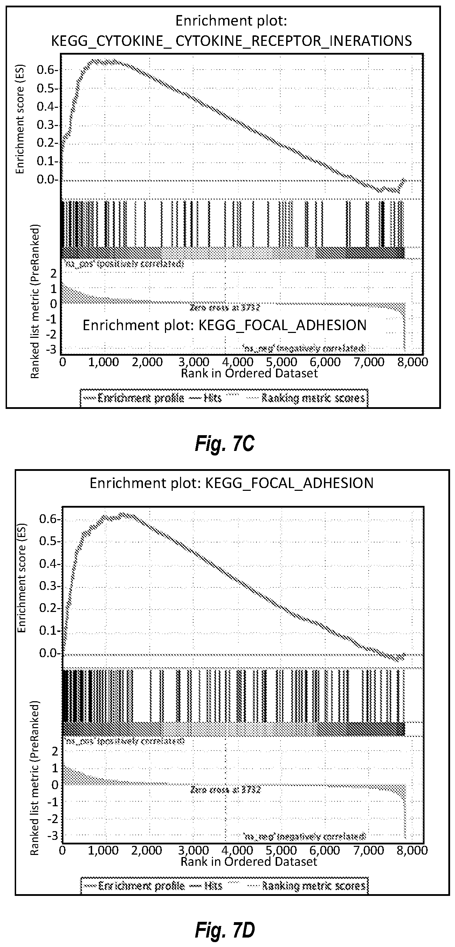

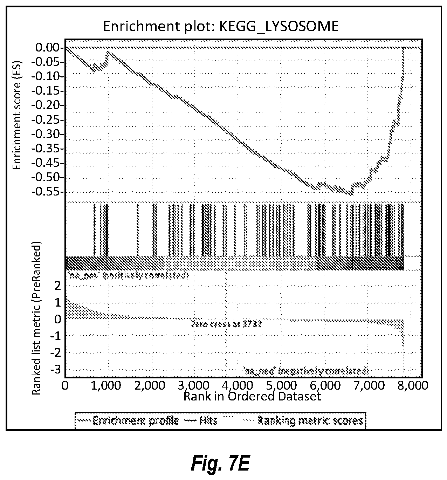

**Figures 7A-7C** show gene-expression analysis of *Tet*2-/- bone marrow derived macrophages. A) BMDM were cultured with 200 mg/dL native low density lipoprotein (LDL) or vehicle for 24 hours and messenger RNA was assessed by RNA-sequencing. At a false discovery rate of q < 0.05, 2090 genes were differentially expressed by genotype (*Tet*2+/+ versus *Tet*2-/-), 479 genes differentially expressed by LDL treatment, and 217 genes were differentially expressed by both variables. **B)** Volcano plot for gene expression changes in *Tet*2+/+ versus *Tet*2-/- BMDM. Highlighted are selected genes from the KEGG groups "Cytokine/Cytokine Receptor Interaction", "Focal Adhesion", and "Lysosome". **C)** Gene set enrichment analysis plots for the KEGG sets "Cytokine/Cytokine Receptor Interaction", "Focal Adhesion", and "Lysosome". KEGG - Kyoto Encyclopedia of Genes and Genomes, LDL - low density lipoprotein KO - *Tet*2-/-*,* WT - *Tet*2+/+*.*

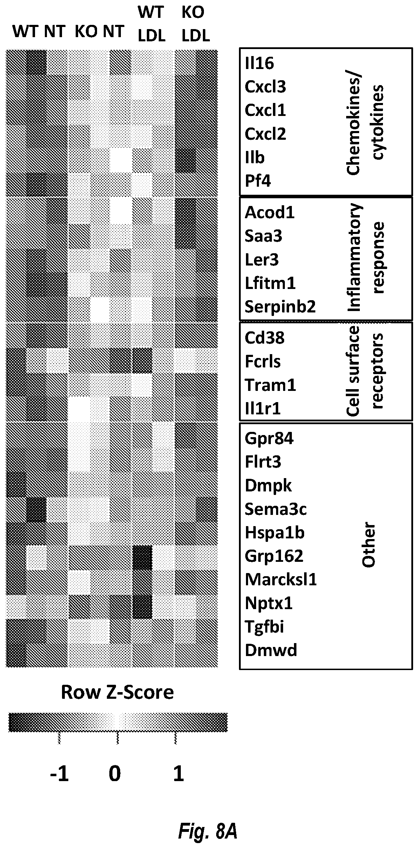

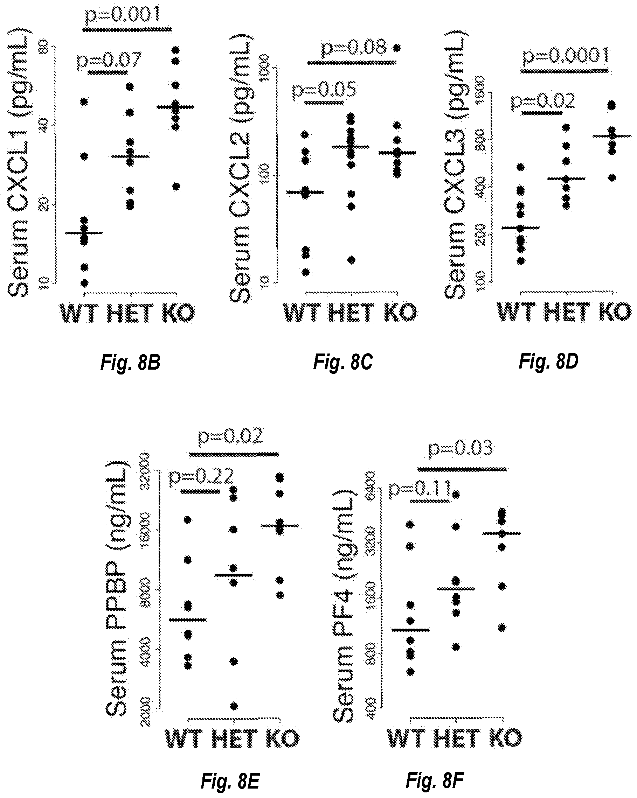

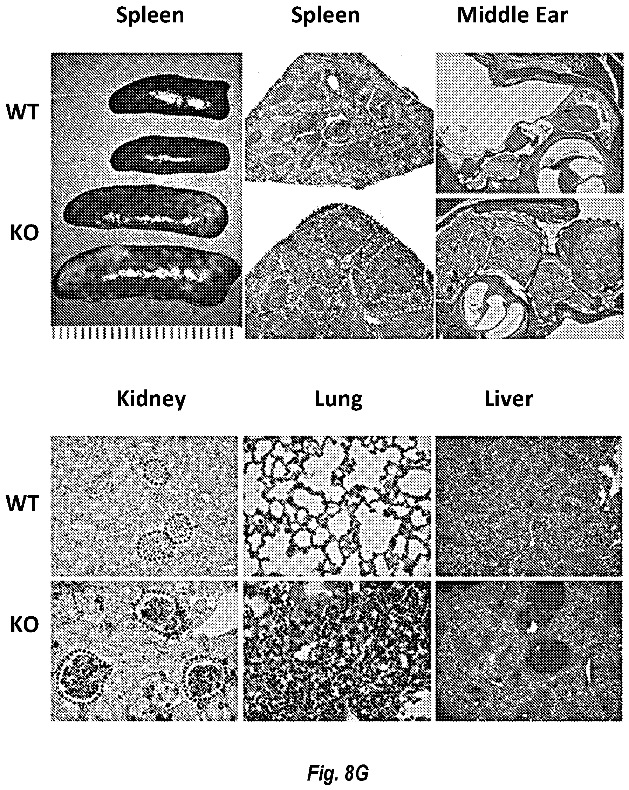

**Figures 8A-8C** show that *Tet2* deficiency causes dysregulated chemokine expression in macrophages *in vitro* and *in vivo.* A) Heatmap of the top 25 most up-regulated genes of the 217 genes that were differentially expressed in both LDL treated and in *Tet*2+/+*; Vav1-Cre* (WT) versus *Tet*2-/-*; Vav1-Cre* (KO) bone marrow derived macrophages (BMDM) *in vitro.* **B)** Quantification of serum chemokine levels in *Ldlr*-/- mice transplanted with either *Tet*2+/+*; Vav1-Cre* (WT), *Tet*2+/-*; Vav1-Cre* (HET), or *Tet*2-/-*; Vav1-Cre* (KO). Serum samples were obtained after 13 or 17 weeks on diet. P-values obtained by Dunn's Kruskal-Wallis test for multiple comparisons using Benjamini-Hochberg correction. C) Tissue examination of *Ldlr*-/- mice transplanted with either *Tet*2+/+*; Vav1-Cre* (WT) or *Tet*2-/-*; Vavl-Cre* (KO) marrow after 17 weeks on diet. Shown are spleen (gross), spleen, Mac-2 immunohistochemistry (40X), middle ear H/E (40X), kidney, Mac-2 immunohistochemistry (200X), lung H/E (400X), and liver H/E (40X). Xanthomatous areas are depicted within white dashed lines, glomeruli are shown within dashed black lines. P-value obtained by Wilcoxon rank sum test on natural logarithm transformed values. For all charts, box represents 25th to 75th percentile, whiskers represent 1.5 times the interquartile range, and black line represents the median. LDL (low density lipoprotein), IL-8 (interleukin-8).

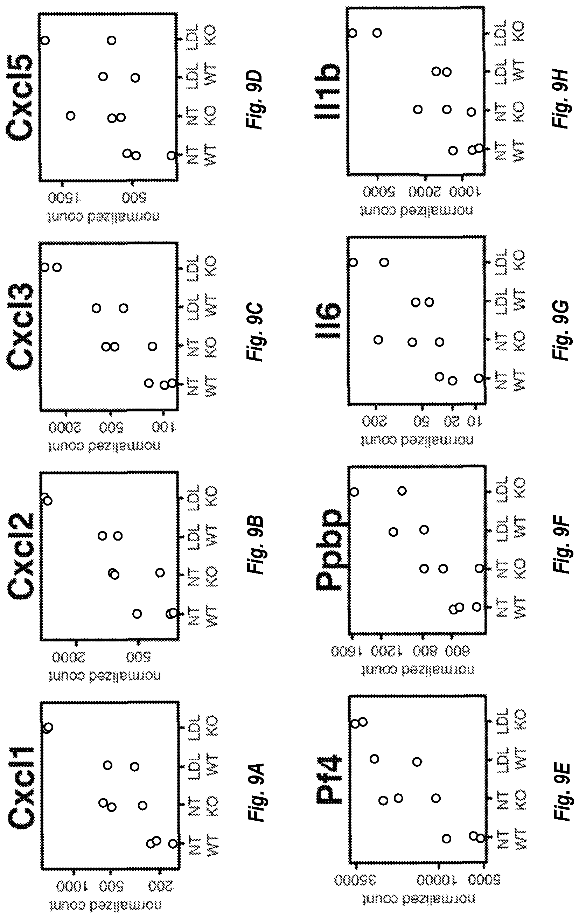

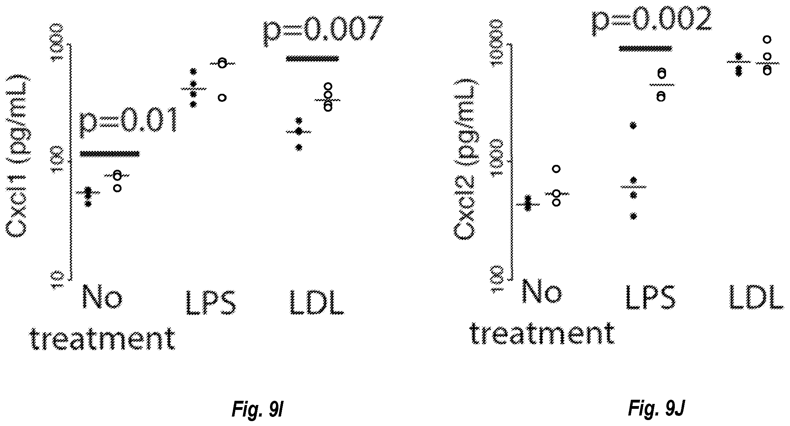

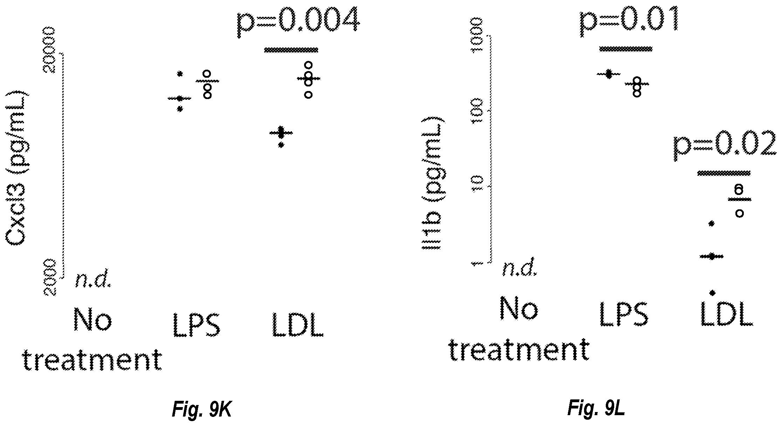

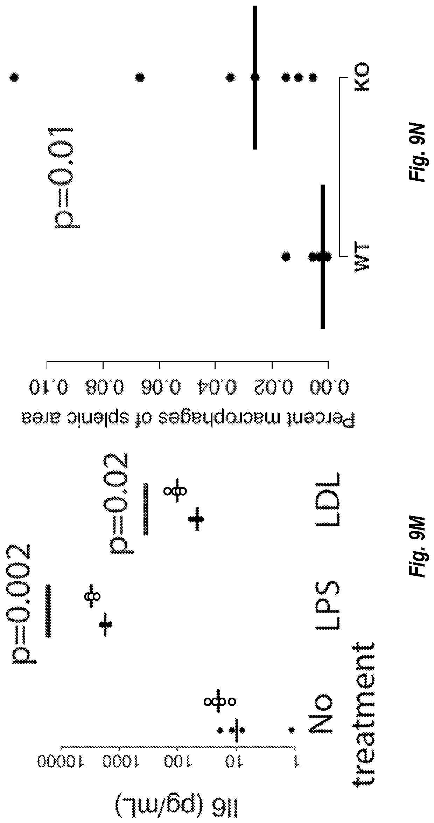

**Figures 9A-9C** show chemokine expression related to *Tet2* deficiency. **A)** BMDM were cultured with 200 mg/dL native low density lipoprotein (LDL) or vehicle for 24 hours and messenger RNA was assessed by RNA-sequencing. Shown are normalized reads counts per sample for select genes. **B)** BMDM were cultured with 200 mg/dL native LDL, 10 ng/mL lipopolysaccharide (LPS), or vehicle for 24 hours and protein secretion was measured in the cell culture supernatant. *Tet*2+/+ macrophage supernatant is shown in left-hand groups for each treatment, and *Tet*2-/- is shown in right-hand groups for each treatment. P-values by Welch's t-test, only values less than 0.05 are shown. Four biological replicates per treatment are shown. **C)** Proportion of Mac-2 (macrophage marker) positive staining area in spleens (4X magnification) from *Ldlr*-/- mice receiving *Tet*2+/+ or *Tet*2+/+ marrow after 17 weeks on diet. P-value obtained by Wilcoxon rank-sum test. NT - no treatment, LDL - low density lipoprotein, LPS - lipopolysaccharide, KO - *Tet*2-/-*,* WT - *Tet*2+/+*,* VAF - variant allele fraction, n.d. - not detected.

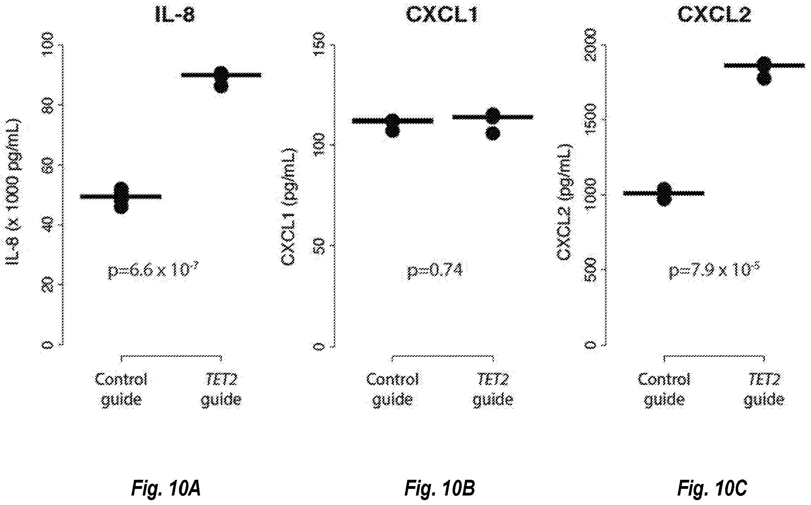

**Figure 10** shows IL-8, CXCL1, and CXCL2 levels in control cells and cells modified by CRISPR to express TET2 mutations. IL-8 and CXCL2 levels were higher in the cells with TET2 mutations.

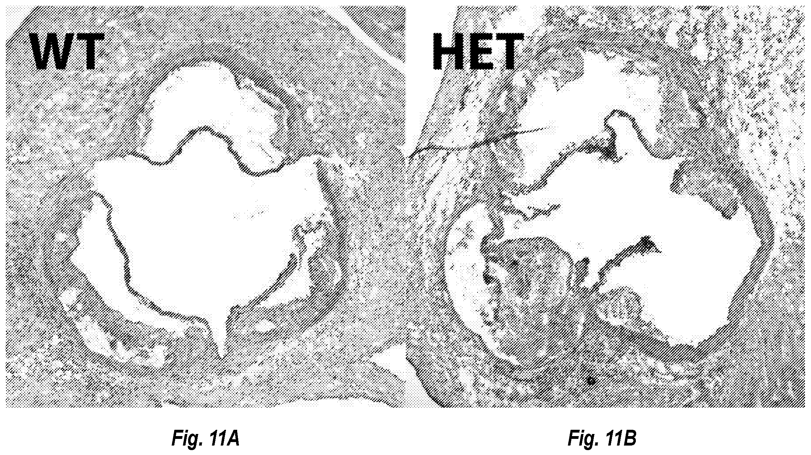

Figure 11 shows representative sections from an experiment where *Ldlr*-/- mice were lethally irradiated and transplanted with either *Dnmt3a*+/+ (WT) or Dnmt3a+/- (HET) bone marrow. After 10 weeks on high cholesterol diet, lesions in the aortic root were assessed.

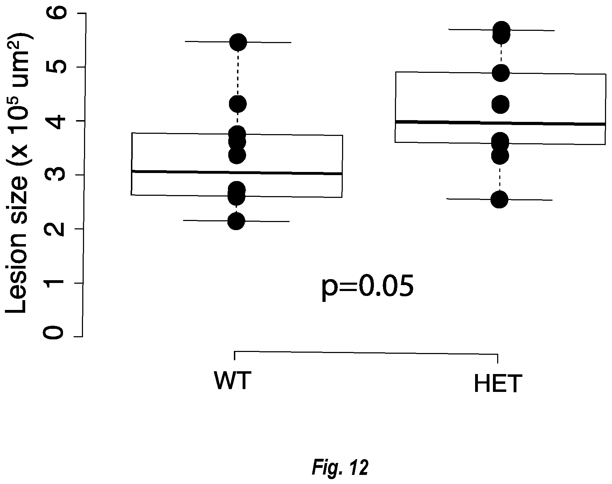

Figure 12 shows quantitative assessment of aortic root lesion size from an experiment where *Ldlr*-/- mice were lethally irradiated and transplanted with either *Dnmt3a*+/+ (WT) or Dnmt3a+/- (HET) bone marrow. After 10 weeks on high cholesterol diet, lesions in the aortic root were assessed.

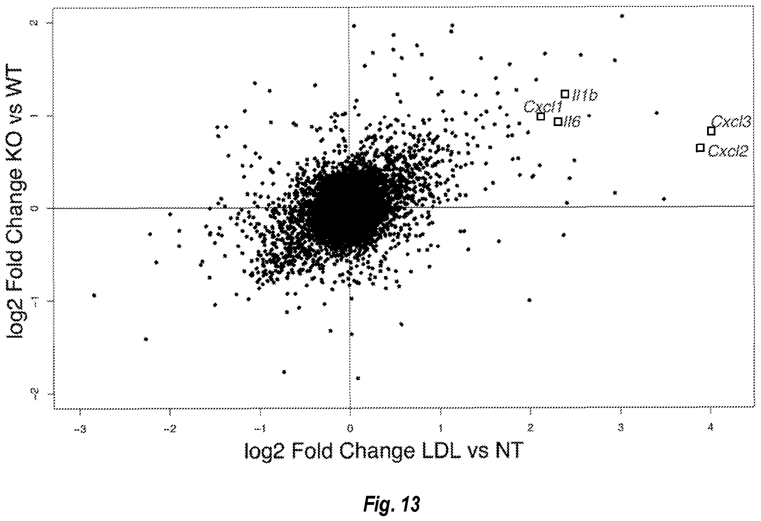

Figure 13 shows a plot of relative expression of genes by treatment with LDL (x-axis) and by genotype (y-axis). BMDM from *Dnmt3a*+/+ or *Dnmt3a*-/- mice were loaded with vehicle or 200 mg/dL LDL and RNA sequencing was performed. Highlighted are *Il6, Illb, Cxcl1, Cxcl2,* and *Cxcl3.*

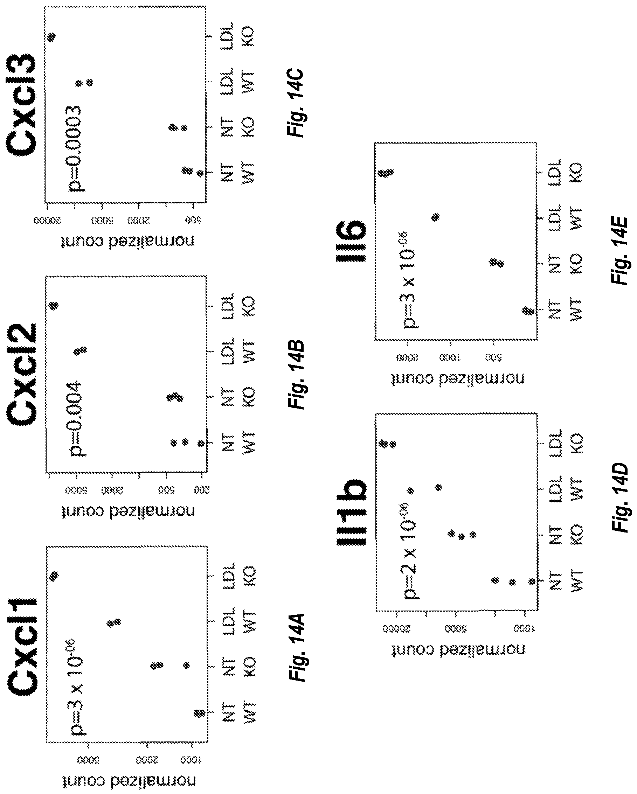

Figure 14 shows normalized read counts from an experiment where BMDM from *Dnmt3a*+/+ or *Dnmt3a*-/- mice were loaded with vehicle or 200 mg/dL LDL and RNA sequencing was performed. Shown are normalized read counts for *Il6, Il1b, Cxcl1, Cxcl2,* and *Cxcl3.*

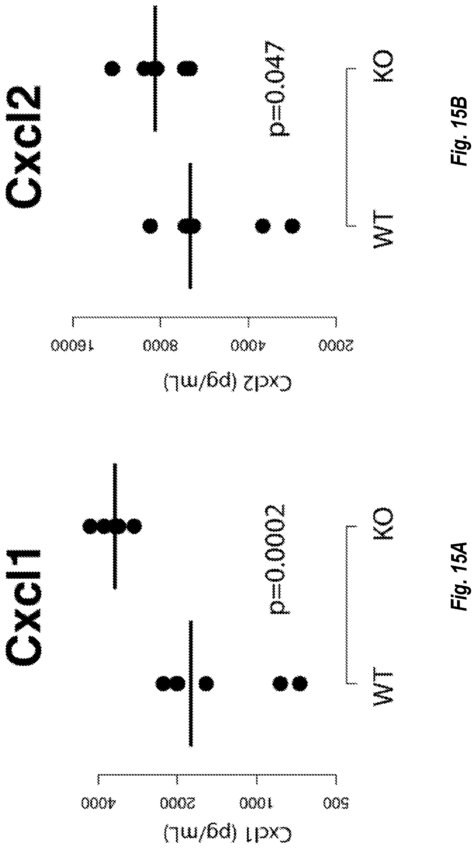

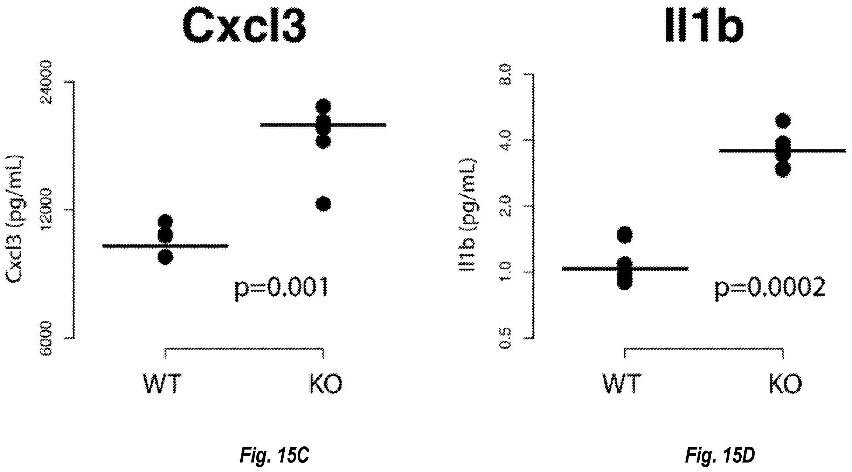

Figure 15 shows protein levels from an experiment where BMDM from *Dnmt3a*+/+ or *Dnmt3a*-/- mice were loaded with vehicle or 200 mg/dL LDL and proteins secreted into the media were assessed by ELISA. Shown are protein levels for IL-6, IL-1β, Cxcl1*,* Cxcl2, and Cxcl3.

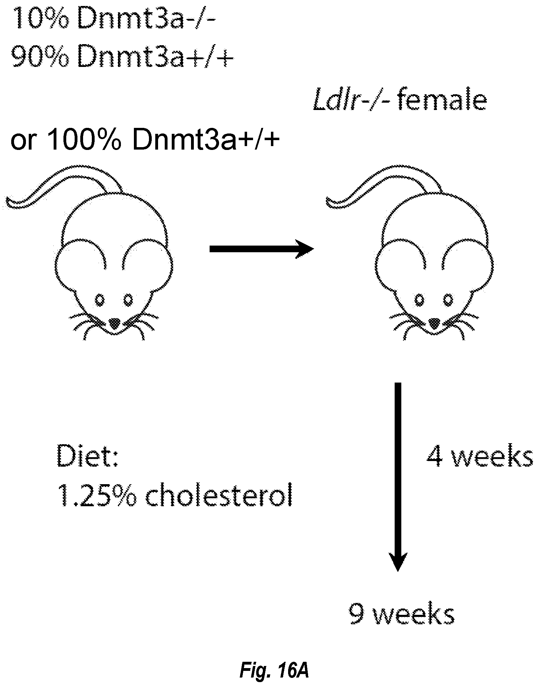

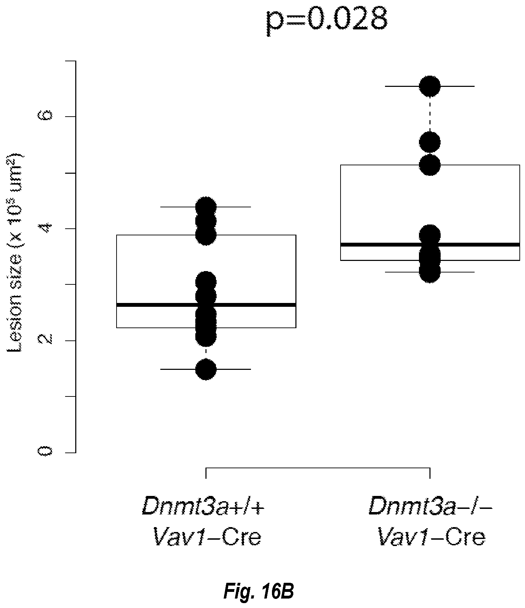

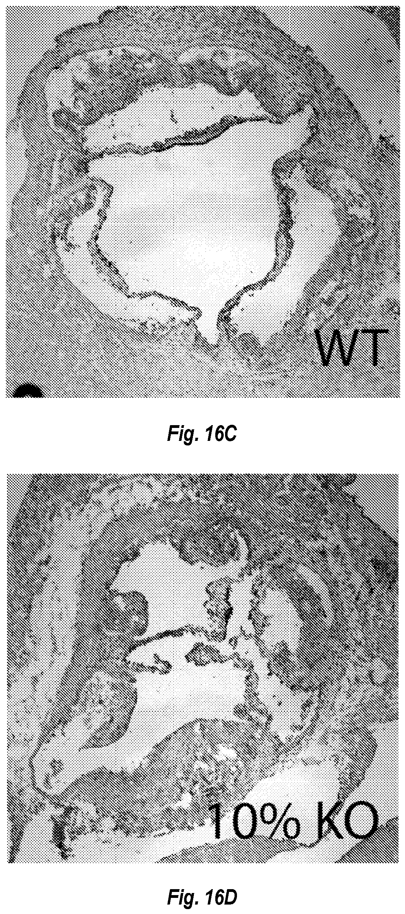

Figures 16A-16D show the size of aortic root lesions in wild-type and *Dnmta* deficient mice. A) Female *Ldlr*-/-*mice* were transplanted with either *Vav1-Cre Dnmt3a*+/+ (wild-type (WT)) or *Vav1-Cre Dnmt3a*-/- (knockout (KO)) bone marrow cells. Four weeks posttransplant, the mice were fed a high cholesterol diet for 9 weeks. At that time the mice were sacrificed and evaluated for aortic root lesions using histology. B) Atherosclerotic lesion size was measured in WT and *Dnmt3a* deficient mice. C-D) To detect atherosclerotic lesions tissues from mice were sectioned and stained with Oil Red O.

### DESCRIPTION OF THE EMBODIMENTS

### I. Methods of Treatment

The present application relates to an IL-8 inhibitor, IL-6 inhibitor, and/or IL-1β inhibitor for use in a method of treating atherosclerosis . Atherosclerosis is the leading cause of death in the United States; however, little is known about non-lipid risk factors in humans. This application relates to a mechanism behind the proposed causal association between somatic *TET*2 and/or DNMT3A mutations in blood cells, involvement of IL-8, IL-6, IL-1β, and atherosclerosis.

As *TET*2 and DNMT3A are enzymes that alters DNA methylation, it is likely that perturbing their function results in an abnormal epigenetic state. For example, *TET*2 converts 5-methylcytosine to 5-hydroxymethylcytosine, which ultimately leads to demethylation. As methylation at promoters and enhancers anti-correlates with gene expression and transcription factor binding, Applicants hypothesize that loss of *TET*2 function results in abnormal methylation of cis-regulatory elements for LXR/PPARG targets, reduced binding of transcription factors at these elements, and ultimately attenuated expression of the target genes. Alternatively, intermediates such as 5-hydroxymethylcytosine may be needed to repress the activity of pro-inflammatory transcription factors such as NF-kB. Likewise, DNMT3A catalyzes the transfer of methyl groups to specific CpG structures in DNA and is responsible for *de novo* DNA methylation. How and why these alterations may lead to atherosclerosis is unknown. As these epigenetic marks are known to influence gene expression, we hypothesize that they lead to increased expression of inflammatory genes in macrophages, reduced expression of cholesterol metabolism genes in macrophages, or both.

Thus, the present invention relates to an IL-8 inhibitor, IL-6 inhibitor, and/or IL-1β inhibitor for use in a method of treating atherosclerosis in a human subject comprising administering an effective amount of an IL-8 inhibitor, an IL-6 inhibitor, and/or an IL-1β inhibitor wherein the subject has a loss-of-function *TET*2 and/or DNMT3A mutation, thereby treating atherosclerosis.

In some embodiments, the presence of said mutations has been determined by sequencing at least a part of a genome comprising *TET*2 and/or *DNMT3A* of one or more cells in a blood sample of the subject

In addition to evaluating a subject's *TET*2 and/or DNMT3A status, IL-8, IL-6, and/or IL-1β status can be important to treatment. Thus, the use in a method of treating atherosclerosis in a human subject may, in some embodiments, comprise administering an effective amount of an IL-8 inhibitor, wherein the subject's plasma IL-8 level is at least 20 ng/mL thereby treating atherosclerosis. Other levels or sources of IL-8 levels may be employed, as described in Section I.H under.

For example, the subject further has an increased level of plasma IL-8, wherein the level of plasma IL-8 is at least 20 ng/mL. Other levels or sources of IL-8 levels may be employed, as described in Section I.H under.

### A. TET2 Mutations

Loss-of-function *TET*2 mutations, either alone or in combination with other indicators, may cause or be associated with atherosclerosis. One or more than one loss-of function *TET*2 mutation may be present in a somatic blood cell clone. A loss-of-function *TET*2 mutation may be a frameshift mutation, a nonsense mutation, a missense mutation, or a splice-site variant mutation.

In some embodiments, a loss-of-function mutation in *TET*2 leads to nonexpression or decreased expression of the TET2 protein or expression of a truncated or nonfunctional form of the TET2 protein. In some embodiments, a loss-of-function mutation in *TET*2 leads to a change in the structure or function of the TET2 protein. The NM_001127208 sequence is a representative wild-type sequence of *TET2.*

In some embodiments, the loss-of-function mutation in *TET*2 is a frameshift mutation. In some embodiments, the frameshift mutation is caused by insertion or deletion of a number of nucleotides that is not divisible by three. The loss-of-function mutation in *TET*2 may also be an insertion or deletion of a number of nucleotides that is divisible by three, wherein one or more amino acids are added or deleted from the wild-type TET2 amino acid sequence.

In some embodiments, the loss-of-function mutation in *TET*2 is a nonsense mutation. In some embodiments, the nonsense mutation is a point mutation (i.e., single nucleotide change) that results in a premature stop codon or a nonsense codon (i.e., a codon that does not code for an amino acid) in the transcribed RNA. In some embodiments, the nonsense mutation leads to a truncated, incomplete and/or nonfunctional TET2 protein.

In some embodiments, the loss-of function mutation in *TET*2 is a missense mutation. In some embodiments, the missense mutation is a point mutation that codes for a different amino acid than that found in the wildtype TET2 sequence. In some embodiments, the missense mutation is within nucleotides that encode one of the catalytic domains of the TET2 protein. In some embodiments, the missense mutation causes a change in amino acid from that encoded by the wildtype sequence at amino acids 1104-1481 or 1843-2002 of the TET2 protein.

In some embodiments, the mutation in *TET*2 results in an amino acid change in TET2 chosen from S145N, S282F, A308T, N312S, L346P, P399L, S460F, D666G, S817T, P941S, C1135Y, R1167T, I1175V, S1204C, R1214W, D1242R, D1242V, Y1245S, R1261C, R1261H, R1261L, F1287L, W1291R, K1299E, K1299N, R1302G, E1318G, P1367S, C1396W, L1398R, V1417F, G1869W, L1872P, I1873T, C1875R, H1881Q, H1881R, R1896M, R1896S, S1898F, V1900A, G1913D, A1919V, R1926H, P1941S, P1962L, R1966H, R1974M, and R2000K.

In some aspects, the human subject has clonal hematopoiesis of indeterminate potential (CHIP).

In some situations, the human subject has at least one loss-of-function *TET*2 mutation with a variant allele fraction of at least 2%, 5%, 10%, 13.5%, 15%, 20%, 25%, 27%, 30%.

Identification of a loss-of-function TET2 mutation may be detected in a patient's genome, including an exome. For example, sequencing may comprise whole exome sequencing (WES). The sequenced nucleic acid may include DNA.

### B. DNMT3A Mutations

Loss-of-function *DNMT3A* mutations, either alone or in combination with other indicators, may cause or be associated with atherosclerosis. One or more than one loss-of-function *DNMT3A* mutation may be present in a somatic blood cell clone. A loss-of-function *DNMT3A* mutation may be a frameshift mutation, a nonsense mutation, a missense mutation, or a splice-site variant mutation.

In some embodiments, a loss-of-function mutation in *DNMT3A* leads to nonexpression or decreased expression of the *DNMT3A* protein or expression of a truncated or nonfunctional form of the *DNMT3A* protein. In some embodiments, a loss-of-function mutation in *DNMT3A* leads to a change in the structure or function of the *DNMT3A* protein. The Q9Y6K1-1 sequence is a representative wild-type amino acid sequence of DNMT3A.

In some embodiments, the loss-of-function mutation in *DNMT3A* is a frameshift mutation. In some embodiments, the frameshift mutation is caused by insertion or deletion of a number of nucleotides that is not divisible by three. The loss-of-function mutation in *DNMT3A* may also be an insertion or deletion of a number of nucleotides that is divisible by three, wherein one or more amino acids are added or deleted from the wild-type *DNMT3A* amino acid sequence.

In some embodiments, the loss-of-function mutation in *DNMT3A* is a nonsense mutation. In some embodiments, the nonsense mutation is a point mutation (i.e., single nucleotide change) that results in a premature stop codon or a nonsense codon (i.e., a codon that does not code for an amino acid) in the transcribed RNA. In some embodiments, the nonsense mutation leads to a truncated, incomplete and/or nonfunctional *DNMT3A* protein.

In some embodiments, the loss-of-function mutation in *DNMT3A* is a missense mutation. In some embodiments, the missense mutation is a point mutation that codes for a different amino acid than that found in the wildtype *DNMT3A* sequence. In some embodiments, the missense mutation is within nucleotides that encode one of the catalytic domains of the DNMT3A protein. In some embodiments, the missense mutation causes a change in amino acid from that encoded by the wildtype sequence at amino acids 634-914 of the DNMT3A protein.

In some embodiments, the mutation in *DNMT3A* results in an amino acid change of I310N, Y365C, D529N, G532S, M548K, C549R, L648P, G699D, P700L, F732A, R749C, R771Q, V778G, N838D, R882C/H/P, F902S, P904L, or the absence of an amino acid corresponding to position 731. In some additional embodiments, the mutation in *DNMT3A* results in an amino acid change of F290I, F290C, V296M, P307S, P307R, R326H, R326L, R326C, R326S, G332R, G332E, V339A, V339M, V339G, L344Q, L344P, R366P, R366H, R366G, A368T, A368V, R379H, R379C, I407T, I407N, I407S, F414L, F414S, F414C, A462V, K468R, C497G, C497Y, Q527H, Q527P, Y533C, S535F, C537G, C537R, G543A, G543S, G543C, L547H, L547P, L547F, M548I, M548K, G550R, W581R, W581G, W581C, R604Q, R604W, R635W, R635Q, S638F, G646V, G646E, L653W, L653F, I655N, V657A, V657M, R659H, Y660C, V665G, V665L, M674V, R676W, R676Q, G685R, G685E, G685A, D686Y, D686G, R688H, G699R, G699S, G699D, P700L, P700S, P700R, P700Q, P700T, P700A, D702N, D702Y, V704M, V704G, I705F, I705T, I705S, I705N, G707D, G707V, C710S, C710Y, S714C, V716D, V716F, V716I, N717S, N717I, P718L, R720H, R720G, K721R, K721T, Y724C, R729Q, R729W, R729G, F731C, F731L, F731Y, F731I, F732del, F732C, F732S, F732L, E733G, E733A, F734L, F734C, Y735C, Y735N, Y735S, R736H, R736C, R736P, L737H, L737V, L737F, L737R, A741V, P742P, P743R, P743L, R749C, R749L, R749H, R749G, F751L, F751C, F752del, F752C, F752L, F752I, F752V, W753G, W753C, W753R, L754P, L754R, L754H, F755S, F755I, F755L, M761I, M761V, G762C, V763I, S770L, S770W, S770P, R771Q, F772I, F772V, L773R, L773V, E774K, E774D, E774G, I780T, D781G, R792H, W795C, W795L, G796D, G796V, N797Y, N797H, N797S, P799S, P799R, P799H, R803S, R803W, P804L, P804S, K826R, S828N, K829R, T835M, N838D, K841Q, Q842E, P849L, D857N, W860R, E863D, F868S, G869S, G869V, M880V, S881R, S881I, R882H, R882P, R882C, R882G, A884P, A884V, Q886R, L889P, L889R, G890D, G890R, G890S, V895M, P896L, V897G, V897D, R899L, R899H, R899C, L901R, L901H, P904L, F909C, P904Q, A910P, C911R, C911Y.

### C. IL-8 Inhibitors

Various approaches to inhibiting IL-8 function may be employed. IL-8 activity may be reduced using an IL-8 depleting drug or an IL-8 activity reducing drug. For instance, the IL-8 inhibitor may comprise an anti-IL-8 antibody or antigen binding fragment thereof. For example, an anti-IL-8 antibody or antigen binding fragment thereof may comprise HuMaxIL-8, HuMab-10F8, or an antigen binding fragment thereof, but others may be employed as well. Other IL-8 inhibitors include reparixin, 10Z-hymenialdisine, azelastine, celastrol, TNFRSF1A protein, TNFSF10 protein, TNFRSF10B protein, Ac-RRWWCR-NH₂ hexapeptide, and curcumin.

An IL-8 inhibitor may, in some instances, interfere with IL-8 binding or activity at its receptor, CXCR2, or the level or activity of CXCR2 itself. Thus, an IL-8 inhibitor may comprise an inhibitor of CXCR2. These include, but are not limited to, an anti-CXCR2 antibody or antigen binding fragment thereof. An IL-8 inhibitor may also comprise the CXCR2 inhibitor SB-332235 (GlaxoSmithKline) or the CXCR2 antagonist AZD5069.

### D. IL-6 Inhibitors

Various approaches to inhibiting IL-6 function may be employed. IL-6 activity may be reduced using an IL-6 depleting drug or an IL-6 activity reducing drug. For example, the IL-6 inhibitor may comprise an IL-6 antibody or antigen binding fragment thereof. For example, an IL-6 antibody or antigen binding fragment thereof may comprise siltuximab, olokizumab, elsilimomab, mAb 1339, BMS-945429 (also known as ALD518), sirukumab, CPSI-2364, ALX-0061, clazakizumab, ARGX-109, MEDI5117, FE301, FM101, or C326.

An IL-6 inhibitor may, in certain embodiments, interfere with IL-6 binding or activity at its receptor, IL-6R, or the level of activity of IL-6R itself. It may also interfere with binding or activity to gp130. As a transmembrane signal transduction protein, gp130 associates with the complex of IL-6 and IL-6R to produce downstream signals. Thus, an IL-6 inhibitor may comprise an inhibitor of IL-6R and/or gp130. These include, but are not limited to tocilizumab or sarilumab.

Other IL-6 inhibitors are disclosed in US20120294852 and include tamibarotene, all*-trans* retinoic acid (ATRA). Low-dose methotrexate has also been shown to improve IL-6 levels in patients with rheumatoid arthritis and may be useful for treatment of atherosclerosis, as is being tested in the CIRT study. Low-dose methotrexate may include doses of from 15 to 20 mg/week.

### E. IL-1β inhibitors

Various approaches to inhibiting IL-1β function may be used herein. IL-1β activity may be reduced using an IL-1β depleting drug or an IL-1β activity reducing drug. For example, the IL-1β inhibitor may comprise an IL-1β antibody or antigen binding fragment thereof. For example, an IL-1β antibody or antigen binding fragment thereof may comprise canakinumab. An IL-1 receptor antagonist, such as anakinra, can also serve as an IL-1β inhibitor.

In other instances, IL-1β inhibitor is an inhibitor of the IL-1β receptor. For example, an anti- IL-1β antibody or antigen binding fragment thereof may be used. Low-dose methotrexate (for example from 15 to 20 mg/week) has also been shown to improve IL-1β levels in patients with rheumatoid arthritis and may be useful for treatment of atherosclerosis, as is being tested in the CIRT study.

### F. Patient Profiles

In addition to having a loss-of function TET2 and/or *DNMT3A* mutation, a subject or patient benefitting herein may have one or more of the following patient profile characteristics. For example, the human subject may also exhibit one or more risk factors of being a smoker, having level of total cholesterol of at least 200 mg/dL, or having level of low-density lipoprotein (LDL) of at least 130 mg/dL.

In some aspects, the human subject has a total cholesterol of at least 240 mg/dL and/or an LDL of at least 160 mg/dL.

In some embodiments, the human subject has elevated hsCRP and optionally an hsCRP level of at least 2 mg/L.

### G. Combination Therapy

Other agents, treatments, or lifestyle changes may be employed along with the uses described in this application. These combination therapy approaches may increase benefit to the subjects with atherosclerosis.

In some embodiments, the use may include (i) administering an effective amount of cholesterol-lowering medication and/or (ii) prescribing exercise, cessation of smoking, diet modification, and/or stress reduction to the subject.

### 1. Cholesterol-Lowering Medications

Various cholesterol-lowering medications may be employed in combination with the IL-8, IL-6, and/or IL-1β inhibitor. For example, cholesterol-lowering medication for combination therapy may include, but is not limited to, comprises at least one PCSK9 inhibitor, at least one statin, at least one selective cholesterol absorption inhibitor, at least one resin, at least one lipid-lowering therapy, at least one CETP inhibitor, at least one pantothenic acid derivative, at least one microsomal triglyceride transfer protein (MTP) inhibitor, at least one adenosine triphosphate-binding cassette transporter A1 (ABCA1)-promoter, aspirin, estrogen, and/or at least one lipoprotein complex. Other agents may also be employed.

### a) PCSK9 inhibitor

Various PCSK9 inhibitors may be used, including but not limited to a PCSK9 antibody or antigen binding fragment thereof. For example, specific PCSK9 antibodies, as well as antigen binding fragments of those antibodies, are disclosed in US 2015/0140002A1. Specific PCSK9 antibodies include evolocumab, alirocumab, bococizumab, LGT209, RG7652, or LY3015014.

PCSK9 inhibitors also include RNAi therapeutic agents that inhibit the synthesis of PCSK9, such as inclisiran. PCSK9 inhibitors also include an antisense RNA that inhibits the synthesis of PCSK9, such as ISIS-405879/BMS-844421.

PCSK9 inhibitors also include a PCSK9-targeting vaccine, such as AT04A or AT06A.

PCSK9 inhibitors further include a polypeptide that binds PCSK9 (such as adnectin) or a locked nucleic acid targeting PCSK9 (such as SPC5001).

### 2. Statins (also known as HMG CoA reductase inhibitors)

Statins, also known as HMG CoA reductase inhibitors, are also included in the class of cholesterol-lowering medication. This class of drugs works in the liver to prevent the formation of cholesterol, thus lowering the amount of cholesterol circulating in the blood. Statins are most effective at lowering LDL cholesterol, but also have modest effects on lowering triglycerides and raising HDL cholesterol.

Exemplary statins include, but are not limited to, atorvastatin (Lipitor^{®}), fluvastatin (Lescol^{®}), lovastatin (Mevacor^{®}, Altoprev^{™}), pravastatin (Pravachol^{®}), rosuvastatin (rosuvastatin calcium, Crestor^{®}), simvastatin (Zocor^{®}), and pitavastatin. Statins are also found in the combination medications Advicor^{®} (lovastatin + niacin), Caduet^{®} (atorvastatin + amlodipine), and Vytorin^{™} (simvastatin + ezetimibe).

### 3. Selective cholesterol absorption inhibitors

Selective cholesterol absorption inhibitors may also be used as cholesterol-lowering medication. This relatively new class of cholesterol-lowering medications works by preventing the absorption of cholesterol from the intestine. Selective cholesterol absorption inhibitors are most effective at lowering LDL cholesterol, but may also have modest effects on lowering triglycerides and raising HDL cholesterol.

An example of a selective cholesterol absorption inhibitor includes ezetimibe (Zetia^{®}).

### 4. Resins

Cholesterol-lowering medication also includes resins (i.e., bile acid sequesterant or bile acid-binding drugs or bile-acid resin). This class of LDL-lowering drugs works in the intestines by promoting increased disposal of cholesterol. The medications bind to bile, which then cannot be used in digestion, and the patient's body responds by making more bile and using stores of cholesterol. Resins may include, but are not limited to, cholestyramine (Questran^{®}, Questran^{®} Light, Prevalite^{®}, Locholest^{®}, Locholest^{®} Light), Colestipol (Colestid^{®}), and Colesevelam HCl (WelChol^{®}).

### 5. Lipid-Lowering Therapies

Cholesterol-lowering medication further includes lipid-lowering therapies, such as at least one fibrate, niacin, and at least one omega-3 fatty acid. Fibrates are best at lowering triglycerides and in some cases increasing HDL levels, but are not as effective in lowering LDL cholesterol. Fibrates include gemfibrozil (Lopid^{®}), fenofibrate (Antara^{®}, Lofibra^{®}, Tricor^{®}, and Triglide^{™}), and clofibrate (Atromid-S). Niacin (nicotinic acid) functions in the liver by affecting the production of blood fats.

Omega-3 fatty acids help decrease triglyceride secretion and facilitate triglyceride clearance. Omega-3 fatty acids include omega-3 fatty acid ethyl esters are derived from fish oils that may be chemically changed and purified. Omega-3 fatty acid ethyl esters available in the U.S. include Lovaza^{®} (omega-3-acid ethyl esters) and Vascepa^{™} (icosapent ethyl). Omega-3 fatty acids also include omega-3 polyunsaturated fatty acids, including but not limited to marine-derived omega-3 polyunsaturated fatty acids (PUFA).

### 6. CETP Inhibitor

Cholesterol-lowering medications include at least one CETP inhibitor. These medications inhibit cholesterylester transfer protein (CETP) and are intended to improve blood lipid levels by increasing HDL, lowering LDL, and by reversing the transport of cholesterol. These medications include anacetrapib and obicetrapib.

### 7. Microsomal Triglyceride Transfer Protein (MTP) Inhibitors

Cholesterol-lowering medications also include microsomal triglyceride transfer protein (MTP) inhibitors, which inhibit very-low-density lipoprotein production in the liver and chylomicron inhibition in the intestine. In some instances, the MTP inhibitor is chosen from at least one of (i) a small molecule that inhibits function of MTP, (ii) an RNAi therapeutic agent that inhibits the synthesis of MTP, and (iii) an antisense RNA that inhibits synthesis of MTP. For example, the small molecule that inhibits function of MTP may be chosen from at least one of lomitapide, JTT-130, Slx-4090, and dirlotapide.

### 8. Adenosine Triphosphate-Binding Cassette Transporter A1 (ABCA1)-Promoting Drugs

Cholesterol-lowering medications further include at least one adenosine triphosphate-binding cassette transporter A1 (ABCA1)-promoter. These may be chosen from at least one of (i) an apoA-1 mimetic peptide, (ii) a full-length apoA-1, and (iii) a reconstituted HDL. For instance, the apoA-1 mimetic peptide may be FAMP type 5 (FAMP5). In some instances, the full-length apoA-1 may be ApoA-1-Milano or ETC-216.

### 9. Lipoprotein Complex

Other cholesterol lowering medications at least one lipoprotein complex. A lipoprotein complex may be CER-001, CSL-111, CSL-112, and ETC-216. A lipoprotein complex may be chosen from at least one of apolipoprotein or apolipoprotein peptide mimic. For example, (i) apolipoprotein is chosen from at least one of ApoA-I, ApoA-II, ApoA-IV, and ApoE and/or (ii) the peptide mimetic is chosen from at least one of ApoA-I, ApoA-II, ApoA-IV, and ApoE peptide mimic.

### H. Lifestyle Modifications

Along with use of an IL-8, IL-6, and/or IL-1β inhibitor and optionally in combination with a cholesterol-lowering medication as described herein, lifestyle modification may be prescribed to the subject. Exercise may be prescribed to the subject, for example for at least 3, 4, 5, 6, or 7 days a week. Exercise may include cardiovascular conditioning exercise and/or strength training exercise. In some embodiments, the subject performs the prescribed exercise as directed.

The use may also include prescribing cessation of smoking and/or the subject stopping smoking or reducing smoking levels. The use may also comprise administering a medication to support smoking cessation (including medication chosen from at least one of nicotine replacement therapy, antidepressants (such as bupropion, nortriptyline, or an SSRI), varenicline, and clonidine).

The use may also comprise a prescription for diet modification and/or the subject modifying his or her diet. Diet modification may include at least one of a reduction in fat consumption, a reduction in cholesterol consumption, a reduction in sugar consumption, an increase in fruit and/or vegetable consumption, an increase in omega fatty acids, and/or reduction of alcohol consumption. Weight loss may be accomplished through a variety of factors including medications to promote weight loss, including celastrol.

The use may also include prescription of stress reduction and/or the subject reducing his or her stress levels, including but not limited to at least one of relaxation techniques, mediation, breathing exercises, exercise, and/or anger management. Stress reduction includes managing constant levels of stress more effectively.

The use may also include prescribing psychiatric medication and/or the subject taking psychiatric medication, such as but not limited to anti-anxiety medication and/or anti-depressant medication. Such medications may include at least one of citalopram, escitalopram, fluoxetine, paroxetine, sertraline, duloxetine, venlafaxine, imipramine, hydroxyzine, propanolol, gabapentin, and pregabalin. The use may also comprise prescribing or conducting psychological counseling.

In some embodiments, the increased level of IL-8 is an increased level of plasma IL-8. The increased level of plasma IL-8 may be at least about 20 ng/mL. Or it may be at least about 25, 30, 40, 45, 50, 55, 60, 65, 70, 75, or 80 ng/mL. In some situations, the increased level of IL-8, IL-6, and/or IL-1β may be an increased level of IL-8, IL-6, and/or IL-1β RNA. In some situations, the increased level of IL-8, IL-6, and/or IL-1β may be an increased level of IL-8, IL-6, and/or IL-1β in cells. The increased levels of IL-8, IL-6, and/or IL-1β may be about 20%, 30%, 40%, 50%, 60%, 70%, 80%, 90%, or 100% increased over baseline levels for normal subjects.

In some the use includes both evaluating IL-8, IL-6, and/or IL-1β levels and determining whether the subject has a loss-of-function *TET*2 and/or DNMT3A mutation. Such additional steps could comprise detecting whether the sample contains at least one loss-of-function *TET*2 and/or DNMT3A mutation with a probe of sufficient length and composition to detect a loss-of-function *TET*2 and/or DNMT3A mutation; and diagnosing the subject as having atherosclerosis when at least one loss-of-function *TET*2 and/or DNMT3A mutation is detected.

Therefore, in some embodiments use may involve detecting at least one loss-of-function *TET*2 and/or DNMT3A mutation along with an increase in plasma level of IL-8, IL-6, and/or IL-1β in a human subject by: (a) obtaining a nucleic acid sample from the subject; (b) detecting whether the sample contains at least one *TET*2 and/or DNMT3A mutation with a probe of sufficient length and composition to detect a *TET*2 and/or DNMT3A mutation; (c) obtaining a plasma sample from the subject; and (d) determining whether the subject has an increased level of IL-8, IL-6, and/or IL-1β, as further described in paragraph [00102] over.

### II. Definitions and Supporting Information

"Atherosclerosis" means any form of hardening and/or narrowing of the arteries. This includes any amount of plaque build-up in the artery wall. Plaque is made up of cholesterol, fatty substances, cellular waste products, calcium, and fibrin. Atherosclerosis includes formation of early plaques before diagnosis would usually occur. Plaques have been shown to form in much younger adults than those individuals generally diagnosed with atherosclerosis.

"Loss-of-function mutation" means any inactivating mutation resulting in the gene product having less or no function (partially or wholly inactivated). A loss-of-function mutation may result in the mutant form having no activity or 30%, 40%, 50%, 60%, 70%, 80%, 90%, 95%, or higher percentage reduction in activity.

The term "antibody" is used herein in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. In some embodiments, an antibody may be a chimeric antibody, a humanized antibody, or a human antibody.

The term antibody includes, but is not limited to, fragments that are capable of binding to an antigen, such as Fv, single-chain Fv (scFv), Fab, Fab', di-scFv, sdAb (single domain antibody) and (Fab')₂ (including a chemically linked F(ab')₂). The term antibody also includes, but is not limited to, chimeric antibodies, humanized antibodies, and antibodies of various species such as mouse, human, cynomolgus monkey, etc. Antibody fragments also include either orientation of single chain scFvs, tandem di-scFv, diabodies, tandem tri-sdcFv, minibodies, etc. Antibody fragments also include nanobodies (sdAb, an antibody having a single, monomeric domain, such as a pair of variable domains of heavy chains, without a light chain). An antibody fragment can be referred to as being a specific species in some embodiments (for example, human scFv or a mouse scFv).

The term "antisense oligonucleotide" refers to a single-stranded oligonucleotide comprising 8 to 50 monomeric units and having a nucleobase sequence that permits hybridization to a corresponding segment of a target nucleic acid. An antisense oligonucleotide may comprise natural, non-natural, and/or modified nucleosides and/or internucleoside linkages.

The term "peptide" as used herein refers to a molecule formed by linking at least two, and up to 300, amino acids by amide bonds. The amino acids of a peptide may be natural, non-natural, and/or modified amino acids. In some embodiments, a peptide comprises 2-200 amino acids, or 2-100 amino acids, or 2-50 amino acids, or 2-30 amino acids, or 10-300 amino acids, or 10-200 amino acids, or 10-100 amino acids, or 10-50 amino acids.

A "reference" as used herein, refers to any sample, standard, or level that is used for comparison purposes. A reference may be obtained from a healthy and/or nondiseased sample. In some examples, a reference may be obtained from an untreated sample, or may be a sample from the subject prior to treatment. In some examples, a reference is obtained from one or more healthy individuals who are not the subject or patient.

As used herein "diagnosis" or "identifying a patient having" refers to a process of determining if an individual is afflicted with, or has a genetic predisposition to develop, atherosclerosis.

As used herein, a "companion diagnostic" refers to a diagnostic method and or reagent that is used to identify subjects susceptible to treatment with a particular treatment or to monitor treatment and/or to identify an effective dosage for a subject or subgroup or other group of subjects. For purposes herein, a companion diagnostic refers to reagents, such as DNA isolation and sequencing reagents, that are used to detect somatic mutations in a sample. The companion diagnostic refers to the reagents and also to the test(s) that is/are performed with the reagent.

The terms "treat," treating," "treatment," and the like refer to reducing or ameliorating atherosclerosis or symptoms associated therewith. It will be appreciated that, although not precluded, treating atherosclerosis or the risk of developing atherosclerosis does not require that the disease or the risk be completely eliminated.

In the context this application, a "treatment" is a procedure which alleviates or reduces the negative consequences of atherosclerosis. Any treatments or potential treatments can be used in the context herein. A treatment is not necessarily curative, and may reduce the effect of atherosclerosis by a certain percentage over an untreated subject. The percentage reduction or diminution can be from 10% up to 20, 30, 40, 50, 60, 70, 80, 90, 95, 99 or 100%. "Treatment" also includes methods or preventing, inhibiting the development, or reducing the risk of atherosclerosis, unless otherwise stated.

In connection with the uses as described herein, methods of treatment may be personalized medicine procedures, in which the DNA of an individual is analyzed to provide guidance on the appropriate therapy for that specific individual. The methods may provide guidance as to whether treatment is necessary, as well as revealing progress of the treatment and guiding the requirement for further treatment of the individual.

As used herein, "inhibiting the development of," "reducing the risk of," "prevent," "preventing," and the like refer to reducing the probability of developing atherosclerosis in a patient who may not have atherosclerosis, but may have a genetic predisposition to developing atherosclerosis. As used herein, "at risk," "susceptible to," or "having a genetic predisposition to," refers to having a propensity to develop atherosclerosis. For example, a patient having a genetic mutation in a gene associated with atherosclerosis has increased risk (e.g., "higher predisposition") of developing the disease relative to a control subject having a "lower predisposition" (e.g., a patient without a *TET*2 mutation and/or increased IL-8 levels).

As used herein, "reduces," "reducing," "inhibit," or "inhibiting," may mean a negative alteration of at least 10%, 15%, 25%, 50%, 75%, or 100%.

As used herein, "increases" or "increasing" may mean a positive alteration of at least 10%, 15%, 25%, 50%, 75%, or 100%.

A "therapeutically effective amount" refers to the amount of a compound required to improve, inhibit, or ameliorate a condition of a patient, or a symptom of a disease, in a clinically relevant manner. Any improvement in the patient is considered sufficient to achieve treatment. A sufficient amount of an active compound used for the treatment of atherosclerosis varies depending upon the manner of administration, the age, body weight, genotype, and general health of the patient. Ultimately, the prescribers or researchers will decide the appropriate amount and dosage regimen. Such determinations are routine to one of ordinary skill in the art.

As used herein "patient" or "subject" refers to any human being receiving or who may receive medical treatment. These terms also include mammals. Mammals include, but are not limited to, domesticated animals (e.g., cows, sheep, cats, dogs, and horses), primates (e.g., humans and non-human primates such as monkeys), rabbits, and rodents (e.g., mice and rats).

A "somatic mutation" refers to a change in the genetic structure that is not inherited from a parent, and also not passed to offspring.

Administration of medicaments in connection with the uses herein may be by any suitable means that results in a compound concentration that is effective for treating or inhibiting (e.g., by delaying) the development of atherosclerosis. The compound is admixed with a suitable carrier substance, e.g., a pharmaceutically acceptable excipient that preserves the therapeutic properties of the compound with which it is administered. One exemplary pharmaceutically acceptable excipient is physiological saline. The suitable carrier substance is generally present in an amount of 1-95% by weight of the total weight of the medicament. The medicament may be provided in a dosage form that is suitable for oral, rectal, intravenous, intramuscular, subcutaneous, inhalation, nasal, topical or transdermal, vaginal, or ophthalmic administration. Thus, the medicament may be in form of, e.g., tablets, capsules, pills, powders, granulates, suspensions, emulsions, solutions, gels including hydrogels, pastes, ointments, creams, plasters, drenches, delivery devices, suppositories, enemas, injectables, implants, sprays, or aerosols.

In order to determine the genotype of a patient according to the uses, it may be necessary to obtain a sample of genomic DNA from that patient. That sample of genomic DNA may be obtained from a sample of tissue or cells taken from that patient.

The tissue sample may comprise but is not limited to hair (including roots), skin, buccal swabs, blood, saliva, or plasma, including but not limited to cell-free DNA from plasma. The tissue sample may be marked with an identifying number or other indicia that relates the sample to the individual patient from which the sample was taken. The identity of the sample advantageously remains constant throughout, thereby guaranteeing the integrity and continuity of the sample during extraction and analysis. Alternatively, the indicia may be changed in a regular fashion that ensures that the data, and any other associated data, can be related back to the patient from whom the data was obtained. The amount/size of sample required is known to those skilled in the art.

Generally, the tissue sample may be placed in a container that is labeled using a numbering system bearing a code corresponding to the patient. Accordingly, the genotype of a particular patient is easily traceable.

In one embodiment, a sampling device and/or container may be supplied to the physician. The sampling device advantageously takes a consistent and reproducible sample from individual patients while simultaneously avoiding any cross-contamination of tissue. Accordingly, the size and volume of sample tissues derived from individual patients would be consistent.

Accordingly, a sample of DNA may be obtained from the tissue sample of the patient of interest. Whatever source of cells or tissue is used, a sufficient amount of cells must be obtained to provide a sufficient amount of DNA for analysis. This amount will be known or readily determinable by those skilled in the art.

DNA may be isolated from the tissue/cells by techniques known to those skilled in the art (see, e.g., U.S. Pat. Nos. 6,548,256 and 5,989,431, Hirota et al., Jinrui Idengaku Zasshi. September 1989; 34(3):217-23 and John et al., Nucleic Acids Res. Jan. 25. 1991; 19(2):408). For example, high molecular weight DNA may be purified from cells or tissue using proteinase K extraction and ethanol precipitation. DNA may be extracted from a patient specimen using any other suitable methods known in the art.

The genotype of a given patient of interest may be determined by analyzing the DNA from the patient, in order to identify a patient carrying specific somatic loss-of-function mutations that are associated with developing atherosclerosis.

There are many methods known in the art for determining the genotype of a patient and for identifying or analyzing whether a given DNA sample contains a particular somatic mutation. Any method for determining genotype can be used. Such methods include, but are not limited to, amplimer sequencing, DNA sequencing, fluorescence spectroscopy, fluorescence resonance energy transfer (or "FRET")-based hybridization analysis, high throughput screening, mass spectroscopy, nucleic acid hybridization, polymerase chain reaction (PCR), RFLP analysis and size chromatography (e.g., capillary or gel chromatography), all of which are well known to one of skill in the art.

The methods in connection with the uses herein, such as whole exome sequencing and targeted amplicon sequencing, have commercial applications in diagnostic kits for the detection of the somatic mutations in patients. A test kit may comprise any of the materials necessary for whole exome sequencing and targeted amplicon sequencing, for example. In some embodiments, a companion diagnostic may comprise testing for *TET*2 and/or *DNMT3A* mutations. The kit further comprises additional means, such as reagents, for detecting or measuring *TET*2 and/or *DNMT3A* sequences, and also ideally a positive and negative control.

The uses further encompass probes that are immobilized on a solid or flexible support, such as paper, nylon or other type of membrane, filter, chip, glass slide, microchips, microbeads, or any other such matrix, all of which are within the scope of this application. The probe of this form is now called a "DNA chip". These DNA chips can be used for analyzing the somatic mutations. Arrays or microarrays of nucleic acid molecules that are based on one or more of the sequences described herein are included. As used herein "arrays" or "microarrays" refers to an array of distinct polynucleotides or oligonucleotides synthesized on a solid or flexible support, such as paper, nylon or other type of membrane, filter, chip, glass slide, or any other suitable solid support. In one embodiment, the microarray is prepared and used according to the methods and devices described in U.S. Pat. Nos. 5,446,603; 5,545,531; 5,807,522; 5,837,832; 5,874,219; 6,114,122; 6,238,910; 6,365,418; 6,410,229; 6,420,114; 6,432,696; 6,475,808 and 6,489,159 and PCT Publication No. WO 01/45843 A2.

Sequence identity or homology may be determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990;87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988;85: 2444-2448.

WU-BLAST (Washington University BLAST) version 2.0 software may be used. WU-BLAST version 2.0 executable programs for several UNIX platforms can be downloaded from the FTP site for Blast at the Washington University in St. Louis website. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993; Nature Genetics 3: 266-272; Karlin & Altschul, 1993; Proc. Natl. Acad. Sci. USA 90: 5873-5877).

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology" or "identity", for instance, with respect to a nucleotide or amino acid sequence, can indicate a quantitative measure of homology between two sequences. The percent sequence homology can be calculated as (N_{ref}-N_{dif})^{∗} 100/- N_{ref}, wherein N_{dif} is the total number of non-identical residues in the two sequences when aligned and wherein N_{ref} is the number of residues in one of the sequences. Hence, the DNA sequence AGTCAGTC will have a sequence identity of 75% with the sequence AATCAATC (N N_{ref} =8; N N_{dif} =2). "Homology" or "identity" can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur & Lipman, Proc Natl Acad Sci USA 1983;80:726), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics.TM. Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the application and can be derived from DNA sequences, by thymidine (T) in the DNA sequence being considered equal to uracil (U) in RNA sequences. Without undue experimentation, the skilled artisan can consult with many other programs or references for determining percent homology.

In some embodiments, the step of assaying is chosen from: restriction fragment length polymorphism (RFLP) analysis, minisequencing, MALD-TOF, SINE, heteroduplex analysis, single strand conformational polymorphism (SSCP), denaturing gradient gel electrophoresis (DGGE) and temperature gradient gel electrophoresis (TGGE).

### EXAMPLES

### Example 1. Clonal hematopoiesis associates with coronary heart disease

Whole exome sequencing was used to detect the presence of clonal hematopoiesis of indeterminate potential (CHIP) in peripheral blood cells in case-control cohorts with coronary heart disease. Individuals from the Malmö Diet and Cancer Study and the BioImage Study were newly sequenced. These data will be deposited in dbGaP in accordance with institutional procedures. The other sequence data were obtained from publicly available sources as listed below.

The MDC study is a community-based, prospective observational study of ~30,000 participants drawn from ~230,000 residents of Malmö, Sweden who were enrolled between 1991 and 1996. From this cohort, 6,103 participants were randomly selected to participate in the cardiovascular cohort *(see* Kathiresan S et al., N Engl J Med 358:1240-9 (2008)). Among these participants, those who sustained incident major adverse cardiovascular disease events, including fatal or non-fatal myocardial infarction, coronary artery bypass grafting, or percutaneous coronary intervention were selected for whole exome sequencing. DNA was obtained from granulocytes in peripheral blood samples at the time of study enrollment and individuals were followed for the development of coronary heart disease.

The BioImage study (NCT00738725) is a multi-ethnic, observational study aimed at characterizing subclinical atherosclerosis in 6,699 US adults (55-80 years at baseline, 2008-2009) at risk for, but without, clinical cardiovascular disease. From this cohort, those of European ancestry who sustained incident major adverse cardiovascular disease events, including fatal or non-fatal myocardial infarction, coronary artery bypass grafting, or percutaneous coronary were selected intervention for whole exome sequencing *(see* Baber U et al., J Am Coll Cardiol 65:1065-74 (2015)). DNA was obtained from whole blood samples at the time of study enrollment and individuals were followed for the development of coronary heart disease.

The Atherosclerosis, Thrombosis, and Vascular Biology (ATVB) Study is a nationwide case-control study of early-onset myocardial infarction involving 125 Italian coronary care units *(see* ATVB Italian Study Group 2003). Cases were men and women hospitalized for a first myocardial infarction before the age of 45 years who underwent coronary angiography at the time of index hospitalization. Acute myocardial infarction was defined as resting chest pain lasting >30 minutes accompanied by ST-segment elevation evolving into pathological Q waves with total creatinine kinase or MB fraction levels of >2X the upper normal limit of normal. Controls were selected in a 1:1 fashion free of a history of thromboembolic disease and were matched by age, sex, and geographical origin. All participants of this study underwent whole exome sequencing *(see* Do R et al., Nature 518:102-6 (2015)). DNA was obtained from whole blood samples obtained at the time of index presentation. Data for ATVB are available in dbGap at /www.ncbi.nlm.nih.gov/projects/gap/cgi-bin/study.cgi?study_id= phs000814.v1.p1.

The Jackson Heart Study (JHS) is a large population-based cohort of African-Americans in Jackson, Mississippi (see Sempos CT et al., Am J Med Sci 317:142-6 (1999)), who were sequenced and analyzed in a prior study (see (*see* Jaiswal S et al., N Eng J Med 371:2488-98 (2014)). A total of 2,408 subjects from JHS were exome sequenced and analyzed of the ~3,400 consented for genetic studies (-70%). DNA was obtained from whole blood samples at the time of study enrollment and individuals were followed for the development of coronary heart disease.

Data for JHS are available from T2D-GENES and HEART-GO in dbGaP: (www.ncbi.nlm.nih.gov/projects/gap/cgi-bin/study.cgi?study_id=phs001098.v1.p1) and (www.ncbi.nlm.nih.gov/projects/gap/cgi-bin/study.cgi?study_id=phs000402.v3.p1).

The Finland United States Study of NIDDM Genetics (FUSION) is a case-control cohort for type 2 diabetes analyzed in a previous study (see Valle T et al., Diabetes Care 21:949-58 (1998)). Cases were type 2 diabetics in Finland and controls were matched by birth province and body mass index. A total of 474 type 2 diabetes cases and 470 controls were exome sequenced and analyzed. DNA was obtained from whole blood samples at the time of study enrollment and individuals were followed for the development of coronary heart disease.

The Framingham Heart Study (FHS) is a prospective, multi-generational, longitudinal study of European Americans established in 1948 in Framingham, MA. Participants in this analysis were from the FHS Offspring (children and spouses of the Original cohort, n=362), and Generation 3 (children of the Offspring n=246) cohorts (*see* Feinleib M et al., Prev Med 4:518-25 (1975) and Splansky Am J Epidemiol 165:1328-35 (2007)). Offspring participants were examined every 4-8 years, for a total of 8 exams. Generation 3 participants were examined twice. Samples were sequenced as part of the National Heart, Lung and Blood Institute (NHLBI) Exome Sequencing Project *(see* Tennessen JA et al., Science 337:64-9 (2012)) (DbGaP accession # phs000651) and Cohorts for Heart and Aging Research in Genomic Epidemiology Studies *(see* Psaty BM et al., Circ Cardiovasc Genet 2:73-80 (2009)) (DbGaP accession # phs000401). Samples in the Exome Sequencing Project were sequenced at the Broad Institute and University of Washington. Samples in the Cohorts for Heart and Aging Research in Genomic Epidemiology Studies were sequenced at the Baylor College of Medicine. Samples in these two analyses were initially selected for exome sequencing as cases or controls for studies of myocardial infarction, blood pressure, LDL cholesterol, stroke, atrial fibrillation as well as randomly selected. For this analysis, all available FHS exomes on dbGaP derived from peripheral blood samples were utilized. Exomes derived from lymphoblast cell lines were excluded. DNA was obtained from whole blood samples at the time of study enrollment and individuals were followed for the development of coronary heart disease.

Data for FHS are available from CHARGE and GO-ESP in dbGaP: (www.ncbi.nlm.nih.gov/projects/gap/cgi-bin/study.cgi?study_id=phs000651.v9.p10) and (www.ncbi.nlm.nih.gov/projects/gap/cgi-bin/study.cgi?study_id=phs000401.vl2.p10).

DNA was obtained from individual cohorts and further processed at the Broad Institute of MIT and Harvard. For BioImage, and MDC phase II DNA libraries were bar coded using the Illumina index read strategy, exon capture was performed using Illumina Rapid Capture Exome (ICE) kit, and sequencing was performed by Illumina HiSeq4000. For ATVB, JHS, and FUSION, DNA libraries were bar coded using the Illumina index read strategy, exon capture was performed using Agilent Sure-Select Human All Exon v2.0, and sequencing was performed by Illumina HiSeq2000.

Sequence data were aligned by the Picard (www.picard.sourceforge.net) pipeline using reference genome hg19 with the BWA algorithm *(see* Li H et al., Bioinformatics 25:1754-60 (2009)) and processed with the Genome Analysis Toolkit (GATK) to recalibrate base-quality scores and perform local realignment around known insertions and deletions (indels) (*see* DePristo MA et al., Nature Genetics 43:491-8 (2011). BAM files were then analyzed for single nucleotide variants using MuTect (www.broadinstitute.org/cancer/cga/mutect) with Oxo-G filtering (www.broadinstitute.org/cancer/cga/dtoxog) and for indels using Indelocator (www.broadinstitute.org/cancer/cga/indelocator), followed by annotation using Oncotator (www.broadinstitute.org/cancer/cga/oncotator/) *(see* Cibulskis K et al., Nat Biotechnol 31:213-9 (2013)). All MuTect and Indelocator analyses were performed using the Firehose pipeline (www.broadinstitute.org/cancer/cga/Firehose) at the Broad Institute.

A nested case-control study design was utilized from two prospective study cohorts, BioImage and Malmö Diet and Cancer (MDC). BioImage was selected because of the cohort's enrichment in aged and high cardiovascular risk participants *(see* Muntendam P, et al. Am Heart J 160:49-57 (2010)), while MDC was selected because of its longer follow-up period and extensive phenotypic data *(see* Berglund G, et al., J Intern Med 1993;233:45-51(1993)).

**Table 1** shows the baseline characteristics for each cohort. After excluding those with prevalent events, cases were defined as those having myocardial infarction or coronary revascularization procedures incident to the time of DNA collection and were matched to event-free controls by age (+/- 2 years), sex, type 2 diabetes status, and smoking history.

**Table 1: Baseline characteristics of subjects in BioImage and MDC**

| | **Biolmage** | | | **MDC** | |
|---|---|---|---|---|---|
| | **Cases** | **Controls** | | **Cases** | **Controls** |
| **Number of individuals** | 113 | 257 | | 320 | 320 |
| *No CHIP* | 94 | 232 | | 299 | 308 |
| *CHIP* | 19 | 25 | | 21 | 12 |

| **Age (years), median** | 70 | 70 | | 60 | 60 |
|---|---|---|---|---|---|
| *No CHIP* | 69 | 70 | | 64 | 60 |
| *CHIP* | 71 | 73 | | 63 | 60 |

| **Female sex, n (Percent)** | 41 (36.2) | 101 (39.3) | | 125 (39.1) | 125 (39.1) |
|---|---|---|---|---|---|
| *No CHIP* | 32 (24.0) | 92 (39.7) | | 116 (38.8) | 121 (39.3) |
| *CHIP* | 9 (47.4) | 9 (36.0} | | 9 (42.3) | 4 (33.3) |

| **Smoker, n (Percent)** | 19 (16.8) | 41 (15.9} | | 104 (32.5) | 104 (32.5) |
|---|---|---|---|---|---|
| *No CHIP* | 16 (17.0) | 38 (16.3) | | 95 (31.8) | 98 (31.8) |
| *CHIP* | 3 (15.8) | 3 (12) | | 9 (42.9) | 6 (50.0) |

| **Has hypertension, n (Percent)** | 98 (36.7) | 194 (75.5) | | 246 (76.9) | 196 (61.3) |
|---|---|---|---|---|---|
| *No CHIP* | 83 (88.3) | 175 (75.4) | | 230 (76.9) | 189 (61.4) |
| *CHIP* | 15 (78.9) | 19 (76.0) | | 16 (76.2) | 5 (41.7) |

| **Has T2D, n (Percent)** | 29 (25.7) | 63 (24.5) | | 49 (15.3) | 49(15.3) |
|---|---|---|---|---|---|
| *No CHIP* | 26 (27.6) | 53 (22.8) | | 44 (14.7) | 49(15.9) |
| *CHIP* | 3 (15.8) | 10 (40.0) | | 5/23.8) | 0(0.0) |

| **Totol cholesterol (mg/dL), mean (SD)** | 213 (35) | 208 (36) | | 275 (171) | 261 (168) |
|---|---|---|---|---|---|
| *No CHIP* | 204 (38) | 209 (36) | | 277 (177) | 258 (166) |
| *CHIP* | 219 (24) | 211 (37) | | 242(40) | 316 (224) |

| **HDL-C (mg/dL), mean (SD)** | 50 (14) | 53 (15) | | 49 (13) | 51 (13) |
|---|---|---|---|---|---|
| *No CHIP* | 53 (11) | 54 (15) | | 49 (13) | 51 (13) |
| *CHIP* | 45 (12) | 51 (15) | | 48 (13) | 50 (9) |

Hypertension defined as systolic blood pressure > 140 mm Hg or use of hypertensive medications. T2D = type 2 diabetes. HDL-C = high density lipoprotein cholesterol

The association between clonal hematopoiesis (CHIP) and coronary heart disease was analyzed based on previous exploratory results *(see* Jaiswal 2014) by utilizing a nested case-control study design. A power calculation based on a prevalence of clonal hematopoiesis of 7% (corresponding to a mean age of 65 in the cohorts), 500 cases and 500 controls (1:1 ratio), and a hazard ratio of 2.0 for coronary heart disease resulted in 89% power to detect a difference if one existed at the 0.05 two-sided significance level. Under the same assumptions, 333 cases and 667 controls (1:2 ratio) resulted in 82% power to detect a difference if one existed at the 0.05 two-sided significance level.

Based on these calculations, 439 cases and 584 controls (326 cases and 326 controls in MDC (1:1 matching), and 113 cases and 258 controls in BioImage (1:1 to 1:3 matching)) were selected for this study after excluding those with prevalent events. Cases were defined as described above and were matched to controls based on age (+/- 2 years), sex, type 2 diabetes status, and smoking history. After excluding 6 cases and 6 matched controls from MDC that either were not able to be sequenced or failed quality control, and 1 control from BioImage that did not pass quality control, 320 cases and 320 controls were left in MDC and 113 cases and 257 controls were left in BioImage.

In a traditional prospective nested case-control study, a cutoff is applied at time X, and cases are only selected if they have had an event by time X, while controls must have survived and had follow-up until at least time X. An odds ratio can then be calculated by logistic regression from this case-control set.

However, this traditional analysis may not be the correct analysis for CHIP, as CHIP increases the risk of hematological malignancy and all-cause mortality (Jaiswal 2014). Hence, selecting only those subjects who have survived for a certain period biases against selecting those with CHIP in the control set. Thus, a matched set of controls will be *depleted* for CHIP carriers, thereby *inflating* the estimated CHD risk ratio for CHIP.

Instead, cases and controls were selected without regard to follow-up time, and then a time-to-event analysis was performed. This may be the most conservative, and correct, analysis. The data were also analyzed using two additional methods for comparison: logistic regression and incident density sampling (see below).

To identify those with CHIP, a pre-specified list of variants in 75 genes known to be recurrent drivers of myeloid malignancies was used. The variants were selected on the basis of being reported in the literature and/or the Catalog of Somatic Mutations in Cancer (COSMIC, www.cancer.sanger.ac.uk/cancergenome/projects/cosmic/) from 75 genes known to be recurrent drivers in myeloid malignancies (see **Table 2).** A minimum variant read counts of 3 for MuTect and 6 for Indelocator were used in order to call somatic variants.

**Table 2: List of hematopoietic genes and variants queried**

| Gene name | Reported mutations used for variant calling | Accession |
|---|---|---|
| ASXL1 | Frameshift/nonsense/splice-site in exon 11-12 | NM_015338 |
| ASXL2 | Frameshift/nonsense/splice-site in exon 11-12 | NM_018263 |
| BCOR | Frameshift/nonsense/splice-site | NM_001123385 |
| BCORL1 | Frameshift/nonsense/splice-site | NM_021946 |
| BRAF | G464E, G464V, G466E, G466V, G469R, G469E, G469A, G469V, V471F, V472S, L485W, N581S, I582M, I592M, I592V, D594N, D594G, D594V, D594E, F595L, F595S, G596R, L597V, L597S, L597Q, L597R, A598V, V600M, V600L, V600K, V600R, V600E, V600A, V600G, V600D, K601E, K601N, R603^{∗}, W604R, W604G, S605G, S605F, S605N, G606E, G606A, G606V, H608R, H608L, G615R, S616P, S616F, L618S, L618W | NM_004333 |
| BRCC3 | Frameshift/nonsense/splice-site | NM_024332 |
| CBL | RING finger missense p.381-421 | NM_005188 |
| CBLB | RING finger missense p.372-412 | NM_170662 |
| CEBPA | Frameshift/nonsense/splice-site | NM_004364 |
| CREBBP | Frameshift/nonsense/splice-site, D1435E, R1446L, R1446H, R1446C, Y1450C, P1476R, Y1482H, H1487Y, W1502C, Y1503D, Y1503H, Y1503F, S1680del | NM_004380 |
| CSF1R | L301F, L301S, Y969C, Y969N, Y969F, Y969H, Y969D | NM_005211 |
| CSF3R | T615A, T618I, truncating c.741-791 | NM_000760 |
| CTCF | Frameshift/nonsense, R377C, R377H, P378A, P378L | NM_006565 |
| CUX1 | Frameshift/nonsense | NM_181552 |
| DNMT3A | Frameshift/nonsense/splice-site, F290I, F290C, V296M, P307S, P307R, R326H, R326L, R326C, R326S, G332R, G332E, V339A, V339M, V339G, L344Q, L344P, R366P, R366H, R366G, A368T, A368V, R379H, R379C, I407T, I407N, I407S, F414L, F414S, F414C, A462V, K468R, C497G, C497Y, Q527H, Q527P, Y533C, S535F, C537G, C537R, G543A, G543S, G543C, L547H, L547P, L547F, M548I, M548K, G550R, W581R, W581G, W581C, R604Q, R604W, R635W, R635Q, S638F, G646V, G646E, L653W, L653F, I655N, V657A, V657M, R659H, Y660C, V665G, V665L, M674V, R676W, R676Q, G685R, G685E, G685A, D686Y, D686G, R688H, G699R, G699S, G699D, P700L, P700S, P700R, P700Q, P700T, P700A, D702N, D702Y, V704M, V704G, I705F, I705T, I705S, I705N, G707D, G707V, C710S, C710Y, S714C, V716D, V716F, V716I, N717S, N717I, P718L, R720H, R720G, K721R, K721T, Y724C, R729Q, R729W, R729G, F731C, F731L, F731Y, F731I, F732del, F732C, F732S, F732L, E733G, E733A, F734L, F734C, Y735C, Y735N, Y735S, R736H, R736C, R736P, L737H, L737V, L737F, L737R, A741V, P742P, P743R, P743L, R749C, R749L, R749H, R749G, F751L, F751C, F752del, F752C, F752L, F752I, F752V, W753G, W753C, W753R, L754P, L754R, L754H, F755S, F755I, F755L, M761I, M761V, G762C, V763I, S770L, S770W, S770P, R771Q, F772I, F772V, L773R, L773V, E774K, E774D, E774G, I780T, D781G, R792H, W795C, W795L, G796D, G796V, N797Y, N797H, N797S, P799S, P799R, P799H, R803S, R803W, P804L, P804S, K826R, S828N, K829R, T835M, N838D, K841Q, Q842E, P849L, D857N, W860R, E863D, F868S, G869S, G869V, M880V, S881R, S881I, R882H, R882P, R882C, R882G, A884P, A884V, Q886R, L889P, L889R, G890D, G890R, G890S, V895M, P896L, V897G, V897D, R899L, R899H, R899C, L901R, L901H, P904L, F909C, P904Q, A910P, C911R, C911Y | NM_022552 |
| EED | Frameshift/nonsense/splice-site, L240Q, I363M | NM_003797 |
| EP300 | Frameshift/nonsense/splice_site, VF1148_1149del, D1399N, D1399Y, P1452L, Y1467N, Y1467H, Y1467C, R1627W, A1629V | NM_001429 |
| ETNK1 | N244S, N244T, N244K | NM_018638 |
| ETV6 | Frameshift/nonsense/splice-site | NM_001987 |
| EZH2 | Frameshift/nonsense/splice-site, Q62R, N102S, F145S, F145C, F145Y, F145L, G159R, E164D, R202Q, K238E, E244K, R283Q, H292R, P488S, R497Q, R561H, T568I, K629E, Y641N, Y641H, Y641S, Y641C, Y641F, D659Y, D659G, V674M, A677G, A677V, R679C, R679H, R685C, R685H, A687V, N688I, N688K, H689Y, S690P, I708V, I708T, I708M, E720K, E740K | NM_001203247 |
| FLT3 | V579A, V592A, V592I, F594L, FY590-591GD, D835Y, D835H, D835E, del835 | NM_004119 |
| GATA1 | Frameshift/nonsense/splice-site | NM_002049 |
| GATA2 | Frameshift/nonsense/splice-site, R293Q, N317H, A318T, A318V, A318G, G320D, L321P, L321F, L321V, Q328P, R330Q, R361L, L359V, A372T, R384G, R384K | NM_001145661 |
| GATA3 | Frameshift/nonsense/splice-site ZNF domain, R276W, R276Q, N286T, L348V, | NM_001002295 |
| GNA13 | I34T, G57S, S62F, M68K, Q134R, Y145F, L152F, E167D, Q169H, R264H, E273K, V322G, V362G, L371F | NM_006572 |
| GNAS | R201(844)S, R201(844)C, R201(844)H, R201(844)L, Q227(870)K, Q227(870)R, Q227(870)L, Q227(870)H, R374(1017)C | NM_016592 |
| GNB1 | K57N, K57M, K57E, K57T, I80T, I80N | NM_002074 |
| IDH1 | R132C, R132G, R132H, R132L, R132P, R132V, V178I | NM_005896 |
| IDH2 | R140W, R140Q, R140L, R140G, R172W, R172G, R172K, R172T, R172M, R172N, R172S | NM_002168 |
| IKZF1 | Frameshift/nonsense | NM_006060 |
| IKZF2 | Frameshift/nonsense | NM_016260 |
| IKZF3 | Frameshift/nonsense | NM_012481 |
| JAK1 | T478A, T478S, V623A, A634D, L653F, R724H, R724Q, R724P, T782M, L783F | NM_002227 |
| JAK2 | N533D, N533Y, N533S, H538R, K539E, K539L, I540T, I540V, V617F, R683S, R683G, del/ins537-539L, del/ins538-539L, del/ins540-543MK, del/ins540-544MK, del/ins541-543K, del542-543, del543-544, ins11546-547 | NM_004972 |
| JAK3 | M511T, M511I, A572V, A572T, A573V, R657Q, V715I, V715A | NM_000215 |
| KDM6A | Frameshift/nonsense/splice-site, del419 | NM_021140 |
| KIT | ins503, V559A, V559D, V559G, V559I, V560D, V560A, V560G, V560E, de1560, E561K, de1579, P627L, P627T, R634W, K642E, K642Q, V654A, V654E, H697Y, H697D, E761D, K807R, D816H, D816Y, D816F, D816I, D816V, D816H, del551-559 | NM_000222 |
| KRAS | G12D, G12A, G12E, G12V, G13D, G13C, G13Y, G13F, G13R, G13A, G13V, G13E, V14I, T58I, G60D, G60A, G60V, Q61K, Q61E, Q61P, Q61R, Q61L, Q61H, K117E, K117N, A146T, A146P, A146V | NM_033360 |
| LUC7L2 | Frameshift/nonsense/splice-site | NM_016019 |
| MLL | Frameshift/nonsense | NM_005933 |
| MLL2 | Frameshift/nonsense | NM_003482 |
| MPL | S505G, S505N, S505C, L510P, del513, W515A, W515R, W515K, W515S, W515L, A519T, A519V, Y591D, W515-518KT | NM_005373 |
| NF1 | Frameshift/nonsense | NM_000267 |
| NPM1 | Frameshift p.W288fs (insertion at c.859_860, 860_861, 862_863,863_864) | NM_002520 |
| NRAS | G12S, G12R, G12C, G12N, G12P, G12Y, G12D, G12A, G12V, G12E, G13S, G13R, G13C, G13N, G13P, G13Y, G13D, G13A, G13V, G13E, G60E, G60R, Q61R, Q61L, Q61K, Q61P, Q61H, Q61Q | NM_002524 |
| PDS5B | Frameshift/nonsense/splice-site, R1292Q | NM_015032 |
| PDSS2 | Frameshift/nonsense | NM_020381 |
| PHF6 | Frameshift/nonsense/splice-site, A40D, M125I, S246Y, F263L, R274Q, C297Y, H302Y, H329L | NM_001015877 |
| PHIP | Frameshift/nonsense/splice-site | NM_017934 |
| PPM1D | Frameshift/nonsense, exon 5 or 6 | NM_003620 |
| PRPF40B | Frameshift/nonsense/splice-site, P15H, M58I, P405L, P562S, | NM_001031698 |
| PRPF8 | M1307I, C1594W, D1598Y, D1598N, D1598V (ADD MORE VARS) | NM_006445 |
| PTEN | Frameshift/nonsense/splice-site, D24G, R47G, F56V, L57W, H61R, K66N, Y68H, C71Y, F81C, Y88C, D92G, D92V, D92E, H93Y, H93D, H93Q, N94I, P95L, I101T, C105F, C105S, D107Y, L112V, H123Y, C124R, C124S, K125E, A126D, K128N, R130G, R130Q, R130L, G132D, I135V, I135K, C136R, C136F, K144Q, A151T, D153Y, D153N, Y155H, Y155C, R159K, R159S, R161K, R161I, G165R, G165E, S170N, S170I, R173C, Y174D, Y177C, H196Y, R234W, G251C, D252Y, F271S, D326G | NM_000314 |
| PTPN11 | G60V, G60R, G60A, D61Y, D61V, D61G, Y63C, E69K, E69G, E69D, E69Q, F71L, F71K, A72T, A72V, A72D, T73I, E76K, E76Q, E76M, E76A, E76G, E139G, E139D, N308D, N308T, N339S, P491L, S502P, S502A, S502L, G503V, G503G, G503A, G503E, Q506P, T507A, T507K | NM_002834 |
| RAD21 | Frameshift/nonsense/splice-site, R65Q, H208R, Q474R | NM_006265 |
| RUNX1 | Frameshift/nonsense/splice-site, S73F, H78Q, H78L, R80C, R80P, R80H, L85Q, P86L, P86H, S114L, D133Y, L134P, R135G, R135K, R135S, R139Q, R142S, A165V, R174Q, R177L, R177Q, A224T, D171G, D171V, D171N, R205W, R223C | NM_001001890 |
| SETBP1 | D868N, D868T, S869N, G870S, I871T, D880N, D880Q | NM_015559 |
| SETD2 | Frameshift/nonsense, VI190M | NM_014159 |
| SETDB1 | Frameshift/nonsense, K715E | NM_001145415 |
| SF1 | Frameshift/nonsense/splice-site, T454M, Y476C, A508G | NM_004630 |
| SF3A1 | Frameshift/nonsense/splice-site, A57S, M117I, K166T, Y271C | NM_005877 |
| SF3B1 | G347V, R387W, R387Q, E592K, E622D, Y623C, R625L, R625C, R625G, H662Q, H662D, T663I, K666N, K666T, K666E, K666R, K700E, V701F, A708T, G740R, G740E, A744P, D781G, E783K, R831Q, L833F, E862K, R957Q | NM_012433 |
| SFRS2 | Y44H, P95H, P95L, P95T, P95R, P95A, P107H, P95fs | NM_003016 |
| SMC1A | K190T, R586W, M689V, R807H, R1090H, R1090C | NM_006306 |
| SMC3 | Frameshift/nonsense, R155I, Q367E, D392V, K571R, R661P, G662C | NM_005445 |
| STAG1 | Frameshift/nonsense/splice-site, H1085Y | NM_005862 |
| STAG2 | Frameshift/nonsense/splice-site | NM_006603 |
| SUZ12 | Frameshift/nonsense | NM_015355 |
| TET2 | Frameshift/nonsense/splice-site, missense mutations in catalytic domains (p.1104-1481 and 1843-2002) | NM_001127208 |
| TP53 | Frameshift/nonsense/splice-site, S46F, G105C, G105R, G105D, G108S, G108C, R110L, R110C, T118A, T118R, T118I, S127F, S127Y, L130V, L130F, K132Q, K132E, K132W, K132R, K132M, K132N, F134V. F134L, F134S, C135W, C135S, C135F, C135G, C135Y, Q136K, Q136E, Q136P, Q136R, Q136L, Q136H, A138P, A138V, A138A, A138T, T140I, C141R, C141G, C141A, C141Y, C141S, C141F, C141W, V143M, V143A, V143E, L145Q, W146C, W146L, L145R, V147G, P151T, P151A, P151S, P151H, P151R, P152S, P152R, P152L, T155P, T155A, V157F, R158H, R158L, A159V, A159P, A159S, A159D, A161T, A161D, Y163N, Y163H, Y163D, Y163S, Y163C, K164E, K164M, K164N, K164P, H168Y, H168P, H168R, H168L, H168Q, M169I, M169T, M169V, E171K, E171Q, E171G, E171A, E171V, E171D, V172D, V173M, V173L, V173G, R174W, R175G, R175C, R175H, C176R, C176G, C176Y, C176F, C176S, P177R, P177R, P177L, H178D, H178P, H178Q, H179Y, H179R, H179Q, R181C, R181Y, D186G, G187S, P190L, P190T, H193N, H193P, H193L, H193R, L194F, L194R, I195F, I195N, I195T, R196P, V197L, G199V, Y205N, Y205C, Y205H, D208V, R213Q, R213P, R213L, R213Q, H214D, H214R, S215G, S215I, S215R, V216M, V217G, Y220N, Y220H, Y220S, Y220C, E224D, I232F, I232N, I232T, I232S, Y234N, Y234H, Y234S, Y234C, Y236N, Y236H, Y236C, M237V, M237K, M237I, C238R, C238G, C238Y, C238W, N239T, N239S, S241Y, S241C, S241F, C242G, C242Y, C242S, C242F, G244S, G244C, G244D, G245S, G245R, G245C, G245D, G245A, G245V, G245S, M246V, M246K, M246R, M246I, N247I, R248W, R248G, R248Q, R249G, R249W, R249T, R249M, P250L, I251N, L252P, I254S, I255F, I255N, I255S, L257Q, L257P, E258K, E258Q, D259Y, S261T, G262D, G262V, L265P, G266R, G266E, G266V, R267W, R267Q, R267P, E271K, V272M, V272L, R273S, R273G, R273C, R273H, R273P, | NM_001126112 |
| | R273L, V274F, V274D, V274A, V274G, V274L, C275Y, C275S, C275F, A276P, C277F, C277Y, P278T, P278A, P278S, P278H, P278R, P278L, G279E, R280G, R280K, R280T, R280I, R280S, D281N, D281H, D281Y, D281G, D281E, R282G, R282W, R282Q, R282P, E285K, E285V, E286G, E286V, E286K, K320N, L330R, G334V, R337C, R337L, A347T, L348F, T377P | |
| U2AF1 | D14G, S34F, S34Y, R35L, R156H, R156Q, Q157R, Q157P | NM_006758 |
| U2AF2 | R18W, Q143L, M144I, L187V, Q190L | NM_007279 |
| WT1 | Frameshift/nonsense/splice-site | NM_024426 |
| ZRSR2 | Frameshift/nonsense, R126P, E133G, C181F, H191Y, I202N, F239V, F239Y, N261Y, C280R, C302R, C326R, H330R, N382K | NM_005089 |

Frameshift, nonsense, and splice-site mutations were further excluded if they occurred in the first or last 10% of the gene open reading frame, unless mutations in those regions had been previously reported, (e.g. *DNMT3A).* Frameshift mutations were also excluded if the insertions/deletions occurred in homo-polymer repeats (5 consecutive reads of the same nucleotide) unless there were a total 10 or more supporting reads and a VAF>8% for these indels.

For *TET*2 and *CBL,* all missense variants in particular regions *(see* Hu L et al., Cell 155:1545-55 (2013) and Sanada M et al., Nature 460:904-8 (2009)) were considered somatic if the VAF significantly deviated from the expected distribution for a germline allele (defined as a p-value from a binomial test of less than 0.001 assuming a probability of success in a single Bernoulli experiment of 0.5 and using the alternate allele read count as the number of successes and the alternate allele read count + reference allele read count as the number of trials).

Statistical tests were run for associations with coronary heart disease using a Cox proportional hazards model with the pre-specified co-variables age (continuous variable), sex, type 2 diabetes status, total cholesterol (continuous variable), high density lipoprotein cholesterol (HDL-C) (continuous variable), hypertension (categorical variable, defined as self-reported hypertension OR systolic blood pressure > 140 mm Hg OR diastolic blood pressure > 90 mm Hg OR anti-hypertensive medication), and smoking status (current smoker versus former or never smoker). For those subjects on statins, total cholesterol (TC) was divided by 0.8, and low-density lipoprotein cholesterol (LDL-C) was calculated by the Friedwald equation (LDL-C = TC - HDL-C - triglycerides/5). If triglycerides were > 400 mg/dL, LDL was changed to "not available". If TC was not available, then LDL was divided by 0.7 in the setting of a statin.

A meta-analysis of the two cohorts was also performed using a fixed-effects model.

In some analyses, associations for coronary heart disease were performed using clone size as an input variable. For these analyses, the clone size was divided into two categorical groups using a variant allele frequency (VAF) cutoff of 10%, which has been previously used to assess the risk of hematological malignancies in those with CHIP (see Jaiswal 2014). VAF is defined as the number of reads supporting a variant allele divided by the number of reads supporting a variant allele plus number of reads supporting the reference allele. MDC was not used for these analyses because DNA was obtained from granulocytes as opposed to peripheral blood, which has the likely effect of inflating the VAF (the median VAF in MDC was 15.0% compared to 8.6% in BioImage).

For Cox proportional hazards models, the R package 'survival' was used, and for fixed-effects meta-analysis the R package 'meta' was used.

Analyses were also performed using a traditional nested case-control design and risk set sampling (incidence density sampling without replacement). For MDC, cases were only those who had CHD that occurred within 16.75 years (selected to maximize the number of cases and controls). Controls were only those who were CHD-free with at least 16.75 years of follow-up. Matching cases and controls were also removed if they did not meet these criteria. From the original set of 320 cases and 320 controls, this left 245 cases and 245 controls. The 2 X 2 contingency table for CHIP and CHD is as follows:

| | No CHD | CHD |
|---|---|---|
| No CHIP | 241 | 232 |
| CHIP | 4 | 13 |

In a logistic regression adjusted for age, sex, type 2 diabetes status, smoking status, total cholesterol and HDL cholesterol, CHIP had an OR of 3.6 (p=0.03).

For BioImage, cases were only those who had CHD that occurred within 910 days (selected to maximize the number of cases and controls). Controls were only those who were CHD-free with at least 910 days of follow-up. Matching cases and controls were also removed if they did not meet these criteria. From the original set of 113 cases and 257 controls, this left 96 cases and 186 controls. The 2 X 2 contingency table for CHIP and CHD is as follows:

| | No CHD | CHD |
|---|---|---|
| No CHIP | 170 | 81 |
| CHIP | 16 | 15 |

In a logistic regression adjusted for age, sex, type 2 diabetes status, smoking status, total cholesterol and HDL cholesterol, CHIP had an OR of 2.4 (p=0.03).

Risk set sampling (incidence density sampling without replacement) was also done. For MDC, cases and their matched controls were taken only if the controls had follow-up for at least the same period as the matched case. Controls and matched cases were removed if they did not meet these criteria. From the original set of 320 cases and 320 controls, this left 283 cases and 283 controls. The 2 X 2 contingency table for CHIP and CHD is as follows:

| | No CHD | CHD |
|---|---|---|
| No CHIP | 279 | 266 |
| CHIP | 4 | 17 |

In a Cox proportional hazards model adjusted for age, sex, type 2 diabetes status, smoking status, total cholesterol and HDL cholesterol, CHIP had a HR of 2.6 (p=0.0002).

For BioImage, cases and their matched controls were taken only if the controls had follow-up for at least the same period as the matched case. Controls and matched cases were removed if they did not meet these criteria. From the original set of 113 cases and 257 controls, this left 105 cases and 220 controls. The 2 X 2 contingency table for CHIP and CHD is as follows:

| | No CHD | CHD |
|---|---|---|
| No CHIP | 199 | 89 |
| CHIP | 21 | 16 |

In a Cox proportional hazards model adjusted for age, sex, type 2 diabetes status, smoking status, total cholesterol and HDL cholesterol, CHIP had a HR of 1.8 (p=0.04).

The most commonly mutated genes were *DNMT3A, TET2,* and *ASXL1,* and 94/99 (95%) individuals with CHIP only had a single driver gene mutated *(see* Jaiswal 2014 and Genovese G et al., N Engl J Med 371:2477-87 (2014)). (**Figures 1A-1B****, Table 3**)**.**

**Table 3: List of CHIP-associated somatic variants identified**

| **Hugo Symbol** | **Chr** | **Start pos.** | **End pos.** | **Var. Class** | **Var. Type** | **Ref. All.** | **Alt. All.** | **Protein Change** | **Alt. reads** | **Ref. Reads** | **VAF** | **Coho rt** |
|---|---|---|---|---|---|---|---|---|---|---|---|---|
| ASXL1 | 20 | 31021211 | 31021211 | Nonsense Mutation | SNP | C | T | p.R404* | 22 | 155 | 0.1242 94 | Biol mage |
| ASXL1 | 20 | 31021430 | 31021430 | Nonsense Mutation | SNP | G | T | p.E477* | 22 | 57 | 0.2784 81 | MDC |
| ASXL1 | 20 | 31021637 | 31021637 | Nonsense Mutation | SNP | C | T | p.Q546* | 23 | 68 | 0.2527 47 | Biol mage |
| ASXL1 | 20 | 31022284 | 31022285 | Frame Shift Ins | INS | - | T | p.T590fs | 20 | 137 | 0.13 | ATVB |
| ASXL1 | 20 | 31022286 | 31022287 | Frame Shift Ins | INS | - | A | p.Y591fs | 15 | 114 | 0.12 | Biol mage |
| ASXL1 | 20 | 31022286 | 31022287 | Frame Shift Ins | INS | - | A | p.Y591fs | 11 | 138 | 0.07 | ATVB |
| ASXL1 | 20 | 31022288 | 31022288 | Nonsense Mutation | SNP | C | A | p.Y591* | 9 | 43 | 0.1730 77 | ATVB |
| ASXL1 | 20 | 31022293 | 31022294 | Frame Shift Ins | INS | - | A | p.C594fs | 28 | 51 | 0.35 | MDC |
| *ASXL1* | 20 | 31022403 | 31022425 | Frame Shift Del | DEL | CACC ACTG CCTA GAGA GGC GGC | - | p.HHCHREAA630f s | 12 | 75 | 0.14 | Biol mage |
| ASXL1 | 20 | 31022439 | 31022443 | Frame Shift Del | DEL | GGAG G | - | p.GG644fs | 6 | 71 | 0.08 | Biol mage |
| ASXL1 | 20 | 31022502 | 31022503 | Frame Shift Ins | INS | - | G | p.S663fs | 12 | 43 | 0.22 | Biol mage |
| ASXL1 | 20 | 31022536 | 31022537 | Frame Shift Ins | INS | - | C | p.H674fs | 6 | 26 | 0.19 | ATVB |
| ASXL1 | 20 | 31022757 | 31022757 | Nonsense Mutation | SNP | C | T | p.Q748* | 43 | 125 | 0.2559 52 | MDC |
| *ASXL1* | 20 | 31022838 | 31022838 | Frame Shift Del | DEL | T | - | p.L775fs | 8 | 44 | 0.15 | ATVB |
| ASXL1 | 20 | 31023152 | 31023153 | Frame Shift Ins | INS | - | A | p.D879fs | 9 | 145 | 0.06 | ATVB |
| ASXL1 | 20 | 31023183 | 31023184 | Frame Shift Ins | INS | - | T | p.L890fs | 48 | 109 | 0.31 | MDC |
| ASXL1 | 20 | 31023339 | 31023339 | Frame Shift Del | DEL | G | - | p.G942fs | 6 | 127 | 0.05 | MDC |
| ASXL1 | 20 | 31023702 | 31023702 | Nonsense Mutation | SNP | C | T | p.Q1063* | 28 | 97 | 0.224 | Biol mage |
| ASXL1 | 20 | 31023717 | 31023717 | Nonsense Mutation | SNP | C | T | p.R1068* | 9 | 69 | 0.1153 85 | MDC |
| ASXL1 | 20 | 31023945 | 31023958 | Frame Shift Del | DEL | CATG G CTCG C TACG | - | p.H1144fs | 18 | 63 | 0.22 | ATVB |
| ASXL1 | 20 | 31024481 | 31024482 | Frame Shift Del | DEL | GA | - | p.M1323fs | 11 | 78 | 0.12 | MDC |
| ASXL2 | 2 | 25973203 | 25973203 | Nonsense Mutation | SNP | G | A | p.Q408* | 6 | 30 | 0.1666 67 | Biol mage |
| BRAF | 7 | 140477854 | 140477854 | Missense Mutation | SNP | A | C | p.L485W | 10 | 99 | 0.0917 43 | Biol mage |
| BRCC3 | X | 154299821 | 154299821 | Nonsense Mutation | SNP | C | T | P.Q7* | 10 | 89 | 0.1010 1 | MDC |
| BRCC3 | X | 154301707 | 154301707 | Splice Site | SNP | G | A | c.e3+1 | 3 | 23 | 0.1153 85 | MDC |
| CBL | 11 | 119149005 | 119149005 | Nonsense Mutation | SNP | C | T | p.Q409* | 4 | 44 | 0.0833 33 | Biol mage |
| CBL | 11 | 119149251 | 119149251 | Missense Mutation | SNP | G | A | p.R420Q | 6 | 110 | 0.0517 24 | Biol mage |
| CREBBP | 16 | 3808975 | 3808975 | Splice Site | SNP | T | C | c.e17-2 | 34 | 50 | 0.4047 62 | MDC |
| DNMT3A | 2 | 25457185 | 25457185 | Missense Mutation | SNP | A | C | p.L901R | 14 | 33 | 0.2978 72 | Biol mage |
| DNMT3A | 2 | 25457242 | 25457242 | Missense Mutation | SNP | C | T | p.R882H | 7 | 45 | 0.1346 15 | MDC |
| DNMT3A | 2 | 25457242 | 25457242 | Missense Mutation | SNP | C | T | p.R882H | 6 | 36 | 0.1428 57 | MDC |
| DNMT3A | 2 | 25457242 | 25457242 | Missense Mutation | SNP | C | T | p.R882H | 10 | 39 | 0.2040 82 | MDC |
| DNMT3A | 2 | 25457242 | 25457242 | Missense Mutation | SNP | C | T | p.R882H | 13 | 47 | 0.2166 67 | ATVB |
| DNMT3A | 2 | 25457242 | 25457242 | Missense Mutation | SNP | C | T | p.R882H | 10 | 52 | 0.1612 9 | ATVB |
| DNMT3A | 2 | 25457242 | 25457242 | Missense Mutation | SNP | C | T | p.R882H | 4 | 30 | 0.1176 47 | ATVB |
| DNMT3A | 2 | 25457243 | 25457243 | Missense Mutation | SNP | G | A | p.R882C | 4 | 24 | 0.1428 57 | Biol mage |
| DNMT3A | 2 | 25457243 | 25457243 | Missense Mutation | SNP | G | A | p.R882C | 8 | 21 | 0.2758 62 | Biol mage |
| DNMT3A | 2 | 25457243 | 25457243 | Missense Mutation | SNP | G | A | p.R882C | 4 | 34 | 0.1052 63 | MDC |
| DNMT3A | 2 | 25457243 | 25457243 | Missense Mutation | SNP | G | A | p.R882C | 6 | 44 | 0.12 | ATVB |
| DNMT3A | 2 | 25457290 | 25457290 | Splice Site | SNP | C | T | c.e23-1 | 10 | 10 | 0.5 | MDC |
| DNMT3A | 2 | 25458594 | 25458594 | Nonsense Mutation | SNP | C | T | p.W860* | 4 | 25 | 0.1379 31 | MDC |
| DNMT3A | 2 | 25458595 | 25458595 | Missense Mutation | SNP | A | G | p.W860R | 4 | 29 | 0.1212 12 | Biol mage |
| DNMT3A | 2 | 25458661 | 25458661 | Missense Mutation | SNP | T | C | p.N838D | 5 | 16 | 0.2380 95 | MDC |
| DNMT3A | 2 | 25459806 | 25459806 | Splice Site | SNP | T | C | p.K826R | 10 | 31 | 0.2439 02 | MDC |
| DNMT3A | 2 | 25459871 | 25459874 | Frame Shift Del | DEL | CGGC | - | p.RP803fs | 6 | 45 | 0.12 | MDC |
| DNMT3A | 2 | 25459875 | 25459875 | Splice Site | SNP | C | G | c.e21-1 | 13 | 56 | 0.1884 06 | MDC |
| DNMT3A | 2 | 25461999 | 25461999 | Splice Site | SNP | C | G | c.e20+1 | 4 | 32 | 0.1111 11 | ATVB |
| DNMT3A | 2 | 25463172 | 25463172 | Splice Site | SNP | T | C | p.E774G | 5 | 46 | 0.0980 39 | Biol mage |
| DNMT3A | 2 | 25463181 | 25463181 | Missense Mutation | SNP | C | T | p.R771Q | 16 | 26 | 0.3809 52 | MDC |
| DNMT3A | 2 | 25463184 | 25463184 | Missense Mutation | SNP | G | A | p.S770L | 18 | 32 | 0.36 | Biol mage |
| DNMT3A | 2 | 25463212 | 25463212 | Missense Mutation | SNP | T | C | p.M761V | 8 | 146 | 0.0519 48 | ATVB |
| DNMT3A | 2 | 25463212 | 25463212 | Missense Mutation | SNP | T | C | p.M761V | 7 | 148 | 0.0451 61 | ATVB |
| DNMT3A | 2 | 25463236 | 25463237 | Frame Shift Del | DEL | AG | - | p.F752fs | 8 | 165 | 0.05 | ATVB |
| DNMT3A | 2 | 25463247 | 25463247 | Missense Mutation | SNP | C | T | p.R749H | 4 | 19 | 0.1739 13 | Biol mage |
| DNMT3A | 2 | 25463248 | 25463248 | Missense Mutation | SNP | G | A | p.R749C | 9 | 33 | 0.2142 86 | MDC |
| DNMT3A | 2 | 25463248 | 25463248 | Missense Mutation | SNP | G | C | p.R749G | 5 | 18 | 0.2173 91 | MDC |
| DNMT3A | 2 | 25463268 | 25463268 | Missense Mutation | SNP | C | G | p.R742P | 20 | 105 | 0.16 | ATVB |
| DNMT3A | 2 | 25463287 | 25463287 | Missense Mutation | SNP | G | A | p.R736C | 4 | 26 | 0.1333 33 | MDC |
| DNMT3A | 2 | 25463298 | 25463300 | In Frame Del | DEL | AAG | - | p.731 732FF>F | 9 | 15 | 0.38 | MDC |
| DNMT3A | 2 | 25463553 | 25463553 | Missense Mutation | SNP | C | T | p.C710Y | 4 | 49 | 0.0754 72 | MDC |
| DNMT3A | 2 | 25463575 | 25463579 | Frame Shift Del | DEL | GATC G | - | p.DL702fs | 19 | 37 | 0.34 | MDC |
| DNMT3A | 2 | 25463584 | 25463584 | Missense Mutation | SNP | G | C | p.P700A | 5 | 74 | 0.0632 91 | ATVB |
| DNMT3A | 2 | 25464437 | 25464438 | Frame Shift Ins | INS | - | T | p.Q692fs | 7 | 75 | 0.09 | ATVB |
| DNMT3A | 2 | 25464490 | 25464490 | Frame Shift Del | DEL | C | - | p.V675fs | 7 | 38 | 0.16 | Biol mage |
| DNMT3A | 2 | 25464544 | 25464544 | Missense Mutation | SNP | C | T | p.V657M | 5 | 32 | 0.1351 35 | MDC |
| DNMT3A | 2 | 25467083 | 25467083 | Nonsense Mutation | SNP | G | A | p.R598* | 5 | 24 | 0.1724 14 | ATVB |
| DNMT3A | 2 | 25467099 | 25467099 | Nonsense Mutation | SNP | G | C | p.Y592* | 28 | 87 | 0.2434 78 | Biol mage |
| DNMT3A | 2 | 25467135 | 25467135 | Frame Shift Del | DEL | G | - | p.P580fs | 8 | 129 | 0.06 | Biol mage |
| DNMT3A | 2 | 25467472 | 25467472 | Missense Mutation | SNP | G | A | p.S535F | 6 | 123 | 0.0465 12 | Biol mage |
| DNMT3A | 2 | 25468120 | 25468120 | Splice Site | SNP | A | T | c.e13+1 | 8 | 45 | 0.1509 43 | MDC |
| DNMT3A | 2 | 25468888 | 25468888 | Splice Site | SNP | C | T | c.e12+1 | 4 | 73 | 0.0519 48 | MDC |
| DNMT3A | 2 | 25468920 | 25468920 | Nonsense Mutation | SNP | G | T | p.Y481* | 6 | 73 | 0.0759 49 | Biol mage |
| DNMT3A | 2 | 25469055 | 25469055 | Missense Mutation | SNP | T | C | p.K468R | 5 | 32 | 0.1351 35 | Biol mage |
| DNMT3A | 2 | 25469504 | 25469504 | Frame Shift Del | DEL | G | - | p.L422fs | 18 | 154 | 0.1 | ATVB |
| DNMT3A | 2 | 25469529 | 25469530 | Frame Shift Ins | INS | - | C | p.G413fs | 6 | 44 | 0.12 | Biol mage |
| DNMT3A | 2 | 25469920 | 25469920 | Splice Site | SNP | C | T | c.e9+1 | 14 | 22 | 0.3888 89 | ATVB |
| DNMT3A | 2 | 25469976 | 25469976 | Nonsense Mutation | SNP | G | A | p.Q356* | 6 | 69 | 0.08 | MDC |
| DNMT3A | 2 | 25470026 | 25470026 | Splice Site | SNP | A | C | p.V339G | 9 | 58 | 0.1343 28 | Biol mage |
| DNMT3A | 2 | 25470028 | 25470028 | Splice Site | SNP | C | G | c.e9-1 | 6 | 58 | 0.0937 5 | MDC |
| DNMT3A | 2 | 25470029 | 25470029 | Splice Site | SNP | T | C | c.e9-2 | 7 | 38 | 0.1555 56 | Biol mage |
| DNMT3A | 2 | 25470029 | 25470029 | Splice Site | SNP | T | A | c.e9-2 | 5 | 44 | 0.1020 41 | MDC |
| DNMT3A | 2 | 25470463 | 25470463 | Frame Shift Del | DEL | T | - | p.S337fs | 8 | 129 | 0.06 | ATVB |
| DNMT3A | 2 | 25470484 | 25470484 | Nonsense Mutation | SNP | C | T | p.W330* | 4 | 68 | 0.0555 56 | Biol mage |
| DNMT3A | 2 | 25470489 | 25470489 | Frame Shift Del | DEL | T | - | p.M329fs | 7 | 87 | 0.07 | Biol mage |
| DNMT3A | 2 | 25470498 | 25470498 | Missense Mutation | SNP | G | A | p.R326C | 4 | 82 | 0.0465 12 | ATVB |
| DNMT3A | 2 | 25470533 | 25470533 | Nonsense Mutation | SNP | C | T | p.W314* | 10 | 93 | 0.0970 87 | ATVB |
| DNMT3A | 2 | 25470556 | 25470556 | Nonsense Mutation | SNP | C | T | p.W306* | 7 | 83 | 0.0777 78 | Biol mage |
| FLT3 | 13 | 28608282 | 28608282 | Missense Mutation | SNP | C | T | p.V592I | 20 | 24 | 0.4545 45 | ATVB |
| GNAS | 20 | 57484420 | 57484420 | Missense Mutation | SNP | C | T | p.R844C | 9 | 211 | 0.0409 09 | Biol mage |
| GNAS | 20 | 57484420 | 57484420 | Missense Mutation | SNP | C | A | p.R844S | 12 | 255 | 0.0449 44 | Biol mage |
| GNB1 | 1 | 1747229 | 1747229 | Missense Mutation | SNP | T | C | p.K57E | 5 | 53 | 0.0862 07 | Biol mage |
| GNB1 | 1 | 1747229 | 1747229 | Missense Mutation | SNP | T | C | p.K57E | 10 | 41 | 0.1960 78 | MDC |
| IDH2 | 15 | 90631934 | 90631934 | Missense Mutation | SNP | C | T | p.R140Q | 13 | 196 | 0.0622 01 | Biol mage |
| JAK2 | 9 | 5073770 | 5073770 | Missense Mutation | SNP | G | T | p.V617F | 4 | 23 | 0.1481 48 | MDC |
| JAK2 | 9 | 5073770 | 5073770 | Missense Mutation | SNP | G | T | p.V617F | 7 | 25 | 0.2187 5 | MDC |
| JAK2 | 9 | 5073770 | 5073770 | Missense Mutation | SNP | G | T | p.V617F | 35 | 117 | 0.2302 63 | ATVB |
| JAK2 | 9 | 5073770 | 5073770 | Missense Mutation | SNP | G | T | p.V617F | 6 | 123 | 0.0465 12 | ATVB |
| JAK2 | 9 | 5073770 | 5073770 | Missense Mutation | SNP | G | T | p.V617F | 7 | 124 | 0.0534 35 | ATVB |
| JAK2 | 9 | 5073770 | 5073770 | Missense Mutation | SNP | G | T | p.V617F | 21 | 45 | 0.3181 82 | ATVB |
| JAK2 | 9 | 5073770 | 5073770 | Missense Mutation | SNP | G | T | p.V617F | 16 | 75 | 0.1758 24 | ATVB |
| JAK2 | 9 | 5073770 | 5073770 | Missense Mutation | SNP | G | T | p.V617F | 6 | 138 | 0.0416 67 | ATVB |
| JAK2 | 9 | 5073770 | 5073770 | Missense Mutation | SNP | G | T | p.V617F | 41 | 37 | 0.5256 41 | ATVB |
| JAK2 | 9 | 5073770 | 5073770 | Missense Mutation | SNP | G | T | p.V617F | 7 | 65 | 0.0972 22 | ATVB |
| JAK2 | 9 | 5073770 | 5073770 | Missense Mutation | SNP | G | T | p.V617F | 17 | 44 | 0.2786 89 | ATVB |
| JAK2 | 9 | 5073770 | 5073770 | Missense Mutation | SNP | G | T | p.V617F | 6 | 141 | 0.0408 16 | ATVB |
| KDM6A | X | 44928872 | 44928872 | Nonsense Mutation | SNP | C | T | p.R710* | 19 | 10 | 0.6551 72 | ATVB |
| KDM6A | X | 44948987 | 44948987 | Splice Site | SNP | G | T | c.e25-1 | 3 | 14 | 0.1764 71 | ATVB |
| KRAS | 12 | 25378561 | 25378561 | Missense Mutation | SNP | G | A | p.A146V | 4 | 68 | 0.0555 56 | Biol mage |
| MLL2 | 12 | 49435736 | 49435736 | Nonsense Mutation | SNP | C | T | p.W2049* | 3 | 10 | 0.2307 69 | ATVB |
| NOTCH2 | 1 | 120462929 | 120462930 | Frame Shift Ins | INS | - | T | p.R1801fs | 33 | 32 | 0.51 | ATVB |
| PDS5B | 13 | 33275493 | 33275493 | Nonsense Mutation | SNP | C | T | p.Q592* | 4 | 20 | 0.1666 67 | Biol mage |
| PPM1D | 17 | 58740624 | 58740624 | Frame Shift Del | DEL | A | - | p.Q510fs | 12 | 84 | 0.12 | MDC |
| PPM1D | 17 | 58740749 | 58740749 | Nonsense Mutation | SNP | C | T | p.R552* | 6 | 65 | 0.0845 07 | ATVB |
| RAD21 | 8 | 117866664 | 117866664 | Frame Shift Del | DEL | G | - | p.D327fs | 8 | 38 | 0.17 | Biol mage |
| SF3B1 | 2 | 198267359 | 198267359 | Missense Mutation | SNP | C | G | p.K666N | 10 | 88 | 0.1020 41 | Biol mage |
| SF3B1 | 2 | 198267360 | 198267360 | Missense Mutation | SNP | T | G | p.K666T | 22 | 80 | 0.2156 86 | Biol mage |
| SF3B1 | 2 | 198267371 | 198267371 | Missense Mutation | SNP | G | C | p.H662Q | 15 | 103 | 0.1271 19 | Biol mage |
| SMC3 | 10 | 112357914 | 112357914 | Frame Shift Del | DEL | G | - | p.D712fs | 28 | 16 | 0.64 | MDC |
| SRSF2 | 17 | 74732959 | 74732959 | Missense Mutation | SNP | G | T | p.P95H | 13 | 69 | 0.1585 37 | Biol mage |
| SRSF2 | 17 | 74732959 | 74732959 | Missense Mutation | SNP | G | T | p.P95H | 8 | 39 | 0.1702 13 | ATVB |
| SUZ12 | 17 | 30303572 | 30303572 | Nonsense Mutation | SNP | C | T | p.R286* | 4 | 25 | 0.1379 31 | Biol mage |
| SUZ12 | 17 | 30303572 | 30303572 | Nonsense Mutation | SNP | C | T | p.R286* | 4 | 46 | 0.08 | Biol mage |
| TET2 | 4 | 106155390 | 106155393 | Frame Shift Del | DEL | AGTT | - | p.T118fs | 16 | 47 | 0.25 | ATVB |
| TET2 | 4 | 106155749 | 106155749 | Frame Shift Del | DEL | C | - | p.S217fs | 9 | 32 | 0.22 | MDC |
| TET2 | 4 | 106155897 | 106155897 | Frame Shift Del | DEL | C | - | p.H266fs | 9 | 97 | 0.08 | Biol mage |
| TET2 | 4 | 106156636 | 106156636 | Nonsense Mutation | SNP | A | T | p.K534* | 13 | 52 | 0.2 | ATVB |
| TET2 | 4 | 106156747 | 106156747 | Nonsense Mutation | SNP | C | T | p.R571* | 11 | 49 | 0.1833 33 | ATVB |
| TET2 | 4 | 106156894 | 106156894 | Nonsense Mutation | SNP | C | T | p.Q599* | 12 | 69 | 0.1481 48 | MDC |
| TET2 | 4 | 106157153 | 106157154 | Frame Shift Del | DEL | AA | - | p.Q685fs | 8 | 153 | 0.05 | Biol mage |
| TET2 | 4 | 106157270 | 106157271 | Frame Shift Del | DEL | AT | - | p.H724fs | 14 | 144 | 0.09 | Biol mage |
| TET2 | 4 | 106157480 | 106157480 | Nonsense Mutation | SNP | C | G | p.S794* | 31 | 46 | 0.4025 97 | MDC |
| TET2 | 4 | 106157797 | 106157797 | Frame Shift Del | DEL | A | - | p.K921fs | 12 | 39 | 0.24 | ATVB |
| TET2 | 4 | 106158250 | 106158250 | Nonsense Mutation | SNP | C | T | p.Q1051* | 15 | 180 | 0.0769 23 | Biol mage |
| TET2 | 4 | 106164025 | 106164025 | Nonsense Mutation | SNP | A | T | p.R1179* | 6 | 27 | 0.1818 18 | Biol mage |
| TET2 | 4 | 106164758 | 106164758 | Missense Mutation | SNP | T | C | p.L1209P | 7 | 90 | 0.0721 65 | Biol mage |
| TET2 | 4 | 106164913 | 106164913 | Missense Mutation | SNP | C | T | p.R1261C | 4 | 73 | 0.0519 48 | MDC |
| TET2 | 4 | 106190797 | 106190797 | Missense Mutation | SNP | C | T | p.R1359C | 6 | 51 | 0.1052 63 | MDC |
| TET2 | 4 | 106190803 | 106190803 | Missense Mutation | SNP | G | T | p.G1361C | 4 | 61 | 0.0615 38 | Biol mage |
| TET2 | 4 | 106190834 | 106190834 | Missense Mutation | SNP | T | A | p.V1371D | 22 | 54 | 0.2894 74 | Biol mage |
| TET2 | 4 | 106190905 | 106190905 | Splice Site | SNP | G | C | c.e9+1 | 4 | 40 | 0.0909 09 | MDC |
| TET2 | 4 | 106193952 | 106193952 | Nonsense Mutation | SNP | A | T | p.K1472* | 23 | 24 | 0.4893 62 | Biol mage |
| TET2 | 4 | 106196267 | 106196267 | Nonsense Mutation | SNP | C | T | p.Q1534* | 3 | 20 | 0.1304 35 | MDC |
| TET2 | 4 | 106196282 | 106196282 | Nonsense Mutation | SNP | C | T | p.Q1539* | 34 | 24 | 0.5862 07 | MDC |
| TET2 | 4 | 106196937 | 106196937 | Frame Shift Del | DEL | A | - | p.H1778fs | 19 | 47 | 0.29 | MDC |
| TET2 | 4 | 106197285 | 106197285 | Missense Mutation | SNP | T | C | p.I1873T | 15 | 85 | 0.15 | MDC |
| TET2 | 4 | 106197401 | 106197401 | Missense Mutation | SNP | C | G | p.H1912D | 9 | 87 | 0.0937 5 | Biol mage |
| TP53 | 17 | 7577120 | 7577120 | Missense Mutation | SNP | C | T | p.R273H | 18 | 231 | 0.0722 89 | Biol mage |
| TP53 | 17 | 7577120 | 7577120 | Missense Mutation | SNP | C | T | p.R273H | 15 | 253 | 0.0559 7 | Biol mage |
| TP53 | 17 | 7577120 | 7577120 | Missense Mutation | SNP | C | T | p.R273H | 12 | 245 | 0.0466 93 | MDC |
| ZRSR2 | X | 15821832 | 15821832 | Nonsense Mutation | SNP | G | A | p.W75* | 10 | 13 | 0.4347 83 | Biol mage |

| | | | | | | | | | | | | |
|---|---|---|---|---|---|---|---|---|---|---|---|---|
| all.=allele; CHIP=clonal hematopoiesis of indeterminate potential; Chr=chromosome; Del=deletion; INS=insertion; Pos.=position; Ref=reference; SNP=single nucleotide polymorphism; VAF=variant allele fraction; Var.=variant. | | | | | | | | | | | | |

The median age of participants in BioImage at the time of DNA sample collection was 70 years, the median follow-up time was 2.6 years, and the prevalence of clonal hematopoiesis was 11.9%. Data showed that 19/113 (16.8%) coronary heart disease cases had CHIP as compared to 25/257 (9.7%) controls (hazard ratio (HR) 1.8, 95% confidence interval 1.1-2.9, Wald p=0.03 from a Cox proportional hazards model adjusted for age, sex, type 2 diabetes status, total cholesterol, high density lipoprotein cholesterol, hypertension, and smoking status) **(****Figure 2A-B, Table 4).**

**Table 4. Cox regression model for risk of coronary heart disease**

| **Biolmage** | | | |
|---|---|---|---|
| ***n=370*** | **HR** | **95% Cl** | **p-value** |
| **CHIP** | 1.76 | 1.05-2.92 | 0.03 |
| **Age** | 0.97 | 0.94-1 | 0.08 |
| **Female** | 0.97 | 0.64-1.45 | 0.87 |
| **Has type 2 diabetes** | 0.86 | 0.56-1.32 | 0.48 |
| **Has hypertension** | 2.14 | 1.22-3.75 | 0.008 |
| **Current or former smoker** | 0.96 | 0.57-1.61 | 0.88 |
| **HDL-C** | 0.99 | 0.98-1.01 | 0.22 |
| **Total cholesterol** | 1.00 | 1.00-1.01 | 0.26 |

| **MDC** | | | |
|---|---|---|---|
| ***n=640*** | **HR** | **95% Cl** | **p-value** |
| **CHIP** | 1.97 | 1.25-3.12 | 0.003 |
| **Age** | 1.07 | 0.83-1.37 | 0.62 |
| **Female** | 1.00 | 0.98-1.02 | 0.7 |
| **Has type 2 diabetes** | 0.94 | 0.69-1.29 | 0.72 |
| **Has hypertension** | 1.88 | 1.43-2.45 | 0.000004 |
| **Current or former smoker** | 1.11 | 0.87-1.41 | 0.41 |
| **HDL-C** | 0.99 | 0.98-1 | 0.01 |
| **Total cholesterol** | 1.00 | 1.00-1.00 | 0.4 |

The participants in MDC had a median age of 60 years at the time of DNA sample collection, median follow-up time of 17.7 years, and a prevalence of clonal hematopoiesis of 5.2%. CHIP occurred in 21/320 (6.5%) of coronary heart disease cases but only 12/320 (3.8%) controls (HR 2.0, 95% confidence interval 1.2-3.1, Wald p=0.003 from a Cox proportional hazards model adjusted as above) **(****Figure 2A-B, Table 4).**

Combined analysis of both cohorts in a fixed-effects meta-analysis showed that those with clonal hematopoiesis had a 1.9-fold greater risk of incident coronary heart disease (95% confidence interval 1.4-2.7, p=0.0002) **(****Figure 2A****).**

### Example 2. Risk of coronary heart disease associates with clone size

The effect of clone size on disease outcomes might be expected to be greatest in the near-term; over several years, small clones may expand into larger ones *(see* Jaiswal 2014), mitigating the effect of clone size at the time of initial DNA sampling. In BioImage, a cohort with a short duration of follow-up, the risk for coronary heart disease was greatest among those with a variant allele fraction above or equal to the median (13.5%, corresponding to a clone size of -27% of nucleated peripheral blood cells) compared to those without mutations (HR 2.5, 95% confidence interval 1.3-4.9, Wald p=0.007 from a Cox proportional hazards model adjusted as above) **(****Figure 2C****),** a result that resembles prior exploratory analysis on CHIP and coronary heart disease *(see* Jaiswal 2014). In contrast, in MDC, a cohort with a much longer median follow-up time, that individuals with clones below and above the median had a similarly elevated risk of coronary heart disease (HR 2.1, Wald p=0.05 and HR 1.9, Wald p=0.02 by a Cox proportional hazards model adjusted as above, respectively).

### Example 3. Clonal hematopoiesis associates with early-onset myocardial infarction

Having established an association between clonal hematopoiesis and coronary heart disease in the older individuals, it was next investigated whether CHIP was a risk factor for early-onset (age <50 years) myocardial infarction. Whole exome sequencing data was analyzed from the Atherosclerosis, Thrombosis, and Vascular Biology Italian Study Group (ATVB, see ATVB Italian Study Group, Circulation 107:1117-22 (2003)).

In the ATVB cohort, cases consist of individuals with early-onset myocardial infarction events selected at the time of index presentation to hospitals, with cardiovascular disease-free individuals drawn from the same medical centers as controls. Cases were age 45 or younger and age-matched to controls. In total, there were 1,753 cases and 1,583 controls from ATVB. A panel of 75 genes was used to define CHIP mutations. As expected, this younger cohort had a much lower overall prevalence of CHIP (1.3% in ATVB). Table 5 presents the baseline characteristics for this cohort.

**Table 5. Baseline characteristics of subjects in ATVB**

| | | **Cases** | **Controls** |
|---|---|---|---|
| **Number of individuals** | | 1,753 | 1,583 |
| | *No CHIP* | 1,716 | 1,577 |
| | *CHIP* | 37 | 6 |
| **Age, median** | | 41 | 40 |
| | *No CHIP* | 42 | 40 |
| | *CHIP* | 41 | 41 |
| **Female sex, n (Percent)** | | 189 (11.0) | 184 (11.6) |
| | *No CHIP* | 185 (10.7) | 184 (11.7) |
| | *CHIP* | 4 (10.8) | 0 (0.0) |
| **Smoker, n (Percent)** | | 800 (45.6) | 491 (31.0) |
| | *No CHIP* | 781 (45.5) | 489 (31.0) |
| | *CHIP* | 19 (51.3) | 2 (33.3) |
| **Has T2D, n (Percent)** | | 102 (5.8) | 9 (0.6) |
| | *No CHIP* | 99 (5.8) | 9 (0.6) |
| | *CHIP* | 3 (8.1) | 0 (0.0) |

To test for an association between CHIP and early-onset MI, previously generated whole exome sequencing data from ATVB were utilized. Individuals were excluded if information on type 2 diabetes status and smoking status was not available. All remaining cases and controls aged 45 or younger were used. Cases were matched to controls by age based on the original study design, as described above.

An association between myocardial infarction and CHIP was tested using a logistic regression model that also included age, sex, type 2 diabetes status, and smoking status as co-variables. A fixed effects meta-analysis of the two cohorts was performed using the R package 'meta'.

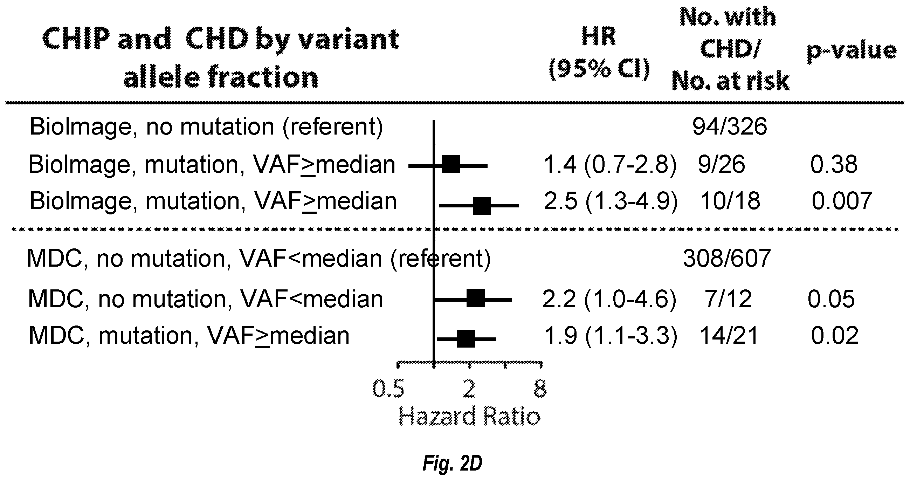

The early-onset myocardial infarction cases had marked enrichment of CHIP compared to controls. In ATVB, 37/1,753 (2.1%) of myocardial infarction cases had CHIP as compared to 6/1,583 (0.4%) controls (odds ratio (OR) 5.4, 95% confidence interval 2.3-13, Wald p=0.0002 from a logistic regression model adjusted for age, sex, type 2 diabetes status, and smoking status). (Fig 2D).

### Example 4. Mutations in DNMT3A, TET2, ASXL1, and JAK2 individually associate with risk of coronary events

To understand which CHIP genes significantly contributed to risk of coronary heart disease, a gene-level analysis was performed on three sets of cohorts: BioImage/MDC, three prospective cohorts unselected for coronary heart disease events: Jackson Heart Study (JHS), the Finland United States Study of NIDDM Genetics (FUSION), and Framingham Heart Study (FHS) *(see* Feinleib 1975), and ATVB **(****Figures 3A-B).** JHS and FUSION were part of a prior association study of CHIP with coronary heart disease *(see* Jaiswal 2014), while FHS was newly analyzed for this study.

Associations between clonal hematopoiesis due to specific mutations and coronary heart disease were tested. The genes *DNMT3A, TET2, ASXL1,* and *JAK2* were selected to specifically test for associations, and all other gene mutations were classified as "other". Rarely, individuals had multiple genes mutated. In these cases, they were classified into the group of specifically picked genes if the other mutation was in the "other" group (e.g., someone with a mutation in *TET2* and *CBL* would be classified into the *TET2* group). If an individual had mutations in multiple genes in the specifically picked group, they were classified based on the mutated gene with the highest variant allele fraction (e.g. someone with a *TET2* mutation with a VAF 23% and a *DNMT3A* mutation with VAF 9% was classified as *TET2).*

For ATVB, a logistic regression using mutated gene was performed as a factor with 6 levels *(DNMT3A* mutation, *TET2* mutation, *ASXL1* mutation, JAK2 mutation, other mutation, or no mutation) in addition to the covariables described above. However, the logistic regression model could not assign weights to some mutations that were only present in cases ("structural zeros"). For example, JAK2 mutations and *ASXL1* mutations were only present in myocardial infarction cases, and not controls. For this reason, odds ratios and p-values were reported based on Fisher's exact test for each gene, using those with no mutations as the comparator group. The p-values from Fisher's exact test were not corrected for multiple hypothesis testing.

For BioImage and MDC, the analysis was performed using a Cox proportional hazards model using mutated gene as a factor with 6 levels (*DNMT3A* mutation, *TET2* mutation, *ASXL1* mutation, JAK2 mutation, other mutation, or no mutation), in addition to the co-variables described above.

Three prospective, population-based cohorts were also assessed. Two of these, Jackson Heart Study (JHS) and the Finland Unite States Study of NIDDM Genetics (FUSION), were previously analyzed (Jaiswal 2014). Whole exome sequencing was also analyzed from 608 individuals in the Framingham Heart Study not previously analyzed for clonal hematopoiesis. Coronary heart disease events were defined as fatal or non-fatal myocardial infarction or coronary revascularization procedures, and those with prevalent events were excluded from analysis. To test for gene-level associations, data were aggregated from all three cohorts into a single, combined analysis because the number of coronary heart disease cases was too small to provide robust gene-level associations for each cohort individually. Because these were population-based cohorts not selected for coronary phenotypes, a combined analysis did not interfere with case-control matching. A Cox proportional hazards model with mutated gene as a factor with 6 levels (*DNMT3A* mutation, *TET2* mutation, *ASXL1* mutation, JAK2 mutation, other mutation, or no mutation), in addition to age (categorical variable, <50 years, 50-59 years, 60-69 years, > 70 years), sex, type 2 diabetes status, total cholesterol, high density lipoprotein cholesterol, smoking status (current or former smoker versus never smoker), and hypertension (systolic blood pressure > 160 mm Hg) as covariables was used.

Within the cohorts with older individuals, *DNMT3A* and *JAK2* significantly associated with coronary heart disease in BioImage and MDC, while *TET2* and JAK2 significantly associated with coronary heart disease in a combined analysis of JHS, FUSION, and FHS **(****Figure 3A****).**

Mutations in *TET2, JAK2,* and *ASXL1* were markedly enriched in early-onset myocardial infarction cases in ATVB (3/3, 9/9, and 7/7 of individuals with these mutations were myocardial infarction cases, respectively, **Figure 3B****).**

### Example 5. Clonal hematopoiesis in humans is associated with increased subclinical atherosclerosis

It was next evaluated whether the association between CHIP and coronary heart disease was driven by increased atherosclerosis, as opposed to other factors that might cause myocardial infarction such as increased thrombosis or vasospasm. To test this, data was evaluated for coronary artery calcification, a noninvasive measure of subclinical coronary atherosclerosis detected by cardiac computed tomography. Coronary artery calcification scores were available for 326 individuals from BioImage with incident coronary heart disease and matched controls, including 36 with CHIP.

Coronary artery calcification (CAC) scores were obtained from participants at the time of study enrollment as previously described (Muntendam P, et al., Am Heart J 160:49O57 e1 (2010)). To test for associations between CHIP and coronary artery calcification, computed tomography generated coronary artery calcification scores were log-transformed in Agatston units (natural logarithm (coronary artery calcification score +1)) and used linear regression with presence of CHIP, age (continuous variable), sex, type 2 diabetes status, total cholesterol (continuous variable), high density lipoprotein cholesterol (continuous variable), hypertension (categorical variable), and smoking status (current or former smoker versus never smoker) as co-variables. In a separate analysis, CHIP was used with a mutation variant allele fraction below or greater than or equal to the median (13.5%) as a variable, in addition to the other co-variables listed above, in a linear regression model.

Median coronary artery calcification scores were 3.4-fold greater in those with CHIP than those without CHIP (534 versus 156 Agatston units, Wald p-value 0.04 by a linear regression model for log-transformed coronary artery calcification score adjusted for age, sex, type 2 diabetes status, total cholesterol, high density lipoprotein cholesterol, hypertension, and smoking status). Those with CHIP with a variant allele fraction above or equal to 13.5% had a median coronary artery calcification score that was 4.6-fold greater than those without mutations (712 versus 156 Agatston units, Wald p=0.02 by a linear regression model adjusted as above). **(****Figure 5A****).**

A coronary artery calcification score of greater than 615 Agatston units has been proposed as an empiric cutoff for identifying older individuals at high-risk for coronary events *(see* Elias-Smale SE et al., J Am Coll Cardiol 56:1407-14 (2010)). It was evaluated whether CHIP with larger clone size increased the likelihood of being in this high-risk group. Those with a variant allele fraction above the median had an 8.9-fold increased risk of being in the high-risk coronary artery calcification group compared to those without mutations (95% confidence interval 4.8-17, p=0.0003 in a logistic regression model adjusted for age, sex, type 2 diabetes status, total cholesterol, high density lipoprotein cholesterol, hypertension, and smoking status) **(****Figure 5B****).**

In summary, coronary events in the near-term increased in relation to clone size and, a dose-response relationship between clone size and subclinical atherosclerosis was observed by imaging. Further, a younger cohort showed an even stronger association between CHIP and coronary heart disease.

### Example 6. Mice with loss of Tet2 function in hematopoietic cells display accelerated atherosclerosis

The human genetic data demonstrated that mutations causing clonal hematopoiesis are robustly associated with coronary heart disease and subclinical atherosclerosis, but this association alone does provide evidence for a causal connection. To assess causality for atherosclerotic cardiovascular disease in one of these CHIP genes, mice with loss-of-function of *Tet2* in all hematopoietic cells (*Tet2*-/-; *Vav1*-Cre mice) were evaluated.

All animals used in these experiments were housed with a standard LD12:12 schedule and had *ad libitum* access to food and water. In line with NIH Guide Notice NOT-OD-15-102, both male and female mice were used in this study, noted above in individual experiments.

Strains used in this study include the *Tet2*-floxed line *B6;129STet*2^{tm1.1Iaai}/J (Jax Cat. No. 017573) *(see* Moran-Crusio K et al., Cancer Cell 20:11-24 (2011)) and the hypercholesterolemia-prone *Ldlr* knockout (KO) line *B6;129S7-Ldlr*^{tm1Her}/*J* (Jax Cat. No. 002207). Mice with constitutive expression of Cre recombinase under control of either the *Vav1* promoter (*B6.Cg-Tg(Vav1-icre)A2Kio*/*J* [Jax Cat. No. 008610]) or *LysM* promoter (*B6.129P2-Lyz2*^{tm1(cre)Ifo}/*J* [Jax Cat. No. 004781]) were crossed with the *Tet*2-floxed line to generate animals with *Tet2* KO specific to the entire hematopoietic or myeloid lineages, respectively. Where appropriate, wild-type *Vav1-Cre* or *LysM-Cre* animals were used as controls. *Ldlr* KO mice were crossed with *B6.SJL-Ptprc*^{a} *Pepc*^{b}/*BoyJ* (Jax Cat. No. 002014) to generate *Ldlr* KO mice homozygous for the panleukocyte marker CD45.1. For transplant experiments, female *Ldlr* KO mice were used exclusively as recipients.

All alleles were genotyped by Transnetyx, Inc. (Cordova, TN, USA). In-house verification of transplant engraftment was performed by PCR analysis of bone-marrow-derived DNA following harvest. *Tet2* PCR used a threeprimer reaction with an annealing temperature of 61°C for 30 cycles *(see* Moran-Crusio 2011). The PCR primers are TAGAGGGAGGGGGCATAAGT (LOXP3R, SEQ ID NO: 1), AAGAATTGCTACAGGCCTGC (Flox F; SEQ ID NO: 2), and TTCTTTAGCCCTTGCTGAGC (Flox R; SEQ ID NO: 3). This assay distinguishes between the wild-type allele (248 bp), the floxed allele (480 bp), and the excised allele (580 bp).

For bone marrow transplantation, recipient *Ldlr* KO CD45.1+ mice were lethally irradiated with two doses of γ-irradiation (475 cGy) separated by 4 hours. Donor CD45.2⁺ bone marrow was obtained from *Tet2* +/+, *Tet*2 +/*flox,* or *Tet2 flox*/*flox* littermates. Post-irradiation, recipients were transplanted with 2×10⁶ whole bone marrow cells in suspension via retro-orbital injection. Following transplantation, recipient mice were provided with sterilized cages, food, and water for a period of four weeks. Water was supplemented with antibiotic (trimethoprim-sulfamethoxazole) for the first three weeks after transplant.

To model hypercholesterolemia, mice were started on high fat, high cholesterol diet at four-weeks post-transplant (Harlan-Teklad, TD.96121; 21% MF, 1.25% Chol. Diet). This hypercholesterolemia-promoting regimen was continued for 5, 9, 13, or 17 weeks.

For analysis of peripheral blood, blood was collected from mice via the retro-orbital sinus into EDTA collection tubes at 5 weeks and 10 weeks after initiation of diet. This EDTA-anticoagulated whole blood was run on an Advia 2120 hematology system to obtain a complete blood count. Cellular subpopulations were also identified by flow cytometry on a FACSCANTO II (Becton Dickinson) using APC-conjugated anti-Ly-6G (Affymetrix, Cat. No. 17-9668-80), PE-Cy7-conjugated anti-CD 115 (Affymetrix, Cat. No. 25-1152-82), Alexa-Fluor-780-conjugated anti-CD3 (eBioscience, Cat. No. 47-0032-82), eFluor450-conjugated anti-CD11b (eBioscience, Cat. No. 48-0112), and PE-conjugated conjugated anti-CD19 (eBioscience, Cat. No. 11-0193-82).

Immediately prior to euthanization, peripheral blood samples from overnight-fasted mice were obtained by terminal bleeding via the retro-orbital sinus. Serum was isolated from EDTA-free blood and frozen at -80°C until characterization for lipids or proteins.
For serum lipid measurements, total cholesterol (Wako, Cat. No. 439-17501), high density lipoprotein cholesterol (Wako, Cat. No. 431-52501), and triglycerides (Cayman Chemical, Cat. No. 10010303) were measured in serum from mice at 17 weeks on diet after overnight fasting.

Quantitation of atherosclerosis and histological analysis of organs was carried out using serial cryostat sections of aortic root (6µm) cut from optimal cutting temperature (OCT) embedded unfixed hearts at the level of aortic valves that were stored at -80°C until use.

Aortic root sections were stained with Oil Red O (ORO) (Sigma Aldrich Cat. No. O0625), a lipophilic red dye, to assess plaque accumulation or with Masson's Trichrome 2000 stain (American MasterTech, Cat. No. KTMTR2) to evaluate sclerosis and fibrosis. Images of roots were acquired using a Nikon Eclipse E400 microscope. Quantification of aortic root lesions was performed using ImageJ (www.rsb.info.nih.gov/ij/index.html) on 5 or 6 adjacent, ORO-stained cryostat sections. The total lesion area on each slide was then averaged to obtain a mean lesion area per mouse.

For immunohistochemistry, cryostat sections were fixed in acetone at
-20°C for 5 min, endogenous peroxidase activity was blocked with 0.3% hydrogen peroxide, and nonspecific binding of antibodies was blocked by incubation with PBS, supplemented with 5% normal rabbit serum (NRS). Primary antibodies were applied for 90 min incubation in a wet chamber at RT. Incubation with rabbit anti-rat biotinylated secondary antibody (1:200, mouse absorbed, Vector; Burlingame, CA) for 45 min was followed by streptavidin (ready-to-use, Dako, Carpinteria, CA) for 30 min, and antibody binding visualized with 3-amino-9-ethyl carbazole (AEC, ready-to-use, Dako). Sections were counterstained with Gill's hematoxylin solution (Sigma, St. Louis, MO) and mount using water-soluble mounting media (glycerol-gelatin, Sigma).

Aortas were cut ~5mm inferior to the branchpoint of the subclavian artery and then fixed in 10% formalin overnight, cleaned of visceral fat deposits, opened longitudinally, pinned onto plates containing, and then stained with ORO *en face.* A Nikon D7000 camera was used to take pictures and the extent of plaques in the root or aorta or sclerosis in the root was quantified in ImageJ. The total ORO staining portion was divided by the total area of the pinned descending aorta to obtain a proportion of aorta involved by lesion.

At the time of harvest, liver, lungs, spleen, and ileum were collected. A portion of each tissue was formalin-fixed-paraffin-embedded (FFPE) and was sectioned and stained with hematoxylin and eosin (H/E). After formalin-fixation and decalcification, heads were sectioned along the sagittal axis and stained for H/E. Images were acquired on a Nikon Eclipse E400 microscope.

Primary antibodies used were rat anti-mouse Mac3 1:900 (cat No.553322), CD4 1:90 (clone RM4-5, cat No.553043), MHC-class II 1:250 (a mouse I-A/I-E, cat No.556999) antibodies (all from BD Pharmingen, Franklin Lakes, NJ). Rat anti-Mac2 1:100 (cat No.CL8942AP, Cedarlane labs, Burlington, Canada) was used on paraffin sections after pre-treatment of tissue sections with hit retrieval solution (Cat No. S1699, Dako).

For quantification of immunohistochemistry (IHC) positive areas, the Colour Deconvolution module in ImageJ2 (Fiji release) in H DAB mode. A threshold was applied to Color 1 (hematoxylin) and Color 2 (3,3'-diaminobenzidine) to convert to black and white pixels. The total number of pixels in Color 2 was divided by total number of pixels in Color 1 to give a percent area that stained positive for IHC.

For statistical comparisons between groups, a Welch's t-test was used when 2 groups were compared, and Dunn's Kruskal-Wallis test for multiple comparisons with Benjamini-Hochberg correction was used when 3 groups were being compared with the R package 'dunn.test' (www.cran.r-project.org/web/packages/dunn.test/index.html).

*Tet*2 was selected because it is the second most commonly mutated gene in CHIP and significantly associated with higher risk of coronary heart disease in two sets of human cohorts in a previous study **(****Figure 3B** **and 3C).** Previous studies have demonstrated that hematopoietic stem cells from these mice recapitulate the clonal advantage of *TET2* mutant hematopoietic cells seen in humans *(see* Moran-Crusio 2011). Bone marrow from these mice, or control mice, was transplanted into irradiated atherosclerosis-prone *Ldlr-*/*-* recipient mice *(see* Ishibashi S et al., J Clin Invest 92:883-93 (1993)), and a high-fat, high-cholesterol diet was initiated after allowing time for hematopoietic reconstitution.

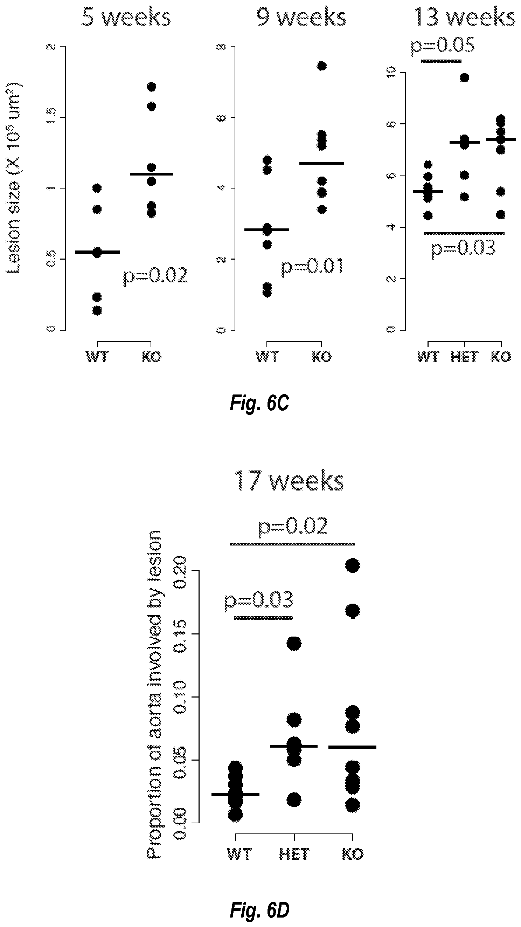

Mice that received *Tet2-*/*-* bone marrow had larger atherosclerotic lesions at all time points tested compared to mice that received control bone marrow. The mean lesion size in the aortic root was 2.2-fold (p=0.02 by Wilcoxon rank sum test), 1.7-fold (p=0.01 by Wilcoxon rank sum test), and 1.3-fold (p=0.03 by Dunn's test) larger in the mice receiving *Tet2-*/*-* bone marrow after 5 weeks, 9 weeks, and 13 weeks on diet, respectively **(****Figure 6A** **and** **6C****).** By 17 weeks on diet, mice receiving *Tet2-*/*-* marrow also had a mean lesion size in the descending aorta that was 3.2-fold larger than mice receiving control marrow (p=0.02 by Dunn's test) **(****Figure 6B** **and** **6D****).**

Most humans with *TET2* clonal hematopoiesis have only a single mutant allele. Therefore, the phenotype *Tet2*+/- bone marrow transplanted into *Ldlr-*/*-* mice was also evaluated. Results with *Tet2*+/- bone marrow transplant indicated a similar increase in atherosclerosis at both 13 and 17 weeks on diet seen with as *Tet2-*/*-* marrow transplant. At 13 weeks on diet, *Ldlr-*/*-* mice receiving *Tet2*+/- bone marrow had a mean aortic root lesion size that was 1.3-fold larger than mice receiving control marrow (p=0.05 by Dunn's test) (Figure 6C). Similarly, the mean lesion size in the descending aorta at 17 weeks on diet was 2.7-fold larger in the mice receiving *Tet2*+/- marrow as compared to mice receiving control marrow (p=0.03 by Dunn's test) **(****Figure 6B** **and** **6D****).**

Fasting serum lipoprotein levels in each group showed no statistically significant differences after 17 weeks on diet (Table 6).

**Table 6. Serum lipid parameter and blood cell indexes in transplanted mice**

| | | ***Tet2*+/+;*Vav1-Cre*** | ***Tet2*-/-;*Vav1-Cre*** |
|---|---|---|---|
| 17 weeks on diet | **Total Cholesterol (mg/dL)** | 1200±230 | 1130±185 |
| | **HDL** | 115±16 | 111±41 |
| | **Triglycerides** | 455±126 | 486±289 |
| | | ***Tet2*+/+;*Vav1*-Cre** | ***Tet2-*/-;*Vav1-Cre*** |
| 5 weeks on diet | **WBC (K/uL)** | 7.3±2.9 | 8.2±1.9 |
| | **Hgb (g/dL)** | 13.4±2.4 | 13.4±2.4 |
| | **Hct** (%) | 50.1±8.6 | 49.1±8.2 |
| | **Plt (K/uL)** | 786±193 | 696±181 |
| | **Monocytes (K/uL)** | 0.77±0.25 | 0.94±0.46 |
| | **Granulocytes (K/uL)** | 1.0±0.33 | 1.1±0.46 |
| | **Lymphocytes (K/uL)** | 5.1±2.5 | 5.5±1.2 |
| | | ***Tet2*+/+;*Vav1*-Cre** | ***Tet2-*/*-;Vav1*-Cre** |
| 11 weeks on diet | **WBC (K/uL)** | 11.9±2.7 | 6.9±1.0 |
| | **Hgb (g/dL)** | 13.3±0.6 | 13.8±1.8 |
| | **Hct** (%) | 50.9±3.4 | 50.5±5.8 |
| | **Plt (K/uL)** | 632±262 | 614±282 |
| | **Monocytes (K/uL)** | 1.5±0.38 | 0.64±0.23 |
| | **Granulocytes (K/uL)** | 0.92±0.22 | 0.88±0.40 |
| | **Lymphocytes (K/uL)** | 8.6±2.2 | 4.7±0.8 |

Differential was performed by flow cytometry. Monocytes - CD11b+ Ly6G- CD115+ CD3- CD19-; Granulocytes - CD11b+ Ly6G+ CD115-CD3- CD19-; Lymphocytes - CD11b- Ly6G- CD115- CD3+ CD19- or CD11b-Ly6G- CD115- CD3- CD19+;
HDL - high density lipoprotein cholesterol; WBC - white blood cell; Hgb - hemoglobin; Hct - hematocrit; Plt - Platelets; and K - thousand.

### Example 7. Loss of Tet2 function in myeloid cells enhances atherosclerosis in mice and alters macrophage inflammatory gene expression in vitro and in vivo

The earliest stages of atherosclerosis involve monocyte infiltration into vessel walls, differentiation into macrophages, and consequent foam cell formation *(see* Ross R N Engl J Med 340:115-26 (1999)). Mice receiving *Tet2*-/- marrow had larger lesion sizes even at very early time points, which may suggest that *Tet2* loss modulated macrophage function in plaques to enhance atherosclerosis. This hypothesis was tested by generating mice that lacked *Tet2* in the majority of myeloid cells, but not other lineages (*Tet2*-/-*; Lyz2-Cre) (see* Abram CL et al., J Immunol Methods 408:89-100 (2014)). In *Ldlr-*/*-* mice transplanted with marrow from these mice, the mean aortic root lesion size was 1.7-fold larger than mice receiving control marrow after 10 weeks on diet (p=0.003 by Wilcoxon rank sum test) **(****Figure 6E****).**

Next, the mechanism by which *Tet2* loss promotes atherogenesis was studied. Tet2 catalyzes DNA hydroxymethylation *(see* Tahiliani M et al., Science 324:930-5 (2009)), an epigenetic modification that can influence gene transcription. Therefore, Tet2 may modulate gene expression in macrophages in response to environmental stimuli such as excess cholesterol or bacterial endotoxin. Bone marrow-derived macrophages (BMDM) were cultured from *Tet2-*/*-* or control mice and exposed to either vehicle or a pathophysiology-relevant dose of native low-density lipoprotein (LDL, 200 mg/dL) *(see* Smith EB et al., Eur Heart J 11(Suppl E):72-81 (1990) and Kruth HS Curr Opin Lipidol 22:386-93 (2011)), and the transcriptome was analyzed by RNA-sequencing.

To generate BMDM, whole bone marrow was isolated from long bones, hips, and vertebrae of 10-14 week old mice by crushing and sequential passage through 70 µm and 40 µm cell strainers (Corning Cat. No. 352350 and 352340). Red cell lysis with 1X PharmLyse (BD Biosciences Cat. No. 555899) was performed and bone marrow was cultured by creating a single-cell suspension of whole bone marrow in Iscove's Modification of DMEM (IMDM) (Corning Cat. No. 10016CV) supplemented with 10% fetal bovine serum (FBS) (Omega Scientific Cat. No. FB-11), 10 ng/mL recombinant mouse macrophage colony stimulating factor (MCSF, Miltenyi Biotec Cat. No. 130-101-706), and 1% penicillin/streptomycin/glutamine (PSG) (Gibco Cat. No. 10378-016) in 30 mL total volume. After 3 days, each dish was supplemented with 15mL of the above media, and macrophages were harvested on day 6 with a cell lifter.

For stimulation of BDMD, cells were grown as described above and harvested on day 6 of culture and re-plated into 48 well plates (750,000 cells per well) in IMDM with 10% FBS, 1% PSG, and 10 ng/mL M-CSF. After 24 hours, the media was replaced with media containing LDL, LPS, or vehicle as described below. Native human low-density lipoprotein (LDL, Alfa Aeser, Cat No. BT-903) was resuspended to a final concentration of 200 mg/dL, along with 10% FBS, 1% PSG, and 10 ng/mL recombinant mouse M-CSF into 1X IMDM from powdered stock (Life Technologies, Cat. No. 12200036). For vehicle treated samples, LDL was replaced with 0.05M TRIS-HCl buffer, with 0.15M NaCl and 0.3mM EDTA, pH 7.4 in the above mixture.

Lipopolysaccharide (LPS, Sigma Aldrich, Cat. No. L4391) from Escherichia coli was also used in some experiments at a final concentration of 10 ng/mL in 1X IMDM with 10% FBS, 1% PSG, and 10 ng/mL M-CSF. For vehicle treated samples, LPS was replaced with phosphate buffered saline (Gibco).

For RNA sequencing, BMDM were treated with LDL or vehicle as described above and harvested after 24h using Trizol reagent (Invitrogen, Cat. No. 15596026). RNA was purified using RNeasy Mini columns (Qiagen, Cat. No. 74104) followed by DNase treatment (TURBO DNA-free Kit, Life Technologies, Cat. No. AM1907).

Ribo-Zero Kit (Illumina, Cat. No. MRZH116) was used to eliminate ribosomal RNA. Library preparation using poly-A selection, multiplexing, and sequencing on two HiSeq2500 lanes were done by Genewiz (South Plainfield, NJ). A total of 10 samples were sequenced (3 *Tet2*+/+ untreated, 3 *Tet2*-/- untreated, *2 Tet2*+/+ LDL treated, and 2 LDL-/- treated).

Reads were then mapped to the *Mus musculus* mm10 reference genome with the CLC Genomics Server program v. 9.0.1. Normalized read counts were obtained from the resulting BAM files using the BiocLite (www.bioconductor.org/biocLite.R) package in R. Differential gene expression was analyzed using the Deseq2 (www.bioconductor.org/packages/release/bioc/html/DESeq2.html) package in R considering the effect of LDL treatment and genotype as separate variables in a linear model (design = ~ genotype + treatment). Genes were assigned p-values based on being differentially expressed due to genotype, and separate p-values were obtained for differential expression based on treatment. Genes with q<0.05 were considered significant in each respective analysis.

Gene set enrichment analysis (www.software.broadinstitute.org/gsea/index.jsp) was performed using the Kyoto Encyclopedia of Genes and Genomes gene set.

For chemokine and cytokine measurements, an ELISA was used to measure the amount of mouse CXCL1 (Abcam, Cat. No. ab100717), mouse CXCL2 (Abcam, Cat. No. ab204517), mouse CXCL3 (Abcam, Cat. No. ab206310), mouse CXCL4 (Abcam, Cat. No. ab100735), mouse IL-6 (R&D Systems, Cat. No. M6000B), mouse IL-1b (Abcam, Cat. No. ab197742), and mouse CXCL7 (Abcam, Cat. No. ab100742) in various experiments as noted in the text. For statistical comparisons between groups, a Welch's t-test was used when 2 groups were compared, and a Dunn's Kruskal-Wallis test for multiple comparisons with Benjamini-Hochberg correction was used when 3 groups were being compared with the R package 'dunn.test' (www.cran.r-project.org/web/packages/dunn.test/index.html).

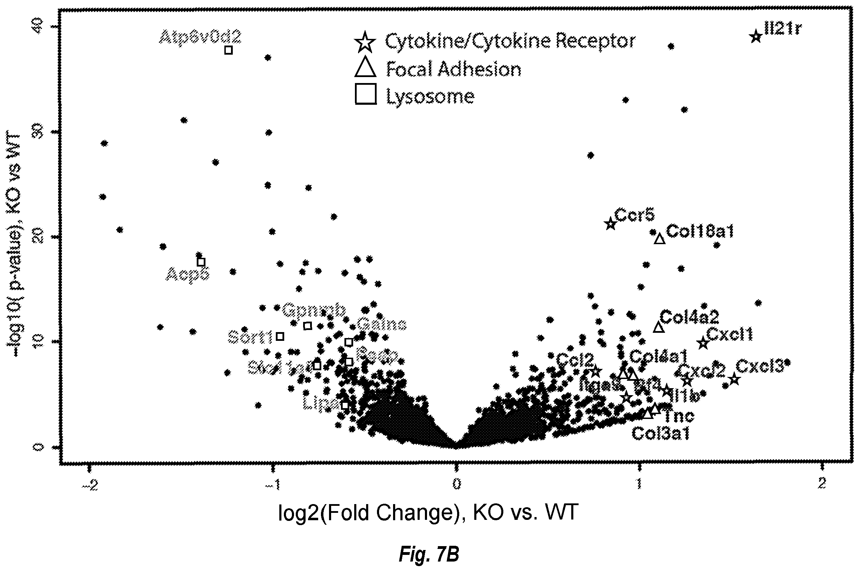

At a false discovery rate of less than 5%, 2,010 genes were differentially regulated between *Tet2*-/- and *Tet2*+/+ macrophages, and 479 genes were differentially regulated by LDL treatment **(****Figure 7A****).** Gene set enrichment analysis revealed that the most significantly up-regulated (KEGG) pathway sets in *Tet2-*/*-* macrophages contained cytokines/chemokines and receptors and focal adhesion genes (including *Col3a1*, *Col4a1*, and *Col18a1*) shown on the right side of Figure 7B. The most significantly suppressed set contained genes involved in lysosomal function (including *Lipa* and *Sort1*) as shown on the left side of Figure 7B. **(Tables 7-8 and** **Figure 7B-C).**

**Table 7. Most significantly up-regulated KEGG pathways**

| | | | **FDR** | **FWER** |
|---|---|---|---|---|
| **NAME** | **SIZE** | **NES** | **q-val** | **p-val** |
| KEGG_CYTOKINE_CYTOKINE_RECEPTOR_INTERACTION | 95 | 1.897 | 1.00E-03 | 0.001 |
| KEGG_FOCAL_ADHESION N | 132 | 1.863 | 0.001 | 0.003 |
| KEGG_ECM_RECEPTOR_INTERACTION | 44 | 1.814 | 0.006 | 0.018 |
| KEGG_NOD_UKE_RECEPTOR_SIGNAUNG_PATHWAY | 41 | 1.783 | 0.006 | 0.024 |
| KEGG_CELL_ADHESION_MOLECULES_CAMS | 45 | 1.75 | 0.009 | 0.047 |
| KEGG_ADHERENS_JUNCTION | 52 | 1.666 | 0.034 | 0.205 |
| KEGG_AXON_GUIDANCE | 76 | 1.655 | 0.036 | 0.242 |
| KEGG_TGF_BETA_SIGNAUNG_PATHWAY | 53 | 1.621 | 0.054 | 0.369 |
| KEGG_PRION_DISEASES | 25 | 1.599 | 0.066 | 0.472 |
| KEGG_SMALL_CELL_LUNG_CANCER | 64 | 1.593 | 0.065 | 0.505 |
| KEGG_VIRAL_MYOCARDITIS | 29 | 1.592 | 0.06 | 0.508 |
| KEGG_PATHWAYS_IN_CANCER | 208 | 1.559 | 0.083 | 0.669 |
| KEGG_LEISHMANlA_INFECTION | 39 | 1.529 | 0.111 | 0.791 |
| KEGG_GAP_JUNCTION | 50 | 1.528 | 0.104 | 0.794 |
| KEGG_CHEMOKINE_SIGNAUNG_PATHWAY | 120 | 1.527 | 0.098 | 0.798 |
| KEGG_CYTOSOLIC_DNA_SENSING_PATHWAY | 36 | 1.512 | 0.108 | 0.839 |
| KEGG_HYPERTROPHIC_CARDIOMYOPATHY_HCM | 38 | 1.491 | 0.128 | 0.896 |
| KEGG_INTESTINAL_IMMUNE_NETWORK_FOR_IGA_PROD | 16 | 1.478 | 0.137 | 0.928 |
| KEGG_HEDGEHOG_SIGNALING_PATHWAY | 18 | 1.478 | 0.131 | 0.93 |
| KEGG_ARRHYTHMOGENIC_RIGHT_VENTRICULAR_CARDIC | 32 | 1.44 | 0.179 | 0.984 |
| KEGG_BLADDER_CANCER | 29 | 1.422 | 0.204 | 0.992 |
| KEGG_BASAL_CELL_CARCINOMA | 16 | 1.414 | 0.208 | 0.993 |
| KEGG_WNT_SIGNAUNG_PATHWAY | 91 | 1.412 | 0.204 | 0.994 |
| KEGG_LEUKOCYTE_TRANSENDOTHEUAL_MlGRATION | 66 | 1.411 | 0.196 | 0.994 |
| KEGG_MELANOGENESIS | 50 | 1.376 | 0.256 | 0.999 |
| KEGG_JAK_STAT_SIGNALlNG_PATHWAY | 77 | 1.374 | 0.249 | 0.999 |
| KEGG_DILATED_CARDIOMYOPATHY | 40 | 1.373 | 0.242 | 0.999 |
| KEGG_DORSO_VENTRAL_AXIS_FORMATION | 16 | 1.371 | 0.237 | 0.999 |
| KEGG_NEUROACTIVE_UGAND_RECEPTOR_INTERACTION | 34 | 1.338 | 0.296 | 1 |
| KEGG_HEMATOPOIETIC_CELL_LINEAGE | 34 | 1.321 | 0.325 | 1 |
| KEGG_REGULATIO_OF_ACTIN_CYTOSKELETON | 135 | 1.31 | 0.34 | 1 |
| KEGG_TIGHT_JUNCTION | 66 | 1.292 | 0.375 | 1 |
| KEGG_P53_SIGNALING_PATHWAY | 50 | 1.29 | 0.369 | 1 |
| KEGG_ONE_CARBON_POOL_BY_FOLATE | 16 | 1.289 | 0.359 | 1 |
| KEGG_MAPK_SIGNAUNG_PATHWAY | 169 | 1.277 | 0.38 | 1 |
| KEGG_GLYCOSAMlNOGLYCAN_BIOSYNTHESIS_HEPARAN_ | 15 | 1.274 | 0.377 | 1 |
| KEGG_RIG_I_UKE_RECECEPTOR_SIGNALING_PATHWAY | 40 | 1.269 | 0.377 | 1 |
| KEGG_TOLL_UKE_RECEPTOR_SIGNALING_PATHWAY | 73 | 1.251 | 0.412 | 1 |
| KEGG_CALCIUM_SIGNALING_PATHWAY | 72 | 1.243 | 0.421 | 1 |
| KEGG_ERBB_SIGNAUNG_PATHWAY | 65 | 1.243 | 0.412 | 1 |
| KEGG_NOTCH_SIGNAUNG_PATHWAY | 34 | 1.226 | 0.446 | 1 |
| KEGG_ARGININE_AND_PROUNE_METABOLISM | 34 | 1.204 | 0.493 | 1 |
| KEGG_PYRIMIDINE_METABOLISM | 77 | 1.202 | 0.488 | 1 |
| KEGG_COMPLEMENT_AND_OOAGULATION_CASCADES | 18 | 1.197 | 0.488 | 1 |
| KEGG_VEGF_SIGNALING_PATHWAY | 52 | 1.197 | 0.478 | 1 |
| KEGG_PROSTATE_CANCER | 71 | 1.192 | 0.481 | 1 |
| KEGG_CELL_CYCLE | 105 | 1.189 | 0.478 | 1 |
| KEGG_DNA_REPUCATION | 33 | 1.167 | 0.523 | 1 |
| KEGG_MELANOMA | 46 | 1.162 | 0.527 | 1 |
| KEGG_GNRH_SIGNALING_PATHWAY | 63 | 1.158 | 0.527 | 1 |
| KEGG_GALAGTOSE_METABOLISM | 19 | 1.152 | 0.531 | 1 |
| KEGG_PANCREATIC_CANCER | 61 | 1.143 | 0.545 | 1 |
| KEGG_NtOOTINATE_AND_NICOTINAMIDE_METABOUSM | 15 | 1.141 | 0.539 | 1 |

**Table 7. Most significantly up-regulated KEGG pathways (cont'd.)**

| | | | | |
|---|---|---|---|---|
| KEGG_PATHOGENIC_ESCHERICHlA_COLI_INFECTION | 35 | 1135 | 0.543 | 1 |
| KEGG_ARACHIDONIC_ACID_METABOLISM | 17 | 1.128 | 0.552 | 1 |
| KEGG_RENAL_CELL_CARCINOMA | 60 | 1.119 | 0.564 | 1 |
| KEGG_EPITHEUAL_CELL_SIGNALING_IN_HEUCOBACTER_F | 50 | 1.088 | 0.637 | 1 |
| KEGG_APOPTOSSS | 68 | 1.079 | 0.649 | 1 |
| KEGG_SPLICEOSOME | 91 | 1.07 | 0.661 | 1 |
| KEGG_COLORECTAL_CANCER | 53 | 1.06 | 0.674 | 1 |
| KEGG_HOMOLOGOUS_RECOMBINATION | 23 | 1.057 | 0.672 | 1 |
| KEGG_FRUCTOSE_AND_MANNOSE_METABOLISM | 27 | 1.054 | 0.669 | 1 |
| KEGG_GUOMA | 50 | 1.048 | 0.672 | 1 |
| KEGG_ENDOCYTOSIS | 111 | 1.022 | 0.73 | 1 |
| KEGG_ADIPOCYTOKINE_SIGNALING_PATHWAY | 44 | 1.021 | 0.721 | 1 |
| KEGG_VASCULAR_SMOOTH_MUSCLE_CONTRACTION | 63 | 0.977 | 0.817 | 1 |
| KEGG_NATURAL_KILLER_CELL_MEDIATED_CYTOTOXICITY | 68 | 0.957 | 0.855 | 1 |
| KEGG_TYPE_II_DIABETES_MELLITUS | 27 | 0.945 | 0.872 | 1 |
| KEGG_MISMATCH_REPAIR | 22 | 0.94 | 0.871 | 1 |
| KEGG_BASE_EXCISION_REPAIR | 32 | 0.924 | 0.894 | 1 |
| KEGG_INOSITOL_PHOSPHATE_METABOLISM | 37 | 0.923 | 0.884 | 1 |
| KEGG_PHOSPHATIDYUNOSITOL_SIGNAUNG_SYSTEM | 52 | 0.909 | 0.904 | 1 |
| KEGG_T_CELL_RECEPTOR_SIGNALlNG_PATHWAY | 77 | 0.908 | 0.894 | 1 |
| KEGG_SYSTEMIC_UUPUS_ERYTHEMATOSUS | 27 | 0.869 | 0.966 | 1 |
| KEGG_ANTIGEN_PROCESSING_AND_PRESENTATION | 32 | 0.862 | 0.968 | 1 |
| KEGG_INSULlN_SIGNAUNG_PATHWAY | 99 | 0.835 | 1 | 1 |
| KEGG_PURINE_METABOUSM | 107 | 0.833 | 1 | 1 |
| KEGG_PENTOSE_PHOSPHATE_PATHWAY | 18 | 0.817 | 1 | 1 |
| KEGG_OOCYTE_MEIOSIS | 81 | 0.817 | 1 | 1 |
| KEGG_STARCH_AND_SUCROSE_METABOLISM | 18 | 0.811 | 1 | 1 |
| KEGG_ENDOMETRIAL_CANCER | 40 | 0.811 | 0.993 | 1 |
| KEGG_B_CELL_RECEPTOR_SIGNAUNG_PATHWAY | 67 | 0.804 | 0.993 | 1 |
| KEGG_GLYCOLYSIS_GLUCONEOGENESIS | 37 | 0.793 | 1 | 1 |
| KEGG_VASOPRESSIN_REGULATED_WATER_REABSORPTIO | 33 | 0.784 | 1 | 1 |
| KEGG_PRIMARY_IMMUNODEFIOENCY | 17 | 0.781 | 0.997 | 1 |
| KEGG_LONG_TERM_DEPRESSION | 38 | 0.75 | 1 | 1 |
| KEGG_NON_SMALL_CELL_LUNG_CANCER | 44 | 0.748 | 1 | 1 |
| KEGG_PROGESTERONE_MEDIATED_OOCYTE_MATURATIC | 64 | 0.747 | 1 | 1 |
| KEGG_CARDIAC_MUSCLE_CONTRACTION | 33 | 0.744 | 1 | 1 |
| KEGG_CHRONIC_MYELOID_LEUKEMIA | 66 | 0.742 | 0.997 | 1 |
| KEGG_UBIOUITIN_MEDIATED_PROTEOLYSIS | 114 | 0.726 | 1 | 1 |
| KEGG_AMINO_SUGAR_AND_NUCLEOTIDE_SUGAR_META | 39 | 0.645 | 1 | 1 |
| KEGG_FC_EPSILON_RI_SIGNALINGG_PATHWAY | 54 | 0.643 | 1 | 1 |
| KEGG_LYSINE_DEGRADATION | 34 | 0.638 | 1 | 1 |
| KEGG_AMINOACYL_TRNA_BIOSYNTHESIS | 32 | 0.609 | 1 | 1 |
| KEGG_ALZHEIMERS_DISEASE | 123 | 0.604 | 1 | 1 |
| HEGG_DRUG_METABOLISM_OTHER_ENZYMES | 15 | 0.593 | 1 | 1 |
| KEGG_PROTEASOME | 41 | 0.568 | 1 | 1 |
| KEGG_ACUTE_MYELOID_LEUKEMIA | 48 | 0.564 | 1 | 1 |
| KEGG_NUCLEOTIDE_EXCISION_REPAIR | 42 | 0.557 | 1 | 1 |
| KEGG_BASAL_TRANSCRIPTION_FACTORS | 29 | 0.551 | 1 | 1 |
| KEGG_HUNTINGTONS_DISEASE | 139 | 0.548 | 1 | 1 |
| KEGG_RNA_DEGRADATION | 48 | 0.458 | 1 | 1 |
| KEGG_PROTEIN_EXPORT | 17 | 0.371 | 1 | 1 |

| | | | | |
|---|---|---|---|---|
| **NES normalized enrichment score** **FDR false discover rate** **FWER family-wise error rate** | | | | |

**Table 8. Most significantly down-regulated KEGG pathways**

| | | | **FDR** | **FWER** |
|---|---|---|---|---|
| **NAME** | **SIZE** | **NES** | **q-val** | **p-val** |
| KEGG_LYSOSOME | 102 | -2.079 | 0.002 | 0.002 |
| KEGG_DRUG_METABOLISM_CYTOCHROME_P450 | 17 | -1.668 | 0.195 | 0.255 |
| KEGG_SELENOAMINO_AOD_METABOLISM | 16 | -1.608 | 0.213 | 0.392 |
| KEGG_REGULATION_OF_AUTOPHAGY | 18 | -1.584 | 0.188 | 0.445 |
| KEGG_OXIDATIE_PHOSPHORYLATION | 95 | -1.58 | 0.157 | 0.459 |
| KEG G_PROPANOATE_METABOLISM | 25 | -1.505 | 0.218 | 0.642 |
| KEGG_BUTANOATE_METABOLISM | 20 | -1.502 | 0.19 | 0.649 |
| KEGG_GLYCEROPHOSPHOLIPID_METABOLISM | 40 | -1.482 | 0.189 | 0.694 |
| KEGG_GLYCEROLIPID_METABOLISM | 24 | -1.476 | 0.175 | 0.709 |
| KEGG_PEROXISOME | 57 | -1.472 | 0.161 | 0.716 |
| KEG G_CYSTEINE_AND_METHIONINE_METABOLISM | 18 | -1.432 | 0.191 | 0.805 |
| KEG G_SNARE_INTERACTIONS_IN_VESICULAR_TRANSPORT | 27 | -1.422 | 0.187 | 0.824 |
| KEG G_PPAR_SIGNALING_PATHWAY | 34 | -1.382 | 0.217 | 0.898 |
| KEGG_ALDOSTERONE_REGULATED_SODIUM_REABSORPION | 22 | -1.356 | 0.229 | 0.928 |
| KEG G_VALINE_LEUCINE_AND_ISOLEUCINE_DEGRADATION | 36 | -1.35 | 0.221 | 0.937 |
| KEG G_ABC_TRANSPORTERS | 22 | -1.346 | 0.212 | 0.939 |
| KEGG_GLYCOSAMINOGLYCAN_DEGRADATION | 15 | -1.322 | 0.226 | 0.95 |
| KEG G_METABOLISM_OF_XENOBIOTICS_BY_CYTOCHROME_P450 | 18 | -1.194 | 0.412 | 0.996 |
| KEGG_THYROID_CANCER | 20 | -1.159 | 0.466 | 0.999 |
| KEGG_GLUTATHIONE_METABOLISM | 34 | -1.124 | 0.518 | 1 |
| KEGG_TRYPTOPHAN_METRBOLISM | 18 | -1.096 | 0.561 | 1 |
| KEG G_SPHINGOLIPID_METABOLISM | 22 | -1.094 | 0.539 | 1 |
| KEG G_VIBRIO_CHOLERAE_INFECTION | 40 | -1.077 | 0.554 | 1 |
| KEGG_ALANINE_ASPARTATE_AND_GLUTAMATE_METABOLISM | 18 | -1.01 | 0.702 | 1 |
| KEGG_PYRUVATE_METABOLlSM | 26 | -0.993 | 0.719 | 1 |
| KEG G_FATTY_ACID_METABOLISM | 28 | -0.979 | 0.731 | 1 |
| KEGG_RIBOSOME | 81 | -0.972 | 0.721 | 1 |
| KEGG_MTOR_SIGNALING_PATHWAY | 40 | -0.898 | 0.891 | 1 |
| KEGG_AMVOTROPHIC_LATERAL_SCLEROSIS_ALS | 32 | -0.893 | 0.874 | 1 |
| KEGG_N_GLYCAN_BIOSYNTHESIS | 40 | -0.868 | 0.901 | 1 |
| KEGG_LONGTERM_POTENTIATION | 44 | -0.77 | 1 | 1 |
| KEGG_PORPHYRIN_AND_CHLOROPHYLL_METABOLISM | 21 | -0.759 | 1 | 1 |
| KEGG_FC_GAMMA_R_MEDIATED_PHAGOCYTOSIS | 71 | -0.757 | 1 | 1 |
| KEG G_GLYCOSYLPHOSPHATIDYLINOSITOL_GPI_ANCHOR_BIOSYNTHESIS | 23 | -0.755 | 0.996 | 1 |
| KEG G_NEUROTROPHIN_SIGNALlNG_PATHWAY | 97 | -0.751 | 0.973 | 1 |
| KEG G_TYROSINE_METABOLISM | 15 | -0.739 | 0.96 | 1 |
| KEGG_PARKINSONS_DISEASE | 92 | -0.715 | 0.959 | 1 |
| KEGG_CITRATE_CYCLE_TCA_CYCLE | 25 | -0.676 | 0.962 | 1 |
| KEGG_RNA_POLYMERASE | 27 | -0.473 | 0.997 | 1 |

| | | | | |
|---|---|---|---|---|
| **NES normalized enrichment score** **FDR false discovery rate** **FWER family-wise error rate** | | | | |

The set of 217 genes that were differentially regulated by both loss of *Tet2* and by LDL treatment were further studied. *Cxcl1*, *Cxcl2*, *Cxcl3, Pf4, Il6*, and *Il1b* transcript levels were among the most highly induced in *Tet2-*/*-* macrophages in this set **(****Figures 8A****, 9A).** *Cxcl1*, *Cxcl2*, *Cxcl3,* and *Pf4* belong to a single C-X-C motif (CXC) chemokine gene cluster, while *Il6* and *Il1b* are classic pro-inflammatory cytokine genes. *Tet2-*/*-* macrophages also secreted more of these proteins *in vitro* in response to LDL loading and/or endotoxin exposure than control macrophages, corroborating the increased level of messenger RNA. While either LDL or endotoxin (LPS) strongly induced the CXC chemokines, endotoxin but not LDL caused robust secretion of IL-1b and IL-6 **(****Figure 9B****).** Therefore, the CXC chemokines may be the most relevant targets of modulation by Tet2 in atherosclerosis.

To assess the *in vivo* significance of the *in vitro* findings, CXC chemokine levels were measured in the transplanted mice after 13-17 weeks on diet. Cxcl1, Cxcl2, Cxcl3, Pf4, and Ppbp levels increased ~2-4 fold in the serum of *Ldlr-*/*-* mice receiving *Tet2-*/*-* marrow (KO) compared to mice receiving control marrow (WT), while mice receiving *Tet2*+/- marrow (HET) showed intermediate levels **(****Figure 8B****).** The CXC family chemokines were initially thought to selectively promote migration of neutrophils via the receptor CXCR2 *(see* Baggiolini M et al., FEBS Lett 307:97-101 (1992)). However, in humans as well as mice, CXC chemokine/CXCR2 interaction can also mediate firm monocyte adhesion to inflamed endothelium *(see* Gerszten RE et al., Nature 398:718-23 (1999) and Schwartz D et al., J Clin Invest 94:1968-73 (1994)), and this interaction promotes atherogenesis *(see* Boisvert WA et al., J Clin Invest 101:353-63 (1998) and Huo Y et al., J Clin Invest 108:1307-14 (2001)). If *Tet2* deficient macrophages caused accelerated atherosclerosis because of augmented production of monocyte and neutrophil chemoattractants, evidence of this may be seen in tissues beyond the vessel wall. Indeed, *Ldlr-l*-mice that received *Tet2-*/*-* marrow had large xanthomas in the spleen and middle ear, marked foam cell accumulation and glomerulosclerosis in the renal glomeruli, and large inflammatory infiltrates in the liver and lung **(****Figures 8C****,** **Figure 9C****).** These changes were unlikely to result from leukocytosis alone as these mice had normal peripheral blood counts and white blood cell differential **(Table 6),** similar to humans with *TET2* mutated CHIP *(see* Jaiswal 2014).

Because increased levels of CXC chemokines were found in the serum of mice receiving *Tet2-*/*-* marrow, an analogous increase in humans with *TET2* clonal hematopoiesis was evaluated. The prototypical CXC chemokine in humans is IL-8, which mice lack.

In summary, experimental *Tet2* deficiency in hematopoietic cells of hyperlipidemic mice accelerates atherosclerosis. Further, studies of gene expression changes in *Tet2* mutant macrophages exposed to LDL demonstrated increased expression of inflammatory mediators implicated in atherosclerosis development.

### Example 8: Assessment of Loss of TET2 Function in Human Monocytic Cells

To test whether loss of TET2 function led to alterations in chemokine expression in human monocytic cells, we used CRISPR to introduce frameshift mutations into the THP1 cell line. Human monocytic THP1 cells were transduced with virus containing Cas9, GFP, and either control (non-targeting) guide or guide targeting exon 3 of human TET2. Transduced cells were sorted on the basis of GFP expression and frameshift mutations in TET2 were confirmed by sequencing. The CRISPR-edited cells were then exposed to E. coli derived LPS (100 ng/mL for 1 x 10₆cells) for 24 hours. Chemokines secreted into the media were measured using ELISA. Results shown are total amount of chemokine secreted into media from 1 x 106 cells. Compared to cells receiving a control guide, cells with engineered TET2 mutations produced nearly twice as much IL-8 and CXCL2. **(****Figure 10****).**

### Example 9: Loss of Dnmt3a function in myeloid cells enhances atherosclerosis in mice and alters macrophage inflammatory gene expression in vitro

Most humans with CHIP have heterozygous loss of function mutations in the gene *Dnmt3a.* To model this situation, we transplanted bone marrow from either *Dnmt3a*+/+ (WT) or *Dnmt3a*+/- (HET) mice into mice lacking the gene for the low-density lipoprotein receptor (*Ldlr*-/-) and initiated a high cholesterol diet. Mice receiving HET marrow had 31% larger lesion size than mice receiving WT bone marrow, which may suggest that *Dnmt3a* loss modulated macrophage function in plaques to enhance atherosclerosis. **(****Figure 11-12****).**

Next, the mechanism by which *Dnmt3a* loss promotes atherogenesis was studied. Dnmt3a catalyzes cytosine methylation of DNA, an epigenetic modification that can influence gene transcription. Therefore, Dnmt3a may modulate gene expression in macrophages in response to environmental stimuli such as excess cholesterol. Bone marrow-derived macrophages (BMDM) were cultured from *Dnmt3a*-/- (KO) or control *Dnmt3a*+/+ (WT) mice and exposed to either vehicle or a pathophysiologically-relevant dose of native low-density lipoprotein (LDL, 200 mg/dL) *(see* Smith EB et al., Eur Heart J 11(Suppl E):72-81 (1990) and Kruth HS Curr Opin Lipidol 22:386-93 (2011)), and the transcriptome was analyzed by RNA-sequencing.

To generate BMDM, whole bone marrow was isolated from long bones, hips, and vertebrae of 10-14 week-old mice by crushing and sequential passage through 70 µm and 40 µm cell strainers (Corning Cat. No. 352350 and 352340). Red cell lysis with 1X PharmLyse (BD Biosciences Cat. No. 555899) was performed and bone marrow was cultured by creating a single-cell suspension of whole bone marrow in Iscove's Modification of DMEM (IMDM) (Corning Cat. No. 10016CV) supplemented with 10% fetal bovine serum (FBS) (Omega Scientific Cat. No. FB-11), 10 ng/mL recombinant mouse macrophage colony stimulating factor (MCSF, Miltenyi Biotec Cat. No. 130-101-706), and 1% penicillin/streptomycin/glutamine (PSG) (Gibco Cat. No. 10378-016) in 30 mL total volume. After 3 days, each dish was supplemented with 15mL of the above media, and macrophages were harvested on day 6 with a cell lifter.

For stimulation of BDMD, cells were grown as described above and harvested on day 6 of culture and re-plated into 48 well plates (750,000 cells per well) in IMDM with 10% FBS, 1% PSG, and 10 ng/mL M-CSF. After 24 hours, the media was replaced with media containing LDL, LPS, or vehicle as described below. Native human low-density lipoprotein (LDL, Alfa Aeser, Cat No. BT-903) was resuspended to a final concentration of 200 mg/dL, along with 10% FBS, 1% PSG, and 10 ng/mL recombinant mouse M-CSF into 1X IMDM from powdered stock (Life Technologies, Cat. No. 12200036). For vehicle treated samples, LDL was replaced with 0.05M TRIS-HCl buffer, with 0.15M NaCl and 0.3mM EDTA, pH 7.4 in the above mixture.

For RNA sequencing, BMDM were treated with LDL or vehicle as described above and harvested after 24h using Trizol reagent (Invitrogen, Cat. No. 15596026). RNA was purified using RNeasy Mini columns (Qiagen, Cat. No. 74104) followed by DNase treatment (TURBO DNA-free Kit, Life Technologies, Cat. No. AM1907).

Ribo-Zero Kit (Illumina, Cat. No. MRZH116) was used to eliminate ribosomal RNA. Library preparation using poly-A selection, multiplexing, and sequencing were done by Broad Institute (Cambridge, MA). A total of 11 samples were sequenced (3 *Dnmt3a*+/+ untreated, 3 *Dnmt3a*-/- untreated, 2 *Dnmt3a*+/+ LDL treated, and 3 *Dnmt3a*-/- LDL-/- treated).

Reads were then mapped to the *Mus musculus* mm10 reference genome. Normalized read counts were obtained from the resulting BAM files using the BiocLite (www.bioconductor.org/biocLite.R) package in R. Differential gene expression was analyzed using the Deseq2 (www.bioconductor.org/packages/release/bioc/html/DESeq2.html) package in R considering the effect of LDL treatment and genotype as separate variables in a linear model (design = ~ genotype + treatment). Genes were assigned p-values based on being differentially expressed due to genotype, and separate p-values were obtained for differential expression based on treatment. Genes with q<0.05 were considered significant in each respective analysis.

Gene set enrichment analysis (www.software.broadinstitute.org/gsea/index.jsp) was performed using the Kyoto Encyclopedia of Genes and Genomes gene set.

For chemokine and cytokine measurements, an ELISA was used to measure the amount of mouse CXCL1 (Abcam, Cat. No. ab100717), mouse CXCL2 (Abcam, Cat. No. ab204517), mouse CXCL3 (Abcam, Cat. No. ab206310), mouse IL-6 (R&D Systems, Cat. No. M6000B), and mouse IL-1b (Abcam, Cat. No. ab197742), in various experiments as noted in the text. For statistical comparisons between groups, a Welch's t-test was used when 2 groups were compared.

At a false discovery rate of less than 5%, 2,171 genes were differentially regulated between *Dnmt3a*-/- and *Dnmt3a* +/+ macrophages, and 2,530 genes were differentially regulated by LDL treatment. Gene set enrichment analysis revealed that the most significantly up-regulated (KEGG) pathway sets in *Dnmt3a*-/- macrophages contained inflammatory genes such as cytokines/chemokines and receptors (Table 9). This pattern of gene expression in *Dnmt3a-*/*-* macrophages was similar to that of *Tet2-*/*-* macrophages, surprisingly.

The set of 771 genes that were differentially regulated by both loss of *Dnmt3a* and by LDL treatment were further studied. *Cxcl1*, *Cxcl2*, *Cxcl3, Il6*, and *Il1b* transcript levels were among the most highly induced in *Dnmt3a* -/macrophages in this set **(****Figures 13-15****).** *Cxcl1*, *Cxcl2*, and *Cxcl3* belong to a single C-X-C motif (CXC) chemokine gene cluster, while *Il6*, and *Il1b* are classic pro-inflammatory cytokine genes. *Tet2-*/*-* macrophages also secreted more of these proteins *in vitro* in response to LDL loading, corroborating the increased level of messenger RNA. **(****Figure 15****).** Therefore, these chemokines and cytokines may be relevant targets in atherosclerosis due to *DNMT3A* mutations.

Loss of *Dnmt3a* function in development of atherosclerosis in mice results in increased aortic root lesion size. In Figure 16 A-D show that a loss of Dnmt3a expression in mice results in increase aortic root lesion size. Figure. A) Female Ldlr-/- mice were transplanted with either 10% Dnmt3a-/-, Vav1-Cre + 90% Dnmt3a+/+, Vav1-Cre, or with 100% Dnmt3a+/+, Vav1-Cre bone marrow cells. After 4 weeks, high cholesterol diet (1.25% cholesterol) was initiated. Nine weeks later, the aortic root lesion size was analyzed histologically. B) Mice receiving 10% Dnmt3a-/- cells had a lesion size that was 40% larger than mice receiving control cells. C-D) Representative oil red O aortic root sections are shown for mice receiving only wildtype bone marrow (WT), or 10% Dnmt3a-/- bone marrow (10% KO).

In summary, experimental *Dnmt3a* deficiency in hematopoietic cells of hyperlipidemic mice accelerates atherosclerosis. Further, studies of gene expression changes in *Dnmt3a* mutant macrophages exposed to LDL demonstrated increased expression of inflammatory mediators implicated in atherosclerosis development.

| **Table 9** | | |
|---|---|---|
| **NAME** | **NES** | **FDR q-val** |
| KEGG_HEMATOPOIETIC_CELL_LINEAGE | 2.4375436 | 0 |
| KEGG_NOD_LIKE_RECEPTOR_SIGNALING_PATHWAY | 2.156624 | 6.25E-04 |
| KEGG_CYTOKINE_CYTOKINE_RECEPTOR_ INTERACTION | 2.1218443 | 4.17E-04 |
| KEGG_ADIPOCYTOKINE_SIGNALING_PATHWAY | 1.9949135 | 0.002980937 |
| KEGG_NEUROACTIVE_LIGAND_RECEPTOR_ INTERACTION | 1.9678583 | 0.003598128 |
| KEGG_GLUTATHIONE_METABOLISM | 1.8761564 | 0.012096957 |
| KEGG_CALCIUM_SIGNALING_PATHWAY | 1.8156636 | 0.024347756 |
| KEGG_LEUKOCYTE_TRANSENDOTHELIAL_ MIGRATION | 1.757996 | 0.039623767 |
| KEGG_JAK_STAT_SIGNALING_PATHWAY | 1.7456719 | 0.04000333 |
| KEGG_CELL_ADHESION_MOLECULES_CAMS | 1.7137383 | 0.047562506 |
| KEGG_LEISHMANIA_INFECTION | 1.7108891 | 0.044534314 |
| KEGG_NEUROTROPHIN_SIGNALING_PATHWAY | 1.6837822 | 0.05306327 |
| KEGG_TOLL_LIKE_RECEPTOR_SIGNALING_PATHWAY | 1.6818763 | 0.049970098 |
| KEGG_PATHWAYS_IN_CANCER | 1.6660783 | 0.05417887 |
| KEGG_TYPE_II_DIABETES_MELLITUS | 1.6533483 | 0.05686756 |
| KEGG_PORPHYRIN_AND_CHLOROPHYLL_ METABOLISM | 1.6310356 | 0.064673394 |
| KEGG_PEROXISOME | 1.6264845 | 0.06346458 |
| KEGG_B_CELL_RECEPTOR_SIGNALING_PATHWAY | 1.6243453 | 0.06087831 |
| KEGG_T_CELL_RECEPTOR_SIGNALING_PATHWAY | 1.6173933 | 0.061086133 |
| KEGG_DORSO_VENTRAL_AXIS_FORMATION | 1.6111614 | 0.06152469 |
| KEGG_APOPTOSIS | 1.5948374 | 0.06704734 |
| KEGG_INSULIN_SIGNALING_PATHWAY | 1.5802207 | 0.07162565 |
| KEGG_ABC_TRANSPORTERS | 1.5631554 | 0.07761307 |
| KEGG_MTOR_SIGNALING_PATHWAY | 1.5388408 | 0.089753255 |
| KEGG_RENAL_CELL_CARCINOMA | 1.520161 | 0.10000245 |
| KEGG_PENTOSE_PHOSPHATE_PATHWAY | 1.5061929 | 0.10542796 |
| KEGG_FC_EPSILON_RI_SIGNALING_PATHWAY | 1.4930495 | 0.11254347 |
| KEGG_ACUTE_MYELOID_LEUKEMIA | 1.472533 | 0.124891445 |
| KEGG_ALDOSTERONE_REGULATED_SODIUM_ REABSORPTION | 1.4704108 | 0.1223177 |
| KEGG_LYSOSOME | 1.4661239 | 0.12173255 |
| KEGG_ECM_RECEPTOR_INTERACTION | 1.4273145 | 0.1535949 |
| KEGG_SPHINGOLIPID_METABOLISM | 1.3800248 | 0.19942488 |
| KEGG_CHRONIC_MYELOID_LEUKEMIA | 1.3419701 | 0.24157485 |
| KEGG_ERBB_SIGNALING_PATHWAY | 1.3377087 | 0.24066778 |
| KEGG_FRUCTOSE_AND_MANNOSE_METABOLISM | 1.3359432 | 0.23639324 |
| KEGG_FOCAL_ADHESION | 1.3327136 | 0.23447825 |
| KEGG_CYTOSOLIC_DNA_SENSING_PATHWAY | 1.3248084 | 0.23890111 |
| KEGG_CHEMOKINE_SIGNALING_PATHWAY | 1.3245015 | 0.23305275 |
| KEGG_PPAR_SIGNALING_PATHWAY | 1.3236754 | 0.22822179 |
| KEGG_GLYCEROLIPID_METABOLISM | 1.3116921 | 0.2376819 |
| KEGG_PROSTATE_CANCER | 1.3102691 | 0.23370388 |
| KEGG_VASCULAR_SMOOTH_MUSCLE_CONTRACTION | 1.3034991 | 0.23729333 |
| KEGG_ADHERENS_JUNCTION | 1.3010145 | 0.23459163 |
| KEGG_NATURAL_KILLER_CELL_MEDIATED_ CYTOTOXICITY | 1.2996798 | 0.23113127 |
| KEGG_VEGF_SIGNALING_PATHWAY | 1.2873526 | 0.24094264 |
| KEGG_TGF_BETA_SIGNALING_PATHWAY | 1.2803128 | 0.24524792 |
| KEGG_STARCH_AND_SUCROSE_METABOLISM | 1.2787697 | 0.24212633 |
| KEGG_ARRHYTHMOGENIC_RIGHT_VENTRICULAR_ CARDIOMYOPATHY_ARVC | 1.2636044 | 0.25705186 |
| KEGG_NOTCH_SIGNALING_PATHWAY | 1.2628195 | 0.25256312 |
| KEGG_MELANOGENESIS | 1.2556198 | 0.25642222 |
| KEGG_EPITHELIAL_CELL_SIGNALING_IN_ HELICOBACTER_PYLORI_INFECTION | 1.2539703 | 0.25345713 |
| KEGG_SMALL_CELL_LUNG_CANCER | 1.2427871 | 0.26373625 |
| KEGG_COMPLEMENT_AND_COAGULATION_ CASCADES | 1.2363237 | 0.26733762 |
| KEGG_ARGININE_AND_PROLINE_METABOLISM | 1.2177081 | 0.2880928 |
| KEGG_MAPK_SIGNALING_PATHWAY | 1.2170558 | 0.28370464 |
| KEGG_GNRH_SIGNALING_PATHWAY | 1.1909842 | 0.31647843 |
| KEGG_BLADDER_CANCER | 1.184118 | 0.3211035 |
| KEGG_HYPERTROPHIC_CARDIOMYOPATHY_HCM | 1.1798418 | 0.32165626 |
| KEGG_ENDOMETRIAL_CANCER | 1.1726468 | 0.32680392 |
| KEGG_MELANOMA | 1.1606491 | 0.33805138 |
| KEGG_GLIOMA | 1.1548382 | 0.34200808 |
| KEGG_PHOSPHATIDYLINOSITOL_SIGNALING_SYSTEM | 1.1477474 | 0.34734458 |
| KEGG_REGULATION_OF_AUTOPHAGY | 1.1400114 | 0.3531016 |
| KEGG_LONG_TERM_DEPRESSION | 1.1325778 | 0.35873744 |
| KEGG_PANCREATIC_CANCER | 1.132314 | 0.35358933 |
| KEGG_NON_SMALL_CELL_LUNG_CANCER | 1.1299928 | 0.35179275 |
| KEGG_FC_GAMMA_R_MEDIATED_PHAGOCYTOSIS | 1.101384 | 0.39127344 |
| KEGG_ALANINE_ASPARTATE_AND_GLUTAMATE_ METABOLISM | 1.0995522 | 0.38807273 |
| KEGG_ARACHIDONIC_ACID_METABOLISM | 1.0684364 | 0.43219072 |
| KEGG_PRION_DISEASES | 1.0447196 | 0.46612352 |
| KEGG_GLYCEROPHOSPHOLIPID_METABOLISM | 1.0417484 | 0.4645672 |
| KEGG_TRYPTOPHAN_METABOLISM | 1.0416081 | 0.45847207 |
| KEGG_DILATED_CARDIOMYOPATHY | 1.0144956 | 0.5001834 |
| KEGG_COLORECTAL_CANCER | 0.94184285 | 0.6326295 |
| KEGG_RIG_I_LIKE_RECEPTOR_SIGNALING_PATHWAY | 0.92332906 | 0.6600244 |
| KEGG_VIBRIO_CHOLERAE_INFECTION | 0.88898605 | 0.7170797 |
| KEGG_THYROID_CANCER | 0.8864258 | 0.7128076 |
| KEGG_REGULATION_OF_ACTIN_CYTOSKELETON | 0.88241583 | 0.711826 |
| KEGG_SNARE_INTERACTIONS_IN_VESICULAR_ TRANSPORT | 0.86853206 | 0.7282674 |
| KEGG_WNT_SIGNALING_PATHWAY | 0.8391328 | 0.7719613 |
| KEGG_BASAL_CELL_CARCINOMA | 0.7695292 | 0.87444025 |
| KEGG_LONG_TERM_POTENTIATION | 0.7461875 | 0.8961165 |
| KEGG_HEDGEHOG_SIGNALING_PATHWAY | 0.70608956 | 0.9301718 |
| KEGG_VASOPRESSIN_REGULATED_WATER_ REABSORPTION | 0.56186324 | 0.99675226 |
| KEGG_N_GLYCAN_BIOSYNTHESIS | 0.46328568 | 0.99788266 |

### DESCRIPTION OF CERTAIN SEQUENCES

| **Table 10** | | |
|---|---|---|
| **Description** | **Sequence** | **SEQ ID NO** |
| LOXP3R primer | TAGAGGGAGGGGGCATAAGT | 1 |
| FLOX F primer | AAGAATTGCTACAGGCCTGC | 2 |
| FLOX R primer | TTCTTTAGCCCTTGCTGAGC | 3 |

## Claims

1. An IL-8 inhibitor, IL-6 inhibitor, and/or IL-1β inhibitor for use in a method of treating atherosclerosis in a human subject, the method comprising administering an effective amount of at least one IL-8 inhibitor, IL-6 inhibitor, and/or IL-1β inhibitor, wherein the subject has at least one loss-of-function *TET2* mutation and/or at least one loss-of-function *DNMT3A* mutation, thereby treating atherosclerosis.

2. The inhibitor for the use in a method of treating atherosclerosis in a human subject according to claim 1, wherein presence of said mutations has been determined by sequencing at least a part of a genome comprising *TET2* and/or *DNMT3A* of one or more cells in a blood sample of the subject.

3. The inhibitor for the use in a method of treating atherosclerosis in a human subject according to claim 1 or 2, wherein the subject further has an increased level of plasma IL-8, wherein the level of plasma IL-8 is at least 20 ng/mL.

4. The inhibitor for the use in a method of treating atherosclerosis in a human subject according to any of the preceding claims, wherein the at least one *TET2* and/or *DNMT3A* mutation comprises a frameshift mutation, nonsense mutation, missense mutation, or splice-site variant mutation.

5. The inhibitor for the use in a method of treating atherosclerosis in a human subject according to claim 4, wherein the mutation in *TET2* results in an amino acid change in *TET2* chosen from S145N, S282F, A308T, N312S, L346P, P399L, S460F, D666G, S817T, P941S, C1135Y, R1167T, I1175V, S1204C, R1214W, D1242R, D1242V, Y1245S, R1261C, R1261H, R1261L, F1287L, W1291R, K1299E, K1299N, R1302G, E1318G, P1367S, C1396W, L1398R, V1417F, G1869W, L1872P, I1873T, C1875R, H1881Q, H1881R, R1896M, R1896S, S1898F, V1900A, G1913D, A1919V, R1926H, P1941S, P1962L, R1966H, R1974M, and R2000K.

6. The inhibitor for the use in a method of treating atherosclerosis in a human subject according to claim 4, wherein the mutation in *DNMT3A* results in an amino acid change in *DNMT3A* chosen from F290I, F290C, V296M, P307S, P307R, R326H, R326L, R326C, R326S, G332R, G332E, V339A, V339M, V339G, L344Q, L344P, R366P, R366H, R366G, A368T, A368V, R379H, R379C, I407T, I407N, I407S, F414L, F414S, F414C, A462V, K468R, C497G, C497Y, Q527H, Q527P, Y533C, S535F, C537G, C537R, G543A, G543S, G543C, L547H, L547P, L547F, M548I, M548K, G550R, W581R, W581G, W581C, R604Q, R604W, R635W, R635Q, S638F, G646V, G646E, L653W, L653F, I655N, V657A, V657M, R659H, Y660C, V665G, V665L, M674V, R676W, R676Q, G685R, G685E, G685A, D686Y, D686G, R688H, G699R, G699S, G699D, P700L, P700S, P700R, P700Q, P700T, P700A, D702N, D702Y, V704M, V704G, I705F, I705T, I705S, I705N, G707D, G707V, C710S, C710Y, S714C, V716D, V716F, V716I, N717S, N717I, P718L, R720H, R720G, K721R, K721T, Y724C, R729Q, R729W, R729G, F731C, F731L, F731Y, F731I, F732del, F732C, F732S, F732L, E733G, E733A, F734L, F734C, Y735C, Y735N, Y735S, R736H, R736C, R736P, L737H, L737V, L737F, L737R, A741V, P742P, P743R, P743L, R749C, R749L, R749H, R749G, F751L, F751C, F752del, F752C, F752L, F752I, F752V, W753G, W753C, W753R, L754P, L754R, L754H, F755S, F755I, F755L, M761I, M761V, G762C, V763I, S770L, S770W, S770P, R771Q, F772I, F772V, L773R, L773V, E774K, E774D, E774G, I780T, D781G, R792H, W795C, W795L, G796D, G796V, N797Y, N797H, N797S, P799S, P799R, P799H, R803S, R803W, P804L, P804S, K826R, S828N, K829R, T835M, N838D, K841Q, Q842E, P849L, D857N, W860R, E863D, F868S, G869S, G869V, M880V, S881R, S881I, R882H, R882P, R882C, R882G, A884P, A884V, Q886R, L889P, L889R, G890D, G890R, G890S, V895M, P896L, V897G, V897D, R899L, R899H, R899C, L901R, L901H, P904L, F909C, P904Q, A910P, C911R, C911Y.

7. The inhibitor for the use in a method of treating atherosclerosis in a human subject according to any of the preceding claims, wherein the human subject has at least one somatic blood cell clone with one mutant *TET2* allele and one wildtype *TET2* allele.

8. The inhibitor for the use in a method of treating atherosclerosis in a human subject according to any of claims 1 to 6, wherein the human subject has at least one somatic blood cell clone with two mutant *TET2* alleles.

9. The inhibitor for the use in a method of treating atherosclerosis in a human subject according to any of the preceding claims, wherein the human subject has at least one somatic blood cell clone with one mutant *DNMT3A* allele and one wildtype *DNMT3A* allele.

10. The inhibitor for the use in a method of treating atherosclerosis in a human subject according to any of claims 1 to 8, wherein the human subject has at least one somatic blood cell clone with two mutant *DNMT3A* alleles.

11. The inhibitor for the use in a method of treating atherosclerosis in a human subject according to any of the preceding claims, wherein the human subject has clonal hematopoiesis of indeterminate potential (CHIP).

12. The inhibitor for the use in a method of treating atherosclerosis in a human subject according to any of the preceding claims, wherein the human subject has at least one *TET2* and/or *DNMT3A* mutation with a variant allele fraction of at least 2%*,* 5%*,* 10%, 13.5%, 15%, 20%, 25%, 27%, 30%.

13. The inhibitor for the use in a method of treating atherosclerosis in a human subject according to any of the preceding claims, wherein the subject's plasma level of IL-8 is at least 25 ng/mL, 30 ng/mL, 40 ng/mL, 45 ng/mL, 50 ng/mL, 55 ng/mL, 60 ng/mL, 65 ng/mL, 70 ng/mL, 75 ng/mL, or 80 ng/mL.

14. The inhibitor for the use in a method of treating atherosclerosis in a human subject according to any of the preceding claims, wherein a *TET2* and/or *DNMT3A* mutation is identified by whole exome sequencing (WES) or by sequencing DNA.

## Patentansprüche

1. IL-8-Inhibitor, IL-6-Inhibitor und/oder IL-lß-Inhibitor zur Verwendung in einem Verfahren zum Behandeln von Atherosklerose bei einem menschlichen Subjekt, wobei das Verfahren Verabreichen einer wirksamen Menge von mindestens einem IL-8-Inhibitor, IL-6-Inhibitor und/oder IL-lß-Inhibitor umfasst, wobei das Subjekt mindestens eine *TET2*-Loss-of-function-Mutation und/oder mindestens eine *DNMT3A*-Loss-of-Function-Mutation aufweist, wodurch Atherosklerose behandelt wird.

2. Inhibitor zur Verwendung in einem Verfahren zum Behandeln von Atherosklerose bei einem menschlichen Subjekt nach Anspruch 1, wobei das Vorhandensein der Mutationen durch Sequenzieren mindestens eines Teils eines Genoms, das *TET2* und/oder *DNMT3A* umfasst, von einer oder mehrerer Zellen in einer Blutprobe des Subjekts, bestimmt worden ist.

3. Inhibitor zur Verwendung in einem Verfahren zum Behandeln von Atherosklerose bei einem menschlichen Subjekt nach Anspruch 1 oder 2, wobei das Subjekt ferner einen erhöhten Spiegel von IL-8 im Plasma aufweist, wobei der Spiegel von IL-8 im Plasma mindestens 20 ng/ml beträgt.

4. Inhibitor zur Verwendung in einem Verfahren zum Behandeln von Atherosklerose bei einem menschlichen Subjekt nach einem der vorhergehenden Ansprüche, wobei die mindestens eine *TET2-* und/oder DNMT3A-Mutation eine Frameshift-Mutation, Nonsense-Mutation, Missense-Mutation oder Spleißstellenvariantenmutation umfasst.

5. Inhibitor zur Verwendung in einem Verfahren zum Behandeln von Atherosklerose bei einem menschlichen Subjekt nach Anspruch 4, wobei die Mutation in *TET2* zu einer Aminosäureänderung in *TET2* führt, die gewählt ist aus S145N, S282F, A308T, N312S, L346P, P399L, S460F, D666G, S817T, P941S, C1135Y, R1167T, I1175V, S1204C, R1214W, D1242R, D1242V, Y1245S, R1261C, R1261H, R1261L, F1287L, W1291R, K1299E, K1299N, R1302G, E1318G, P1367S, C1396W, L1398R, V1417F, G1869W, L1872P, I1873T, C1875R, H1881Q, H1881R, R1896M, R1896S, S1898F, V1900A, G1913D, A1919V, R1926H, P1941S, P1962L, R1966H, R1974M und R2000K.

6. Inhibitor zur Verwendung in einem Verfahren zum Behandeln von Atherosklerose bei einem menschlichen Subjekt nach Anspruch 4, wobei die Mutation in *DNMT3A* zu einer Aminosäureänderung in *DNMT3A* führt, die gewählt ist aus F290I, F290C, V296M, P307S, P307R, R326H, R326L, R326C, R326S, G332R, G332E, V339A, V339M, V339G, L344Q, L344P, R366P, R366H, R366G, A368T, A368V, R379H, R379C, I407T, I407N, I407S, F414L, F414S, F414C, A462V, K468R, C497G, C497Y, Q527H, Q527P, Y533C, S535F, C537G, C537R, G543A, G543S, G543C, L547H, L547P, L547F, M548I, M548K, G550R, W581R, W581G, W581C, R604Q, R604W, R635W, R635Q, S638F, G646V, G646E, L653W, L653F, I655N, V657A, V657M, R659H, Y660C, V665G, V665L, M674V, R676W, R676Q, G685R, G685E, G685A, D686Y, D686G, R688H, G699R, G699S, G699D, P700L, P700S, P700R, P700Q, P700T, P700A, D702N, D702Y, V704M, V704G, I705F, I705T, I705S, I705N, G707D, G707V, C710S, C710Y, S714C, V716D, V716F, V716I, N717S, N717I, P718L, R720H, R720G, K721R, K721T, Y724C, R729Q, R729W, R729G, F731C, F731L, F731Y, F731I, F732del, F732C, F732S, F732L, E733G, E733A, F734L, F734C, Y735C, Y735N, Y735S, R736H, R736C, R736P, L737H, L737V, L737F, L737R, A741V, P742P, P743R, P743L, R749C, R749L, R749H, R749G, F751L, F751C, F752del, F752C, F752L, F752I, F752V, W753G, W753C, W753R, L754P, L754R, L754H, F755S, F755I, F755L, M761I, M761V, G762C, V763I, S770L, S770W, S770P, R771Q, F772I, F772V, L773R, L773V, E774K, E774D, E774G, I780T, D781G, R792H, W795C, W795L, G796D, G796V, N797Y, N797H, N797S, P799S, P799R, P799H, R803S, R803W, P804L, P804S,
K826R, S828N, K829R, T835M, N838D, K841Q, Q842E, P849L, D857N, W860R, E863D, F868S, G869S, G869V, M880V, S881R, S881I, R882H, R882P, R882C, R882G, A884P, A884V, Q886R, L889P, L889R, G890D, G890R, G890S, V895M, P896L, V897G, V897D, R899L, R899H, R899C, L901R, L901H, P904L, F909C, P904Q, A910P, C911R, C911Y.

7. Inhibitor zur Verwendung in einem Verfahren zum Behandeln von Atherosklerose bei einem menschlichen Subjekt nach einem der vorhergehenden Ansprüche, wobei das menschliche Subjekt mindestens einen somatischen Blutzellklon mit einem mutierten TET2-Allel und einem Wildtyp-*TET2-*Allel aufweist.

8. Inhibitor zur Verwendung in einem Verfahren zum Behandeln von Atherosklerose bei einem menschlichen Subjekt nach einem der Ansprüche 1 bis 6, wobei das menschliche Subjekt mindestens einen somatischen Blutzellklon mit zwei mutierten TET2-Allelen aufweist.

9. Inhibitor zur Verwendung in einem Verfahren zum Behandeln von Atherosklerose bei einem menschlichen Subjekt nach einem der vorhergehenden Ansprüche, wobei das menschliche Subjekt mindestens einen somatischen Blutzellklon mit einem mutierten *DNMT3A-Allel* und einem Wildtyp-*DNMT3A*-Allel aufweist.

10. Inhibitor zur Verwendung in einem Verfahren zum Behandeln von Atherosklerose bei einem menschlichen Subjekt nach einem der Ansprüche 1 bis 8, wobei das menschliche Subjekt mindestens einen somatischen Blutzellklon mit zwei mutierten DNMT3A-Allelen aufweist.

11. Inhibitor zur Verwendung in einem Verfahren zum Behandeln von Atherosklerose bei einem menschlichen Subjekt nach einem der vorhergehenden Ansprüche, wobei das menschliche Subjekt klonale Hämatopoese von unbestimmtem Potential (CHIP) aufweist.

12. Inhibitor zur Verwendung in einem Verfahren zum Behandeln von Atherosklerose bei einem menschlichen Subjekt nach einem der vorhergehenden Ansprüche, wobei das menschliche Subjekt mindestens eine *TET2-* und/oder *DNMT3A*-Mutation mit einem Variantenallelanteil von mindestens 2 %, 5 %, 10 %, 13,5 %, 15 %, 20 %, 25 %, 27 %, 30 % aufweist.

13. Inhibitor zur Verwendung in einem Verfahren zum Behandeln von Atherosklerose bei einem menschlichen Subjekt nach einem der vorhergehenden Ansprüche, wobei der Plasmaspiegel von IL-8 des Subjekts mindestens 25 ng/ml, 30 ng/ml, 40 ng/ml, 45 ng/ml, 50 ng/ml, 55 ng/ml, 60 ng/ml, 65 ng/ml, 70 ng/ml, 75 ng/ml oder 80 ng/ml beträgt.

14. Inhibitor zur Verwendung in einem Verfahren zum Behandeln von Atherosklerose bei einem menschlichen Subjekt nach einem der vorhergehenden Ansprüche, wobei eine *TET2-* und/oder *DNMT3A-*Mutation durch Ganzexomsequenzierung (WES) oder durch Sequenzieren von DNA identifiziert wird.

## Revendications

1. Inhibiteur d'IL-8, inhibiteur d'IL-6 et/ou inhibiteur d'IL-1β destiné à être utilisé dans un procédé de traitement de l'athérosclérose chez un sujet humain, le procédé comprenant l'administration d'une quantité efficace d'au moins un inhibiteur d'IL-8, un inhibiteur IL-6 et/ou un inhibiteur d'IL-1β, dans lequel le sujet présente au moins une mutation de perte de fonction *TET2* et/ou au moins une mutation de perte de fonction *DNMT3A,* traitant ainsi l'athérosclérose.

2. Inhibiteur destiné à être utilisé dans un procédé de traitement de l'athérosclérose chez un sujet humain selon la revendication 1, dans lequel la présence desdites mutations a été déterminée par séquençage d'au moins une partie d'un génome comprenant *TET2* et/ou *DNMT3A* d'une ou de plusieurs cellules dans un échantillon de sang du sujet.

3. Inhibiteur destiné à être utilisé dans un procédé de traitement de l'athérosclérose chez un sujet humain selon la revendication 1 ou 2, dans lequel le sujet a en outre un niveau accru d'IL-8 plasmatique, dans lequel le niveau d'IL-8 plasmatique est d'au moins 20 ng/ml.

4. Inhibiteur destiné à être utilisé dans un procédé de traitement de l'athérosclérose chez un sujet humain selon l'une quelconque des revendications précédentes, dans lequel l'au moins une mutation *TET2* et/ou *DNMT3A* comprend une mutation par décalage de cadre de lecture, une mutation non-sens, une mutation faux-sens ou une mutation de variante de site d'épissage.

5. Inhibiteur destiné à être utilisé dans un procédé de traitement de l'athérosclérose chez un sujet humain selon la revendication 4, dans lequel la mutation dans *TET2* entraîne un changement d'acide aminé dans *TET2* choisi parmi S145N, S282F, A308T, N312S, L346P, P399L, S460F, D666G, S817T, P941S, C1135Y, R1167T, I1175V, S1204C, R1214W, D1242R, D1242V, Y1245S, R1261C, R1261H, R1261L, F1287L, W1291R, K1299E, K1299N, R1302G, E1318G, P1367S, C1396W, L1398R, V1417F, G1869W, L1872P, I1873T, C1875R, H1881Q, H1881R, R1896M, R1896S, S1898F, V1900A, G1913D, A1919V, R1926H, P1941S, P1962L, R1966H, R1974M et R2000K.

6. Inhibiteur destiné à être utilisé dans un procédé de traitement de l'athérosclérose chez un sujet humain selon la revendication 4, dans lequel la mutation dans *DNMT3A* entraîne un changement d'acide aminé dans *DNMT3A* choisi parmi F290I, F290C, V296M, P307S, P307R, R326H, R326L, R326C, R326S, G332R, G332E, V339A, V339M, V339G, L344Q, L344P, R366P, R366H, R366G, A368T, A368V, R379H, R379C, I407T, I407N, I407S, F414L, F414S, F414C, A462V, K468R, C497G, C497Y, Q527H, Q527P, Y533C, S535F, C537G, C537R, G543A, G543S, G543C, L547H, L547P, L547F, M548I, M548K, G550R, W581R, W581G, W581C, R604Q, R604W, R635W, R635Q, S638F, G646V, G646E, L653W, L653F, I655N, V657A, V657M, R659H, Y660C, V665G, V665L, M674V, R676W, R676Q, G685R, G685E, G685A, D686Y, D686G, R688H, G699R, G699S, G699D, P700L, P700S, P700R, P700Q, P700T, P700A, D702N, D702Y, V704M, V704G, I705F, I705T, I705S, I705N, G707D, G707V, C710S, C710Y, S714C, V716D, V716F, V716I, N717S, N717I, P718L, R720H, R720G, K721R, K721T, Y724C, R729Q, R729W, R729G, F731C, F731L, F731Y, F731I, F732del, F732C, F732S, F732L, E733G, E733A, F734L, F734C, Y735C, Y735N, Y735S, R736H, R736C, R736P, L737H, L737V, L737F, L737R, A741V, P742P, P743R, P743L, R749C, R749L, R749H, R749G, F751L, F751C, F752del, F752C, F752L, F752I, F752V, W753G, W753C, W753R, L754P, L754R, L754H, F755S, F755I, F755L, M761I, M761V, G762C, V763I, S770L, S770W, S770P, R771Q, F772I, F772V, L773R, L773V, E774K, E774D, E774G, I780T, D781G, R792H, W795C, W795L, G796D, G796V, N797Y, N797H, N797S, P799S, P799R, P799H, R803S, R803W, P804L, P804S, K826R, S828N, K829R, T835M, N838D, K841Q, Q842E, P849L, D857N, W860R, E863D, F868S, G869S, G869V, M880V, S881R, S881I, R882H, R882P, R882C, R882G, A884P, A884V, Q886R, L889P, L889R, G890D, G890R, G890S, V895M, P896L, V897G, V897D, R899L, R899H, R899C, L901R, L901H, P904L, F909C, P904Q, A910P, C911R, C911Y.

7. Inhibiteur destiné à être utilisé dans un procédé de traitement de l'athérosclérose chez un sujet humain selon l'une quelconque des revendications précédentes, dans lequel le sujet humain a au moins un clone de cellule sanguine somatique avec un allèle *TET2* mutant et un allèle *TET2* de type sauvage.

8. Inhibiteur destiné à être utilisé dans un procédé de traitement de l'athérosclérose chez un sujet humain selon l'une quelconque des revendications 1 à 6, dans lequel le sujet humain a au moins un clone de cellule sanguine somatique avec deux allèles *TET2* mutants.

9. Inhibiteur destiné à être utilisé dans un procédé de traitement de l'athérosclérose chez un sujet humain selon l'une quelconque des revendications précédentes, dans lequel le sujet humain a au moins un clone de cellule sanguine somatique avec un allèle *DNMT3A* mutant et un allèle *DNMT3A* de type sauvage.

10. Inhibiteur destiné à être utilisé dans un procédé de traitement de l'athérosclérose chez un sujet humain selon l'une quelconque des revendications 1 à 8, dans lequel le sujet humain a au moins un clone de cellule sanguine somatique avec deux allèles *DNMT3A* mutants.

11. Inhibiteur destiné à être utilisé dans un procédé de traitement de l'athérosclérose chez un sujet humain selon l'une quelconque des revendications précédentes, dans lequel le sujet humain a une hématopoïèse clonale à potentiel indéterminé (CHIP) .

12. Inhibiteur destiné à être utilisé dans un procédé de traitement de l'athérosclérose chez un sujet humain selon l'une quelconque des revendications précédentes, dans lequel le sujet humain a au moins une mutation *TET2* et/ou *DNMT3A* avec une fraction d'allèle variant d'au moins 2 %, 5 %, 10 %, 13,5 %, 15 %, 20 %, 25 %, 27 %, 30 %.

13. Inhibiteur destiné à être utilisé dans un procédé de traitement de l'athérosclérose chez un sujet humain selon l'une quelconque des revendications précédentes, dans lequel le taux plasmatique d'IL-8 du sujet est d'au moins 25 ng/ml, 30 ng/ml, 40 ng/ml, 45 ng/ml, 50 ng/ml, 55 ng/ml, 60 ng/ml, 65 ng/ml, 70 ng/ml, 75 ng/ml ou 80 ng/ml.

14. Inhibiteur destiné à être utilisé dans un procédé de traitement de l'athérosclérose chez un sujet humain selon l'une quelconque des revendications précédentes, dans lequel une mutation *TET2* et/ou *DNMT3A* est identifiée par séquençage de l'exome entier (WES) ou par séquençage d'ADN.


### SEQUENCE LISTING

<110> The Brigham and Women's Hospital, Inc. The General Hospital Corp.
<120> IL-8, IL-6, IL-1 AND TET2 AND DNMT3A IN ATHEROSCLEROSIS
<130> 01179-0001-00PCT
<150> US 62/489,823
   <151> 2017-04-25
<150> US 62/567,735
   <151> 2017-10-03
<160> 3
<170> PatentIn version 3.5
<210> 1
   <211> 20
   <212> DNA
   <213> Artificial Sequence
<220>
   <223> LOXP3R primer
<400> 1
   tagagggagg gggcataagt 20
<210> 2
   <211> 20
   <212> DNA
   <213> Artificial Sequence
<220>
   <223> FLOX F primer
<400> 2
   aagaattgct acaggcctgc 20
<210> 3
   <211> 20
   <212> DNA
   <213> Artificial Sequence
<220>
   <223> FLOX R primer
<400> 3
   ttctttagcc cttgctgagc 20